(19) Europäisches Patentamt
European Patent Office
Office européen des brevets

(11) Veröffentlichungsnummer: **0 296 416 B1**

(12) **EUROPÄISCHE PATENTSCHRIFT**

(45) Veröffentlichungstag der Patentschrift: **29.07.92**

(51) Int. Cl.[5]: **C07D 413/04**, C07D 413/14, C07D 417/04, C07D 417/14, C07D 487/04, C07D 487/14, C07D 263/58, C07D 265/36, C07D 279/36, C07D 277/68, A01N 43/76

(21) Anmeldenummer: **88109234.0**

(22) Anmeldetag: **10.06.88**

Die Akte enthält technische Angaben, die nach dem Eingang der Anmeldung eingereicht wurden und die nicht in dieser Patentschrift enthalten sind.

# (54) Benzo-anellierte cyclische Verbindungen.

(30) Priorität: **22.06.87 JP 155093/87**
**17.09.87 JP 231063/87**
**15.10.87 JP 258462/87**

(43) Veröffentlichungstag der Anmeldung:
**28.12.88 Patentblatt 88/52**

(45) Bekanntmachung des Hinweises auf die Patenterteilung:
**29.07.92 Patentblatt 92/31**

(84) Benannte Vertragsstaaten:
**BE CH DE FR GB IT LI NL**

(56) Entgegenhaltungen:
**EP-A- 0 170 191**
**EP-A- 0 176 101**
**EP-A- 0 218 972**
**EP-A- 0 235 567**
**DE-A- 1 927 759**

(73) Patentinhaber: **NIHON BAYER AGROCHEM K.K.**
**7-1, Nihonbashi Honcho 2-chome Chuo-ku**
**Tokyo 103(JP)**

(72) Erfinder: **Kume, Toyohiko**
**6-7-8, Asahigaoka**
**Hino-shi Tokyo(JP)**
Erfinder: **Goto, Toshio**
**3454-21, Honmachida**
**Machida-shi Tokyo(JP)**
Erfinder: **Kamochi, Atsumi**
**2-24-10 Higashi-Toyoda**
**Hino-shi Tokyo(JP)**
Erfinder: **Yanagi, Akihiko**
**2-1200-1, Nagabuchi**
**Oume-shi Tokyo(JP)**
Erfinder: **Yagi, Shigeki**
**1-30-7, Izumi**
**Suginami-ku Tokyo(JP)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 296 416 B1

CHEMICAL ABSTRACTS, Band 107, Nr. 19, 9. November 1987, Columbus, Ohio, USA; DOMAGALINA E. et al.: "Novel N-(3-indolymethyl) and N-(2-benzimidazolylmethyl) derivates of benzazoles", Seite 717, Spalte 1, Zusammenfassung-Nr. 175948v

CHEMICAL ABSTRACTS, Band 106, Nr. 3, 19. Januar 1987, Columbus, Ohio, USA; SHRIDHAR D.R. et al.: "Synthesis and anthelmintic activity of some new 6- and 7-isothiocyanato-2H-1,4-benzoxa(thia)zin-3(4-H)-ones and benzoxa(thia)zin-3-(4H)-thiones", Seite 598, Spalte 2, Zusammenfassung-Nr. 18463v

CHEMICAL ABSTRACTS, Band 75, Nr. 19, 8. November 1971, Columbus, Ohio, USA; SAM J. et al.: "Reaction of 3-(chloroalkyl)-2-benzoxazolinones with amines. Formation of 3-(aminoalkyl)-2-benzoxazolinones and 5-substituted 2,3,4,5-tetrahydro-1,5-benzoxazepines", Seite 218, Spalte 1, Zusammenfassung-Nr. 118292y

CHEMICAL ABSTRACTS, Band 69, Nr. 5, 29. Juli 1968, Columbus, Ohio, USA; PIECHACEK J. et al.: "Synthesis of 2,3-dihydro-3-oxo-1,4-benzothiazine derivatives", Seite 1799, Spalte 2, Zusammenfassung-Nr. 19098j

CHEMICAL ABSTRACTS, Band 68, Nr. 15, 8. April 1968, Columbus , Ohio, USA; INSTYTUT FARMACEUTYCZNY: "Derivatives of 2,3-dihydro-3-oxo-1,4-benzothiazines", Seite 6665, Spalte 1, Zusammenfassung-Nr. 69009f

CHEMICAL ABSTRACTS, Band 107, Nr. 23, 7. December 1987, Columbus, Ohio, USA; HAGA T. et al.: "Preparation of 2-(6-fluoro-2-oxobenzothiazol-5-yl)tetrahydroimidazo[1,5-a]pyridine-1,3-dione derivatives as herbicides", Seite 591, Spalte 2, Zusammenfassung-Nr. 217620q

CHEMICAL ABSTRACTS, Band 96, Nr. 17, 26. April 1982, Columbus, Ohio, USA; DOMAGALINA E. et al.: "5-, 6-, 7-Succinimidobenzoxazolin-2-ones", Seite 748, Spalte 2, Zusammenfassung-Nr. 142741y

Erfinder: **Shibuya, Katsuhiko**
**39-15, Namiki-cho**
**Hachioji-shi Tokyo(JP)**
Erfinder: **Miyauchi, Hiroshi**
**30-15, Namiki-cho**
**Hachioji-shi Tokyo(JP)**

(74) Vertreter: **Schumacher, Günter, Dr. et al**
**c/o Bayer AG Konzernverwaltung RP Patentabteilung**
**W-5090 Leverkusen 1 Bayerwerk(DE)**

## Beschreibung

Die Vorliegende Erfindung betrifft neue benzo-anellierte cyclische Verbindungen, Verfahren zu ihrer Herstellung und ihre Verwendung als Herbizide.

Es wurde bereits offenbart, daß bestimmte Benzoxazin-Derivate herbizide Aktivitäten aufweisen (siehe die EP-OS 170 191).

In der EP-A-176 101 werden Triazolopyridazine, deren Synthese, sowie deren Verwendung als Herbizide beschrieben.

Die EP-A-218 972 beschreibt Benzothiazolone als Substituenten eines Tetrahydrophthalimidgerüsts, deren Synthese und deren Verwendung als Herbizide.

In der EP-A-235 567 verden Indazol-Produkte beschrieben, die ebenfalls benzothiazolon-substituiert sind und herbizide Wirkeigenschaften aufweisen.

Die DE-A-1 927 759 schließlich beschreibt basische Derivate des Benzoxazolin-2-ons und Verfahren zur Herstellung derselben, was in Bezug auf die in der vorliegenden Anmeldung beanspruchten Zwischenverbindungen relevant ist.

Nunmehr wurden neue benzo-anellierte cyclische Verbindungen der Formel (I)

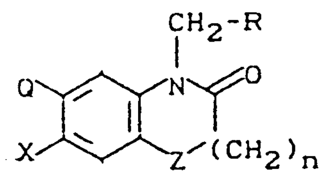

(I)

gefunden, in der

Q

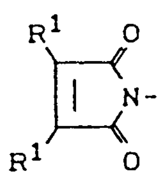

,

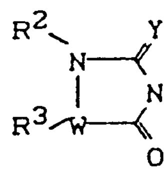

,

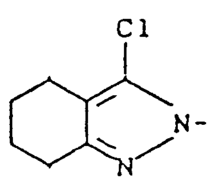

oder

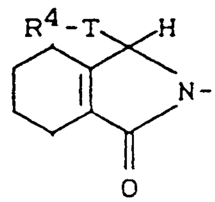

ist, worin

$R^1$ Methyl ist oder ein $R^1$ zusammen mit einem anderen $R^1$ Tetramethylen bilden kann,

$R^2$ zusammen mit $R^3$ Tetramethylen oder $-CH_2CH=CHCH_2-$ bezeichnet,

$R^4$ Wasserstoff, $C_1$-$C_4$-Alkyl, $C_1$-$C_4$-Alkylcarbonyl oder Phenyl ist,

Y O oder S ist,

W - H- oder - - ist,

T O, S, -NH- oder - -$C_1$-$C_4$-alkyl ist und

$R^4$ Zusammen mit T Chlor bezeichnen kann,

Z O oder S ist,

X Wasserstoff oder Halogen ist,

n 0 oder 1 ist und

R $C_{3-6}$-Cycloalkyl, eine gegebenenfalls substituierte 5-gliedrige heterocyclische Gruppe, die ein bis

drei Stickstoff-Atome oder ein bis zwei Stickstoff-Atome zusammen mit einem Sauerstoff- oder einem Schwefel-Atom enthält, oder eine gegebenenfalls substituierte 6-gliedrige heteroaromatische Gruppe, die ein bis drei Stickstoff-Atome enthält, ist, wobei gleiche oder verschiedene Substituenten in jedem Fall Amino, $C_1$-$C_4$-Alkylamino, Di($C_1$-$C_4$-alkyl)amino, Formylamino, $C_1$-$C_4$-Alkylcarbonylamino, Halogen, $C_1$-$C_4$-Alkoxy, $C_1$-$C_4$-Alkylthio, $C_1$-$C_4$-Alkyl, Halogeno-$C_1$-$C_4$-alkyl und/oder $C_1$-$C_4$-Alkoxy-$C_1$-$C_4$-alkyl sind, sowie in dem Fall, in dem R eine 6-gliedrige heteroaromatische Gruppe ist,

sowie deren Salze.

Die Verbindungen der Formel (I) werden mittels eines Verfahrens erhalten, bei dem

a) in dem Fall, in dem Q

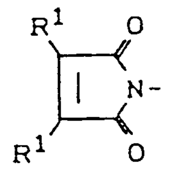

ist,

Verbindungen der Formel (II)

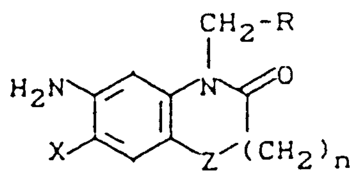

(II)

,

in der Z, X, R und n die im Vorstehenden angegebenen Bedeutungen haben, mit Verbindungen der Formel (III)

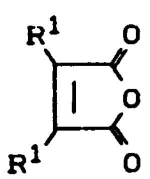

(III)

,

in der $R^1$ die im Vorstehenden angegebenen Bedeutungen hat, in Gegenwart inerter Lösungsmittel umgesetzt werden,

oder

b) in dem Fall, in dem Q

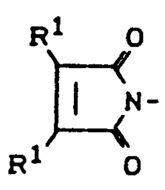

ist,

Verbindungen der Formel (IV)

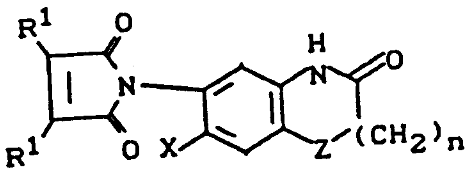

(IV)

,

in der $R^1$, Z, X und n die im Vorstehenden angegebenen Bedeutungen haben, mit Verbindungen der Formel (V)

$M\text{-}CH_2\text{-}R$ (V),

in der R die im Vorstehenden angegebenen Bedeutungen hat und M Halogen, Methansulfonyloxy, Benzolsulfonyloxy oder p-Toluolsulfonyloxy ist, in Gegenwart inerter Lösungsmittel und gegebenenfalls in Gegenwart einer Base umgesetzt werden,
oder
c) in dem Fall, in dem Q

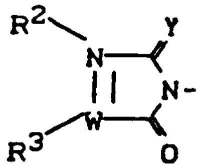

ist, worin
$R^2$ zusammen mit $R^3$ Tetramethylen bezeichnet und
W - H- oder - - ist,
Verbindungen der Formel (VI)

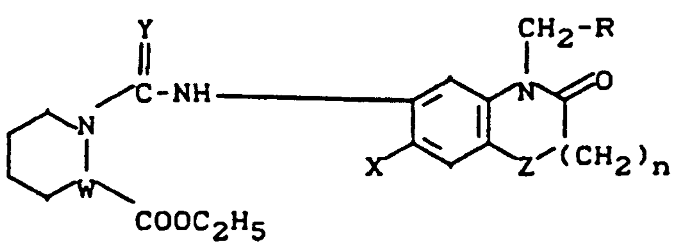

(VI)

,

in der Y, W, Z, X, R und n die im Vorstehenden angegebenen Bedeutungen haben, mit geeigneten Basen in Gegenwart inerter Lösungsmittel umgesetzt werden,
oder
d) in dem Fall, in dem Q

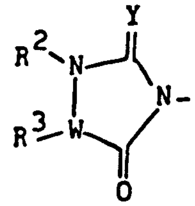

ist, worin

$R^2$ zusammen mit $R^3$ $-CH_2CH=CHCH_2-$ bezeichnet und

W - - ist,

Verbindungen der Formel (VII)

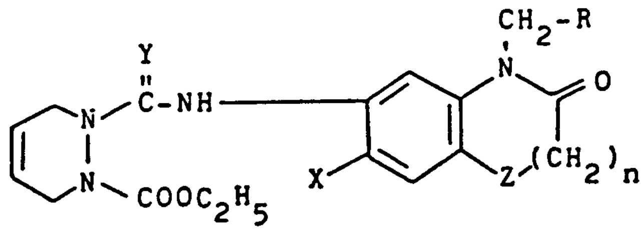

(VII)

,

in der Y, W, Z, X, R und n die im Vorstehenden angegebenen Bedeutungen haben, mit geeigneten Basen in Gegenwart inerter Lösungsmittel umgesetzt werden,

oder

e) in dem Fall, in dem Q

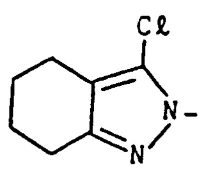

ist,

Verbindungen der Formel (VIII)

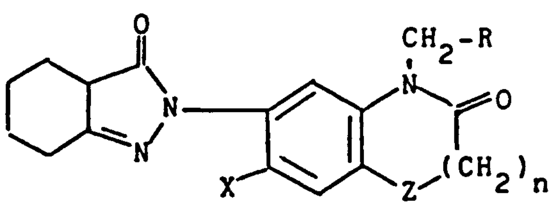

(VIII)

,

in der Z, X, R und n die im Vorstehenden angegebenen Bedeutungen haben, mit einem Chlorierungsmittel in Gegenwart inerter Lösungsmittel umgesetzt werden,

oder

f) in dem Fall, in dem Q

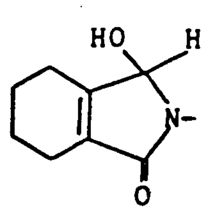

ist,
Verbindungen der Formel (Ia)

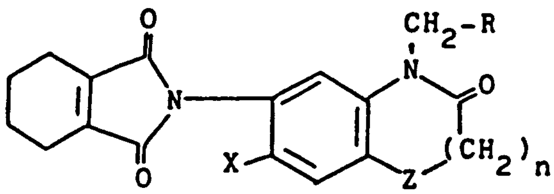

(Ia)

,

in der Z, X, R und n die im Vorstehenden angegebenen Bedeutungen haben, in Gegenwart inerter Lösungsmittel reduziert werden,
oder
g) in dem Fall, in dem Q

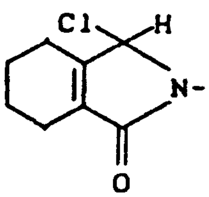

ist,
Verbindungen der Formel (Ib)

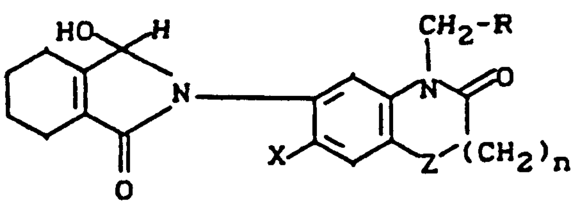

(Ib)

,

in der Z, X, R und n die im Vorstehenden angegebenen Bedeutungen haben, mit einem Chlorierungsmittel in Gegenwart inerter Lösungsmittel umgesetzt werden,
oder
h) in dem Fall, in dem Q

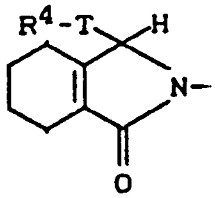

ist, worin $R^4$ Acyl ist und T O ist, $R^4$ durch $R^5$ ersetzt wird und die oben bezeichneten Verbindungen der Formel (Ib)

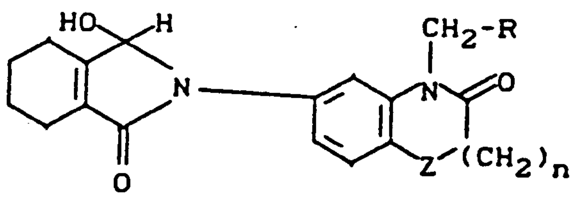 (Ib)

,

in der Z, X, R und n die im Vorstehenden angegebenen Bedeutungen haben, mit Verbindungen der Formel (IX)

$R^5$-Cl (IX) ,

worin $R^5$ Acyl ist,
oder mit Verbindungen der Formel (X)

$(R^5)_2O$ (X) ,

worin $R^5$ die im Vorstehenden angegebenen Bedeutungen hat, in Gegenwart inerter Lösungsmittel und gegebenenfalls in Gegenwart von Basen umgesetzt werden,
oder
i) in dem Fall, in dem Q

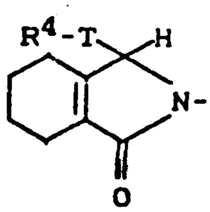

ist, mit der Maßgabe, daß $R^4$-T- nicht -OH, -Cl oder Acyloxy bezeichnet, $R^4$-T- durch $R^6$ ersetzt wird und die oben bezeichneten
Verbindungen der Formel (Ic)

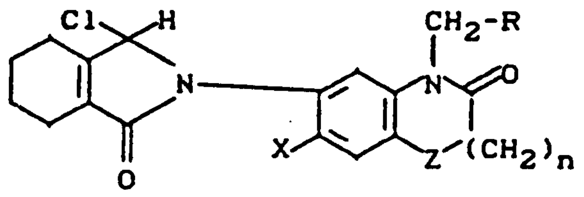

(Ic)

,

in der Z, X, R und n die im Vorstehenden angegebenen Bedeutungen haben, mit Verbindungen der Formel (XI)

$R^6$-H (XI),

worin $R^6$ Alkoxy, Alkylthio, Phenoxy, Phenylthio, Alkylamino oder Dialkylamino ist, in Gegenwart inerter Lösungsmittel und gegebenenfalls in Gegenwart einer Base umgesetzt werden.

Die benzo-anellierten cyclischen Verbindungen gemäß der vorliegenden Erfindung zeigen potente herbizide Eigenschaften.

Überraschenderweise zeigen die erfindungsgemäßen benzo-anellierten cyclischen Verbindungen nicht nur eine wesentlich stärkere herbizide Wirkung als die aus dem oben genannten Stand der Technik bekannten, sondern auch eine günstige Verträglichkeit mit Nutzpflanzen, nämlich ohne Phytotoxizität. Insbesondere vermögen die benzo-anellierten cyclischen Verbindungen Unkräuter auf höher gelegenen Ackerböden präzise und wirksam durch Behandlung vor dem Auflaufen zu bekämpfen.

Unter den benzo-anellierten cyclischen Verbindungen der Formel (I) gemäß der vorliegenden Erfindung sind besonders bevorzugte Verbindungen diejenigen, in denen

Q

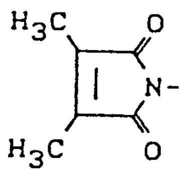

,

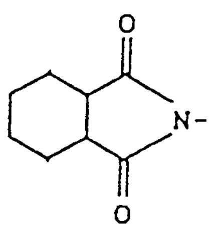

,

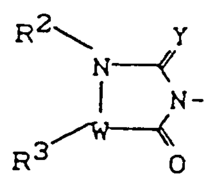

, oder

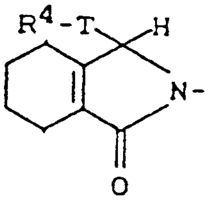

ist, worin
$R^2$ zusammen mit $R^3$ Tetramethylen oder $-CH_2CH=CHCH_2-$ bezeichnet,
$R^4$ Wasserstoff, Methyl, Ethyl, Methylcarbonyl oder Phenyl ist,
T Sauerstoff oder Schwefel ist oder $R^4$ zusammen mit T Chlor bezeichnen kann,
Y O oder S ist und
W - H- oder - - ist,
ist und

- Z O oder S ist,
- X Wasserstoff oder Fluor ist,
- R Cyclopropyl, eine gegebenenfalls substituierte 5-gliedrige heterocyclische Gruppe, die wenigstens ein Stickstoff-Atom und entweder ein Sauerstoff-Atom oder ein Schwefel-Atom enthält, oder eine 6-gliedrige heteroaromatische Gruppe, die ein oder zwei Stickstoff-Atome enthält, ist, wobei gleiche oder verschiedene Substituenten in jedem Fall Amino, Methylamino, Ethylamino, Dimethylamino,

Formylamino, Acetamido, Fluoro, Chloro, Bromo, Methoxy, Ethoxy, Methylthio, Ethylthio, Methyl, Ethyl, Isopropyl, Trichloromethyl, Trifluoromethyl und/oder Methoxymethyl sind und

n 0 oder 1 ist.

Besonders bevorzugte benzo-anellierte cyclische Verbindungen der Formel (I) sind diejenigen, in denen

Q

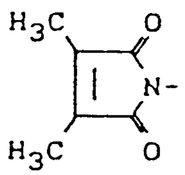 , 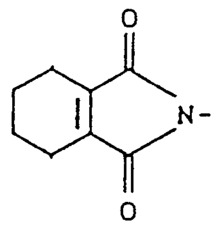 oder 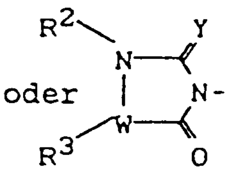

ist, worin

$R^2$ zusammen mit $R^3$ Tetramethylen oder $-CH_2CH=CHCH_2-$ bezeichnet,

Y O oder S ist und

W - H- oder - - ist,

Z O oder S ist,

X Fluor ist,

n 0 oder 1 ist und

R eine gegebenenfalls substituierte heterocyclische Gruppe ist, die die folgenden Systeme Tetrahydrofuran, Furan, Thiophen, Pyrazol, Imidazol, 1,2,4-Triazol, Tetrazol, Isoxazol, Oxazol, 1,2,4-Oxadiazol, 1,2,5-Oxadiazol, 1,3,4-Oxadiazol, Isothiazol, Thiazol, 1,2,3-Thiadiazol, 1,2,4-Thiadiazol, 1,2,5-Thiadiazol, 1,3,4-Thiadiazol, Pyridin, Pyrimidin und Pyrazin umfaßt, wobei gleiche oder verschiedene Substituenten in jedem Fall Amino, Methylamino, Ethylamino, Dimethylamino, Formylamino, Acetamido, Fluoro, Chloro, Bromo, Methoxy, Ethoxy, Methylthio, Ethylthio, Methyl, Ethyl, Isopropyl, Trichloromethyl, Trifluoromethyl und/oder Methoxymethyl sind.

In der Formel (I) seien als besondes bevorzugte Beispiele für R die jeweils auf den folgenden heterocyclischen Systemen basierenden Gruppen genannt:
Tetrahydrofuran, Furan, Thiophen, Pyrazol, Imidazol, 1,2,4-Triazol, Tetrazol, Isoxazol, Oxazol, 1,2,4-Oxadiazol, 1,2,5-Oxadiazol, 1,3,4-Oxadiazol, Isothiazol, Thiazol, 1,2,3-Thiadiazol, 1,2,4-Thiadiazol, 1,2,5-Thiadiazol, 1,3,4-Thiadiazol, Pyridin, Pyrimidin und Pyrazin.

Die vorstehenden heterocyclischen Gruppen können gegebenenfalls durch wenigstens einen Substituenten, der aus Amino, Methylamino, Ethylamino, Dimethylamino, Formylamino, Acetamido, Fluoro, Chloro, Bromo, Methoxy, Ethoxy, Methylthio, Ethylthio, Methyl, Ethyl, Isopropyl, Trichloromethyl, Trifluoromethyl und Methoxymethyl ausgewählt ist, substituiert ist.

Speziell genannt seien die folgenden Verbindungen:
6-Fluoro-3-(pyridin-2-ylmethyl)-5-(3,4-dimethyl-2,5-dioxo-2,5-dihydropyrrol-1-yl)-2(3H)benzothiazolon,
4-(Pyridin-2-ylmethyl)-6-(4,5,6,7-tetrahydro-2H-isoindol-1,3-dion-2-yl)-2H-1,4-benzoxazin-3(4H)-on,
7-Fluoro-4-(pyridin-2-ylmethyl)-6-(4,5,6,7-tetrahydro-2H-isoindol-1,3-dion-2-yl)-2H-1,4-benzoxazin-3(4H)-on,
7-Fluoro-4-(pyrazin-2-ylmethyl)-6-(4,5,6,7-tetrahydro-2H-isoindol-1,3-dion-2-yl)-2H-1,4-benzoxazin-3(4H)-on,
6-Fluoro-3-(isoxazol-3-ylmethyl)-5-(4,5,6,7-tetrahydro-2H-isoindol-1,3-dion-2-yl)-2(3H)-benzoxazolon,
6-Fluoro-3-(5-methyl-1,2,4-oxadiazol-3-ylmethyl)-5-(4,5,6,7-tetrahydro-2H-isoindol-1,3-dion-2-yl)-2(3H)-benzoxazolon,
6-Fluoro-3-(isoxazol-3-ylmethyl)-5-(4,5,6,7-tetrahydro-2H-isoindol-1,3-dion-2-yl)-2(3H)benzothiazolon,
6-Fluoro-3-(5-methylisoxazol-3-ylmethyl)-5-(4,5,6,7-tetrahydro-2H-isoindol-1,3-dion-2-yl)-2(3H)-benzothiazolon,
6-Fluoro-3-(5-methyl-1,2,4-oxadiazol-3-ylmethyl)-5-(4,5,6,7-tetrahydro-2H-isoindol-1,3-dion-2-yl)-2(3H)-benzothiazolon,
6-Fluoro-3-(5-methyl-1,2,4-oxadiazol-3-ylmethyl)-5-(1,2-tetramethylen-1,2,4-triazolidin-3,5-dion-4-yl)-2(3H)-benzothiazolon,
6-Fluoro-3-(isoxazol-3-ylmethyl)-5-(1,2-tetramethylen-1,2,4-triazolidin-3,5-dion-4-yl)-2(3H)benzothiazolon,
6-Fluoro-3-(5-methyl-1,2,4-oxadiazol-3-ylmethyl)-5-(1,2-tetramethylen-1,2,4-triazolidin-3-on-5-thion-4-yl)-2-(3H)benzothiazolon,
6-Fluoro-3-(isoxazol-3-ylmethyl)-5-(1,2-tetramethylen-1,2,4-triazolidin-3-on-5-thion-4-yl)-2(3H)benzothiazolon,

4-(Isoxazol-3-ylmethyl)-6-(3,4-dimethyl-2,5-dihydropyrrol-2,5-dion-1-yl)-2H-1,4-benzoxazin-3(4H)-on,
6-(3,4-Dimethyl-2,5-dihydropyrrol-2,5-dion-1-yl)-4-(5-methyl-1,2,4-oxadiazol-3-ylmethyl)-2H-1,4-benzoxazin-3(4H)-on,
4-(Isoxazol-3-ylmethyl)-6-(4,5,6,7-tetrahydro-2H-isoindol-1,3-dion-2-yl)-2H-1,4-benzoxazin-3(4H)-on und
4-(5-Methyl-1,2,4-oxadiazol-3-ylmethyl)-6-(4,5,6,7-tetrahydro-2H-isoindol-1,3-dion-2-yl)-2H-1,4-benzoxazin-3-(4H)-on.

Wenn beispielsweise in dem vorstehenden Verfahren a) 6-Amino-7-fluoro-4-(pyridin-2-ylmethyl)-2H-1,4-oxazin-3(4H)on und 2,3-Dimethylmaleinsäureanhydrid als Ausgangsstoffe eingesetzt werden, kann der Reaktionsablauf durch die folgende Gleichung dargestellt werden:

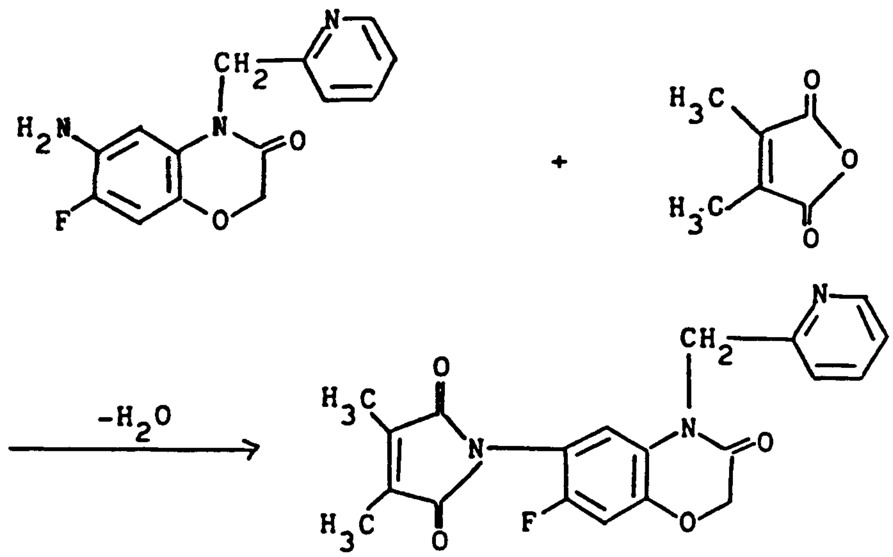


Wenn beispielsweise in dem vorstehenden Verfahren b) 7-Fluoro-6-(4,5,6,7-tetrahydro-2H-isoindol-1,3-dion-2-yl)-2H-1,4-benzoxazin-3(4H)on und 2-Chloromethylpyridin als Ausgangsstoffe eingesetzt werden, kann der Reaktionsablauf durch die folgende Gleichung dargestellt werden:

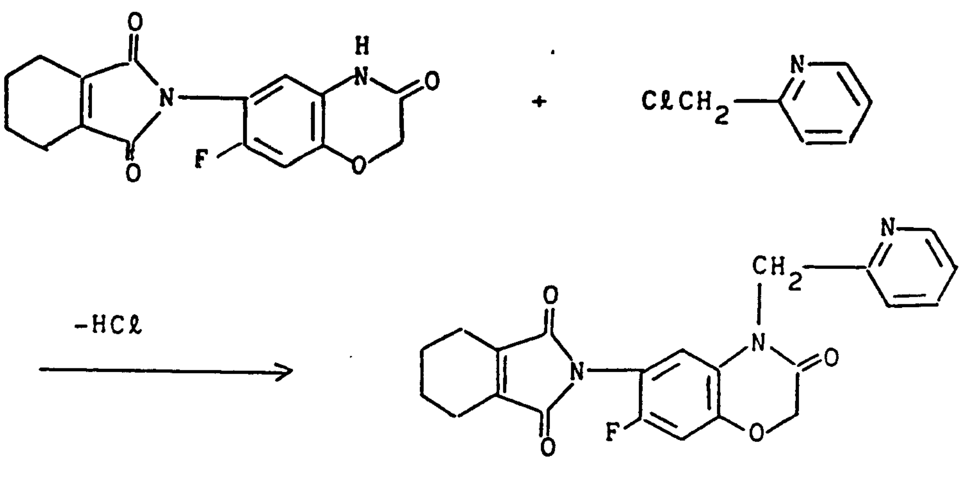


Wenn beispielsweise in dem vorstehenden Verfahren c) 5-(2-Ethoxycarbonyl-hexahydropyridazin-1-ylcarbonylamino)-6-fluoro-3-(5-methyl-1,2,4-oxadiazol-3-ylmethyl)-2(3H)-benzothiazolon und Natriumethoxid als Ausgangsstoffe eingesetzt werden, kann der Reaktionsablauf durch die folgende Gleichung dargestellt werden:

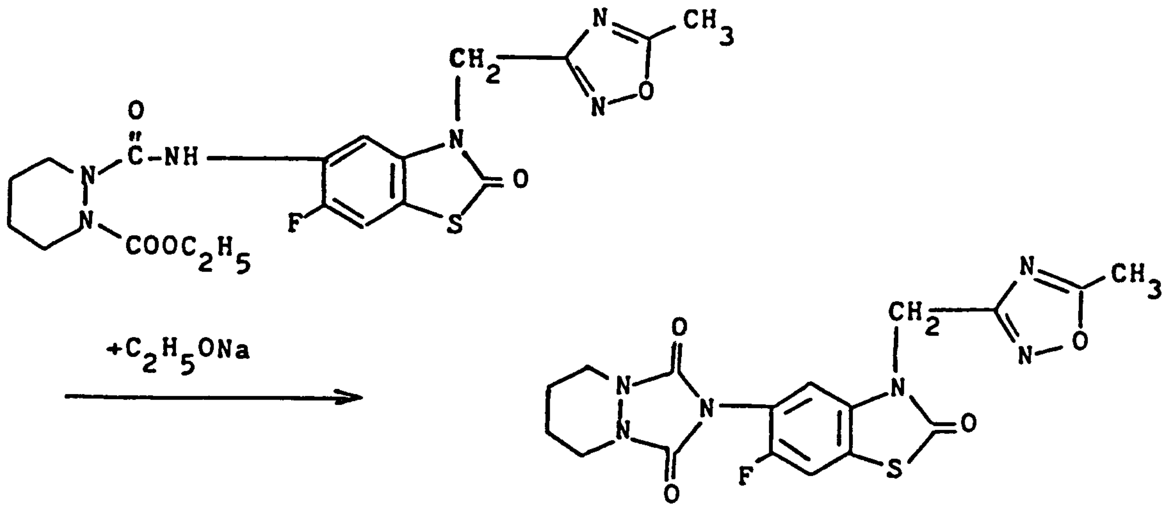


Wenn beispielsweise in dem vorstehenden Verfahren d) 6-(2-Ethoxycarbonyl-1,2,3,6-tetrahydropyridazin-1-ylcarbonylamino)-7-fluoro-4-(pyridin-2-ylmethyl)-2H-1,4-benzoxazin-3(4H)-on und Natriumethoxid als Ausgangsstoffe eingesetst werden, kann der Reaktionsablauf durch die folgende Gleichung dargestellt werden:

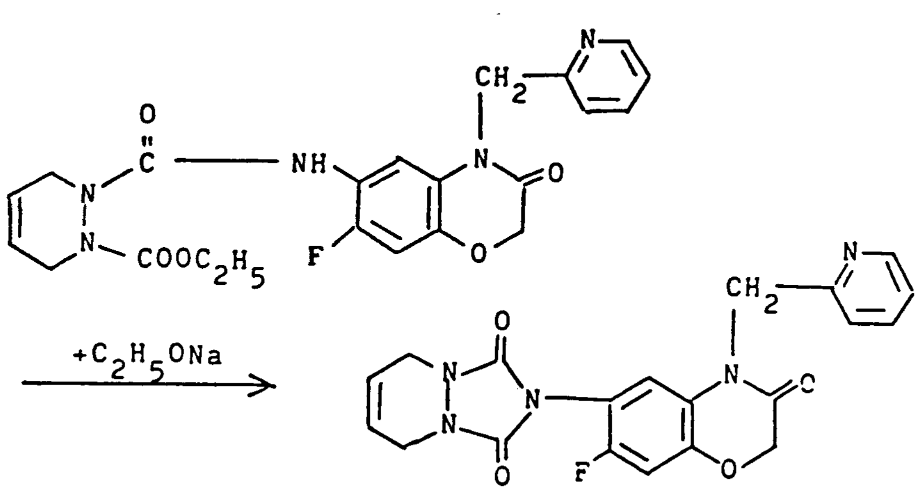


Wenn beispielsweise in dem vorstehenden Verfahren e) 6-Fluoro-5-(3,3a,4,5,6,7-hexahydro-2H-indazol-3-on-2-yl)-3-(isoxazol-3-ylmethyl)-2(3H)-benzothiazolon und Phosphorylchlorid als Ausgangsstoffe eingesetzt werden, kann der Reaktionsablauf durch die folgende Gleichung dargestellt werden:

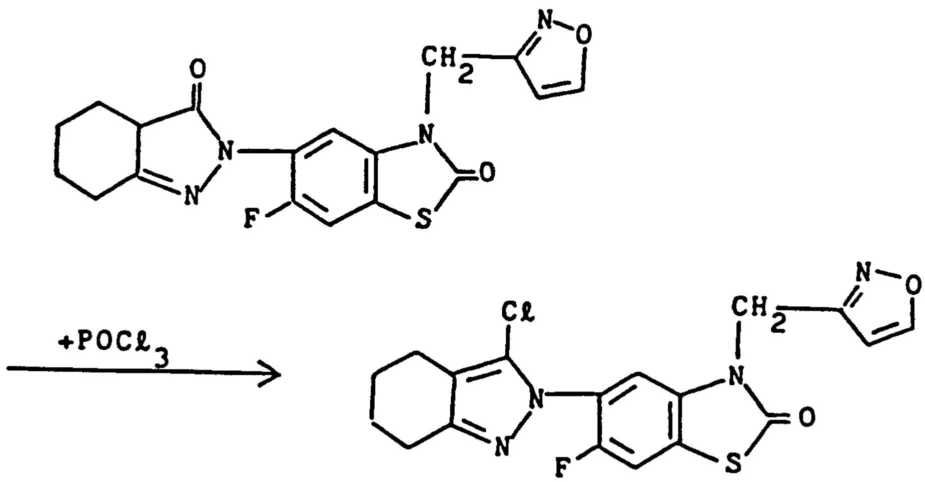


Wenn beispielsweise in dem vorstehenden Verfahren f) 7-Fluoro-4-(pyridin-2-ylmethyl)-6-(4,5,6,7-tetrahydro-2H-isoindol-1,3-dion-2-yl)-2H-1,4-benzoxazin-3(4H)on und Natriumborhydrid als Ausgangsstoffe eingesetzt werden, kann der Reaktionsablauf durch die folgende Gleichung dargestellt werden:

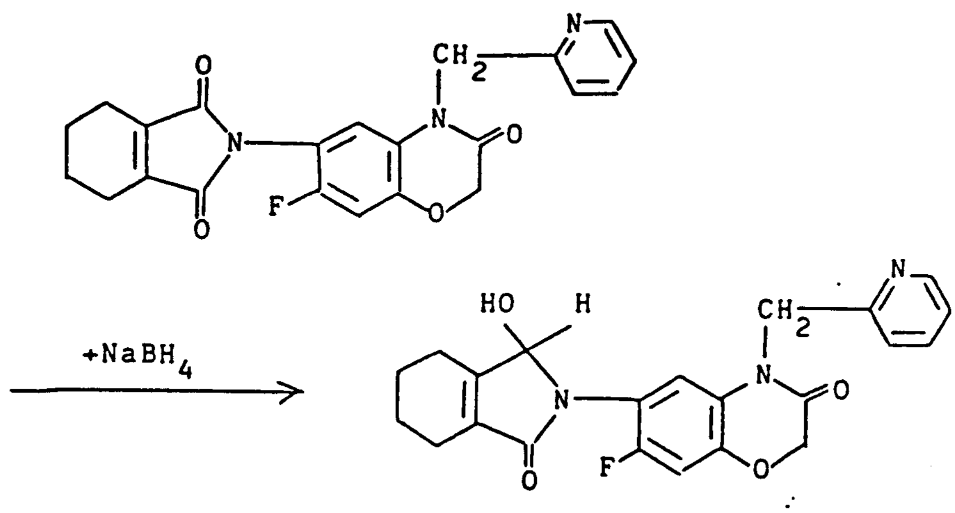


Wenn beispielsweise in dem vorstehenden Verfahren g) 7-Fluoro-4-(pyridin-2-ylmethyl)-6-(3-hydroxy-4,5,6,7-tetrahydroisoindolin-1-on-2-yl)-2H-1,4-benzoxazin-3(4H)on und Thionylchlorid als Ausgangsstoffe eingesetzt werden, kann der Reaktionsablauf durch die folgende Gleichung dargestellt werden:

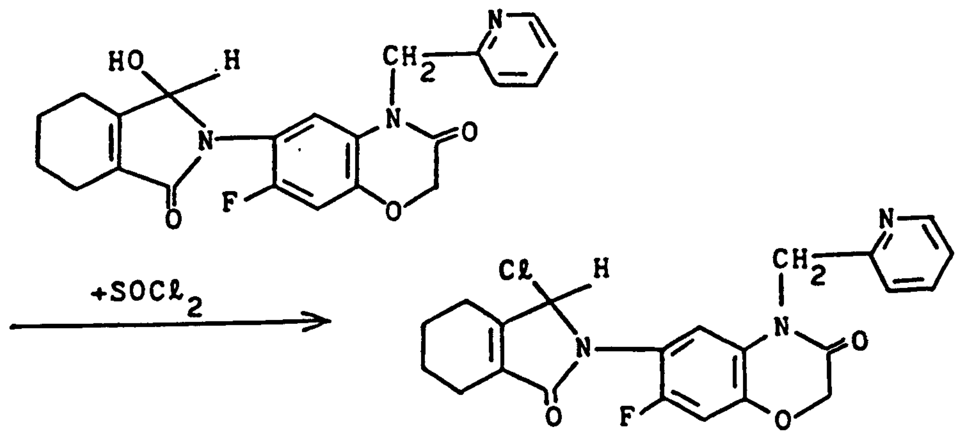


Wenn beispielsweise in dem vorstehenden Verfahren h) 7-Fluoro-4-(pyridin-2-ylmethyl)-6-(3-hydroxy-4,5,6,7-tetrahydroisoindolin-1-on-2-yl)-2H-1,4-benzoxazin-3(4H)on und Acetanhydrid als Ausgangsstoffe eingesetzt werden, kann der Reaktionsablauf durch die folgende Gleichung dargestellt werden:

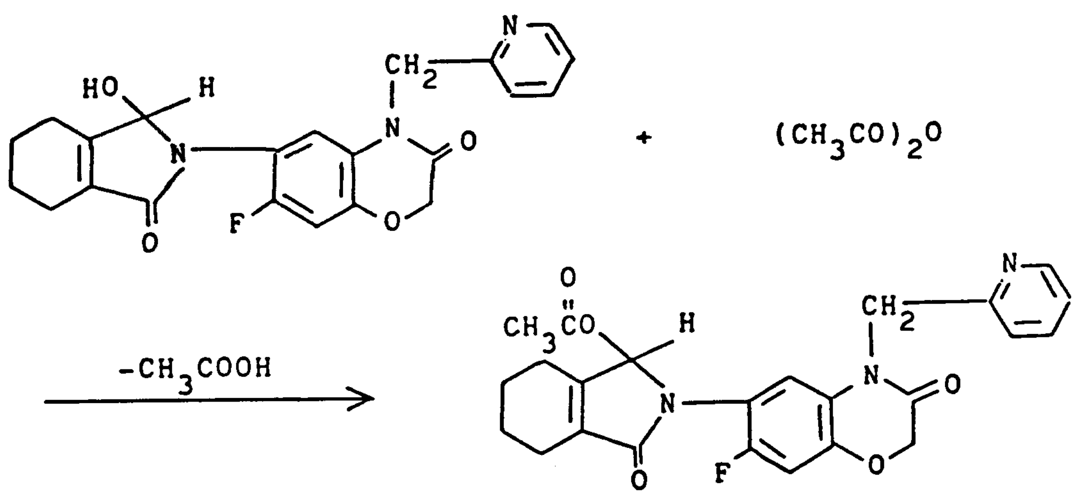


Wenn beispielsweise in dem vorstehenden Verfahren i) 6-(3-Chloro-4,5,6,7-tetrahydroisoindolin-1-on-2-yl)-7-fluoro-4-(pyridin-2-ylmethyl)-2H-1,4-benzoxazin-3(4H)on und Ethanol als Ausgangsstoffe eingesetzt werden, kann der Reaktionsablauf durch die folgende Gleichung dargestellt werden:

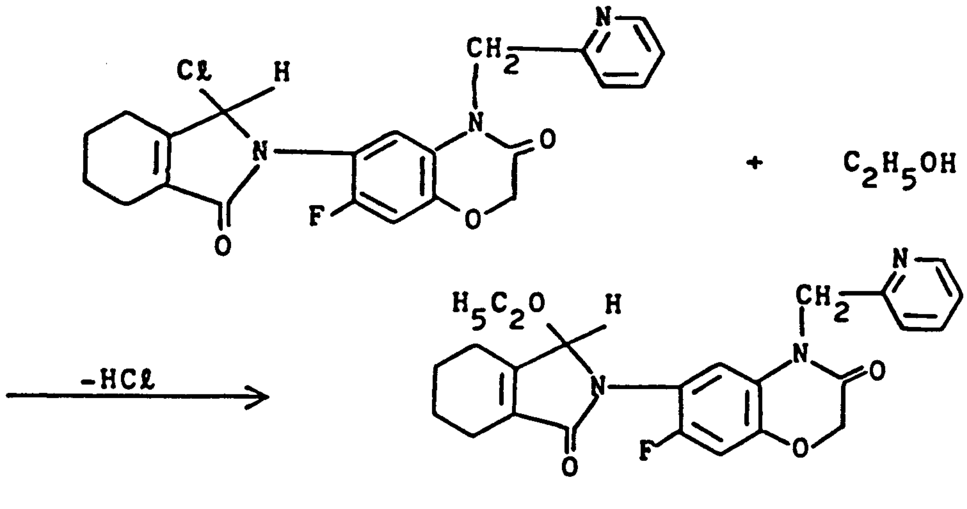


Die Formel (II) gibt eine Definition der Verbindungen, die als Ausgangsstoffe bei dem erfindungsgemäßen Verfahren a) benötigt werden.

In der Formel (II) haben Z, X, R und n vorzugsweise die bereits im Vorstehenden angegebenen Bedeutungen.

Die Verbindungen der Formel (II) sind neu und können im allgemeinen erhalten werden mittels eines Verfahrens, bei dem

j) Verbindungen der Formel (XII)

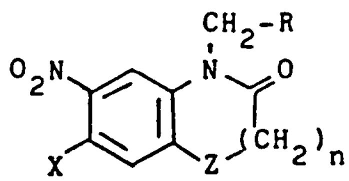

(XII) ,

in der Z, X, R und n die im Vorstehenden angegebenen Bedeutungen haben,
in Gegenwart inerter Lösungsmittel reduziert werden,
oder
k) Verbindungen der Formel (XIII)

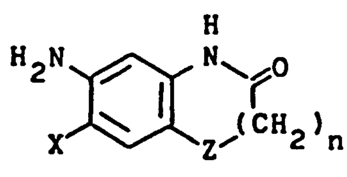

(XIII) ,

in der Z, X und n die im Vorstehenden angegebenen Bedeutungen haben,
mit den oben bezeichneten Verbindungen der Formel (V)

$M-CH_2-R$

in Gegenwart inerter Lösungsmittel und gegebenenfalls in Gegenwart einer Base umgesetzt werden.

Die Verbindungen der Formel (XII) in dem Verfahren j) sind neu und können beispielsweise erhalten werden mittels eines Verfahrens, bei dem

l) Verbindungen der Formel (XIV)

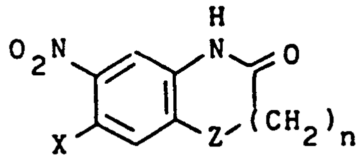

(XIV)

,

in der Z, X und n die im Vorstehenden angegebenen Bedeutungen haben, mit den oben bezeichneten Verbindungen der Formel (V) in Gegenwart inerter Lösungsmittel und gegebenenfalls in Gegenwart einer Base umgesetzt werden,

oder

m) in dem Fall, in dem in der Formel (XII) X Halogen ist, Verbindungen der Formel (XV)

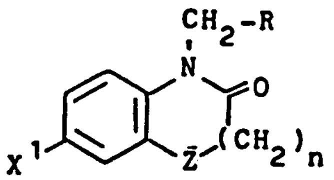

(XV)

,

in der Z, R und n die im Vorstehenden angegebenen Bedeutungen haben und $X^1$ Halogen ist, nitriert werden, gegebenenfalls in Gegenwart inerter Lösungsmittel und gegebenenfalls in Gegenwart eines Katalysators.

Die Verbindungen der Formel (XV) in dem Verfahren m) sind neu und können beispielsweise erhalten werden mittels eines Verfahrens, bei dem

n) Verbindungen der Formel (XVI)

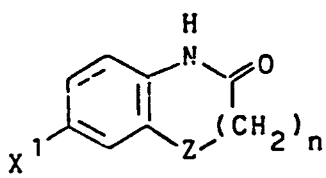

(XVI)

,

in der Z, $X^1$ und n die im Vorstehenden angegebenen Bedeutungen haben, mit den oben bezeichneten Verbindungen der Formel (V) in Gegenwart inerter Lösungsmittel und gegebenenfalls in Gegenwart einer Base umgesetzt werden.

Alternativ können die Verbindungen der Formel (XV), bei denen $X^1$ Fluor ist, aus in der 4-Stellung substituierten Derivaten von 7-Amino-2H-1,4-benzoxazin-3(4H)-on mittels der Schiemann-Reaktion erhalten werden (siehe Organic Reactions V, S. 193).

Die oben genannten, in der 4-Stellung substituierten Derivate von 7-Amino-2H-1,4-benzoxazin-3(4H)-on können durch Reduktion von Produkten hergestellt werden, welche durch Umsetzung von 7-Nitro-2H-1,4-benzoxazin-3(4H)-on mit den oben bezeichneten Verbindungen der Formel (V) erhalten wurden.

Das oben genannte 7-Nitro-2H-1,4-benzoxazin-3(4H)-on ist bekannt (siehe J. Chem. Soc. 1928, 3046, oder Synthesis 1982, 986).

Die Verbindungen der Formel (XVI) in dem Verfahren n) sind bekannt (siehe Chem. Abstr., Band 53, S. 5246, EP-OS 170 191 oder Farmco Ed. Sci., Band 32, Nr. 5, S. 348).

Die Verbindungen der Formel (XVI) können für den Fall, in dem Z S ist und n O ist, in einfacher Weise durch Hydrolysieren eines bekannten 2-Chloro-6-halogenobenzothiazols mit konz. Salzsäure in Ethanol erhalten werden.

Die Verbindungen der Formel (XIV) in dem Verfahren l) sind bekannt (siehe die JP-OSen 125529/1974 und 132872/1987, EP-OS 170 191, DD-PS 615 131).

Die Verbindungen der Formel (XIII) in dem Verfahren k) sind bekannte Verbindungen (siehe J. Am. Chem. Soc., Band 80, S. 1662, EP-OS 170 191, Chem. Abstr., Band 51, 17926f, JP-OS 155267/1987).

Bei der Durchführung des vorstehenden Verfahrens j) können Zinn(II)-chlorid und Salzsäure für die Reduktion verwendet werden.

Für die Durchführung des vorstehenden Verfahrens k) können als inerte Lösungsmittel beispielhaft genannt werden:
Wasser, Alkohole, Ketone, Nitrile, Tetrahydrofuran, Dioxan, Dimethylformamid, Dimethylsulfoxid und dergleichen.

Das Verfahren k) kann in Gegenwart einer Base wie Natriumhydroxid, Kaliumhydroxid, Kaliumcarbonat und dergleichen durchgeführt werden.

Das Verfahren k) kann bei Temperaturen zwischen etwa 0 °C und etwa 150 °C, vorzugsweise zwischen etwa 30 °C und etwa 100 °C, durchgeführt werden.

Bei der Durchführung des Verfahrens l) können als inerte Lösungsmittel die gleichen wie für das Verfahren k) beispielhaft genannt werden.

Das Verfahren l) kann ebenfalls in Gegenwart einer Base durchgeführt werden, die derjenigen gleicht, die für das Verfahren k) beispielhaft genannt wurde.

Das Verfahren l) kann bei Temperaturen zwischen etwa 20 °C und etwa 180 °C, vorzugsweise zwischen etwa 30 °C und etwa 100 °C, durchgeführt werden.

Das Verfahren m) kann nach einer herkömmlichen Nitrier-Methode durchgeführt werden. Mögliche Verdünnungsmittel für die Durchführung der Herstellung nach dem Verfahren m) sind sämtliche Lösungsmittel, die gewöhnlich für solche Nitrierungsreaktionen eingesetzt werden. Die gleichzeitig als Reagens oder als Mischung derselben mit Katalysator-Säuren, zum Beispiel Schwefelsäure, eingesetzte Salpetersäure wird vorzugsweise in einem entsprechenden Überschuß als Verdünnungsmittel verwendet.

Gegebenenfalls können inerte organische Lösungsmittel, wie beispielsweise Eisessig oder chlorierte Kohlenwasserstoffe wie Methylenchlorid, Chloroform oder Kohlenstofftetrachlorid, ebenfalls als Verdünnungsmittel benutzt werden.

Mögliche Katalysatoren oder Reaktionshilfsmittel für die Durchführung des Herstellungsverfahrens sind in gleicher Weise die Katalysatoren, die für derartige Nitrierungsreaktionen gebräuchlich sind, beispielsweise Schwefelsäure, Eisen(III)-chlorid oder andere Lewis-Säuren oder Acetanhydrid.

Die Reaktionstemperaturen können bei der Durchführung des Herstellungsverfahrens innerhalb eines breiten Bereichs variiert werden. Im allgemeinen wird die Reaktion zwischen -50 °C und +200 °C, vorzugsweise zwischen -20 °C und +50 °C, durchgeführt.

Für die Durchführung des Verfahrens m) beträgt das Stoffmengen-Verhältnis ("Mol-Verhältnis") eines Nitrierungsmittels zu den Verbindungen der Formel (XV) 0,98 bis 1,5, vorzugsweise 1 bis 1,2.

Das Verfahren n) kann in gleicher Weise wie das obige Verfahren l) durchgeführt werden, und die Reaktionstemperatur liegt dafür zwischen etwa 0 °C und etwa 150 °C, vorzugsweise zwischen etwa 30 °C und etwa 100 °C.

Die Verbindungen der Formel (III) in dem Verfahren a) sind bekannt, und als Beispiele seien erwähnt:
2,3-Dimethylmaleinsäureanhydrid und
3,4,5,6-Tetrahydrophthalsäureanhydrid.

Die Formel (IV) gibt eine Definition der Verbindungen, die als Ausgangsstoffe bei dem erfindungsgemäßen Verfahren b) benötigt werden.

In der Formel (IV) haben $R^1$, Z, X und n vorzugsweise die bereits im Vorstehenden angegebenen Bedeutungen.

Die Verbindungen der Formel (IV) umfassen zum Teil bekannte Verbindungen (siehe die EP-OS 170 191 und die JP-OS 155276/1987) und können allgemein mittels eines Verfahrens erhalten werden, bei dem

o) die oben bezeichneten Verbindungen der Formel (XIII) mit den oben bezeichneten Verbindungen der Formel (III) in Gegenwart inerter Lösungsmittel umgesetzt werden.

Das vorstehende Verfahren o) kann nach einer Methode durchgeführt werden, die in Agr. Biol. Chem., Band 40, Nr. 4, S. 745, oder in Pestic. Biochem. Physiol., Band 14, S. 153-160, beschrieben ist.

Die Formel (V) gibt eine Definition der Verbindungen, die als Ausgangsstoffe bei dem erfindungsgemäßen Verfahren b) benötigt werden.

In der Formel (V) hat R vorzugsweise die bereits im Vorstehenden angegebenen Bedeutungen, und M bezeichnet vorzugsweise Chlor, Brom, Iod, Methansulfonyloxy, Benzolsulfonyloxy oder p-Toluolsulfonyloxy. Insbesondere bezeichnet M Chlor.

Die Verbindungen der Formel (V) sind als Alkylierungsmittel in der organischen Chemie bekannt.

Für den Fall, in dem R in der Formel (V) eine 5-gliedrige heterocyclische Gruppe ist, seien als spezielle Beispiele erwähnt:
3-(Chloromethyl)isoxazol,
3-Chloromethyl-5-nethylisoxazol,
3-Chloromethyl-1-methyl-1,2,4-triozol,
3-Chloromethyl-5-methyl-1,2,4-oxazol,
5-Chloromethyl-3-methyl-1,2,4-oxazol,
4-(Chloromethyl)thiazol,
4-Chloromethyl-2-methylthiazol,
3-Chloromethyl-4-methyl-1,2,5-oxadiazol und
3-Bromomethyl-1,2,5-thiadiazol.

Für den Fall in dem R in der Formel (V) eine 6-gliedrige heteroaromatische Gruppe ist, seien als spezielle Beispiele erwähnt:
2-(Chloromethyl)pyridin,
2-(Chloromethyl)pyrazin,
2-(Chloromethyl)pyrimidin,
4-(Chloromethyl)pyrimidin,
2-(Chloromethyl)-1,3,5-triazin und
3-(Chloromethyl)pyridazin.

Speziell das oben beispielhaft genannte 2-(Chloromethyl)pyridin kann erhalten werden durch Chlorieren von 2-Picolin mit N-Chlorosuccinimid nach einem konventionellen Verfahren oder durch Chlorieren von 2-(Hydroxymethyl)pyridin mit Thionylchlorid nach einer in J. Pharm. Soc. Japan, Band 79, S. 1277, beschriebenen Methode. Das oben erwähnte 2-(Hydroxymethyl)pyridin kann durch Hydrolysieren des Produkts gewonnen werden, das durch Umsetzung von 2-Methylpyridin-N-oxid mit Acetanhydrid erhalten wurde (siehe J. Am. Chem. Soc., Band 76, S. 1286, und Pharm. Bull., Band 3, S. 413).

Die Formel (VI) gibt eine Definition der Verbindungen, die als Ausgangsstoffe bei dem erfindungsgemäßen Verfahren c) benötigt werden.

In der Formel (VI) haben Y, W, Z, X, R und n vorzugsweise die bereits im Vorstehenden angegebenen Bedeutungen.

Die Verbindungen der Formel (VI) sind neu und können im allgemeinen erhalten werden mittels eines Verfahrens, bei dem

p) Verbindungen der Formel (XVII)

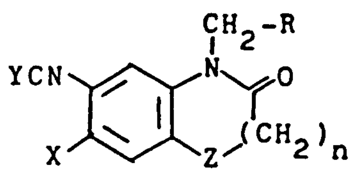

(XVII)

,

in der Y, Z, X, R und n die im Vorstehenden angegebenen Bedeutungen haben,
mit Ethylpipecolinat oder 1-Ethoxycarbonylhexahydropyridazin in Gegenwart inerter Lösungsmittel umgesetst werden.

Die Verbindungen der Formel (XVII) in dem Verfahren p) sind neu und können im allgemeinen erhalten werden mittels eines Verfahrens, bei dem

q) die oben bezeichneten Verbindungen der Formel (II) mit Phosgen oder Thiophosgen in Gegenwart inerter Lösungsmittel umgesetzt werden.

Das Verfahren q) kann für den Fall, in dem Y O ist, nach einer in J. Amer. Chem. Soc., Band 72, S. 1288, oder in Org. Synthesis, Band 2, S. 453, beschriebenen Methode bzw. für den Fall, in dem Y S ist, nach einer in J. Org. Chem. 1953, S. 1092, oder in J. Chem. Soc. 1927, S. 1186, beschriebenen Methode durchgeführt werden.

In dem Verfahren p) ist Ethylpipecolinat eine handelsüblich bekannte Verbindung, und 1-Ethoxycarbonylhexahydropyridazin kann in einfacher Weise nach einer in Bull. Soc. Chim. France 1957, S. 704, oder in der US-PS 2 841 584 beschriebenen Methode erhalten werden.

Zu geeigneten Basen in dem Verfahren c) zählen Alkoxide wie Natriumethoxid, Natriummethoxid, Alkalimetallhydroxide wie Kaliumhydroxid, Natriumhydroxid, Triethylamin und dergleichen.

Die Formel (VII) gibt eine Definition der Verbindungen, die als Ausgangsstoffe bei dem erfindungsgemäßen Verfahren d) benötigt werden.

In der Formel (VII) haben Y, Z, X, R und n vorzugsweise die bereits im Vorstehenden im Zusammenhang mit den erfindungsgemäßen Verbindungen der Formel (I) als bevorzugt angegebenen Bedeutungen.

Die Verbindungen der Formel (VII) sind neu und können im allgemeinen erhalten werden mittels eines Verfahrens, bei dem

r) die oben bezeichneten Verbindungen der Formel (XVII) mit 1-Ethoxycarbonyl-1,2,3,6-tetrahydropyridazin in Gegenwart inerter Lösungsmittel umgesetzt werden.

1-Ethoxycarbonyl-1,2,3,6-tetrahydropyridazin kann mittels einer in Bull. Soc. Chim. France 1957, S. 704, oder in der US-PS 2 841 584 beschriebenen Methode erhalten werden.

Die oben bezeichneten Verfahren p) und q) können nach einer in Agr. Biol. Chem., Band 40, Nr. 4, S. 745, beschriebenen Arbeitsweise durchgeführt werden.

Geeignete Basen in dem Verfahren d) umfassen diejenigen, die bereits beispielhaft für das Verfahren c) genannt wurden.

Die Formel (VIII) gibt eine Definition der Verbindungen, die als Ausgangsstoffe bei dem erfindungsgemäßen Verfahren e) benötigt werden.

In der Formel (VIII) haben Z, X, R und n vorzugsweise die bereits im Vorstehenden angegebenen Bedeutungen.

Die Verbindungen der Formel (VIII) sind neu und können im allgemeinen erhalten werden mittels eines Verfahrens, bei dem

s) Verbindungen der Formel (XVIII)

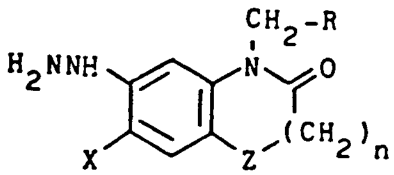

(XVIII) ,

in der Z, X, R und n die im Vorstehenden angegebenen Bedeutungen haben,

mit 2-Ethoxycarbonylcyclohexanon in Gegenwart inerter Lösungsmittel und gegebenenfalls in Gegenwart einer Base umgesetzt werden.

Die Verbindungen der Formel (XVIII) im Verfahren s) sind neu und können im allgemeinen erhalten werden mittels eines Verfahrens, bei dem

t) die oben bezeichneten Verbindungen der Formel (II) in Salzsäure diazotiert und anschließend reduziert werden.

2-Ethoxycarbonylcyclohexanon ist ein Handelsprodukt.

Für die Durchführung des vorstehenden Verfahrens s) können als inerte Lösungsmittel beispielhaft genannt werden:

Alkohole wie Ethanol, Isopropanol; Methylcellosolve, Dioxan, Toluol, Xylol und dergleichen.

Das Verfahren s) kann in Gegenwart einer Base durchgeführt werden, und beispielhaft für diese seien Natriummethoxid, Natriumethoxid, Triethylamin und dergleichen genannt.

Das Verfahren s) kann bei Temperaturen zwischen etwa 20 °C und etwa 150 °C, vorzugsweise zwischen etwa 50 °C und etwa 100 °C, durchgeführt werden.

Das obige Verfahren t) kann nach einer in "Methoden der organischen Chemie", Band 2, S. 177, beschriebenen Arbeitsweise durchgeführt werden.

Als Chlorierungsmittel im Verfahren e) seien genannt:

Phosgen, Thionylchlorid, Phosphorylchlorid, Oxalylchlorid und Trichloromethylchlorameisensäureester.

Die Formel (Ia) gibt eine Definition der Verbindungen, die als Ausgangsstoffe bei dem erfindungsgemäßen Verfahren f) benötigt werden.

Die Formel (Ib) gibt eine Definition der Verbindungen, die als Ausgangsstoffe bei den erfindungsgemäßen Verfahren g) und h) benötigt werden.

Die Formel (Ic) gibt eine Definition der Verbindungen, die als Ausgangsstoffe bei dem erfindungsgemäßen Verfahren i) benötigt werden.

Die Verbindungen der Formeln (Ia), (Ib) und (Ic) stellen Untergruppen der Verbindungen der Formel (I)

gemäß der vorliegenden Erfindung dar.

Chlorierungsmittel in dem Verfahren g) sind die gleichen, die bereits für das Verfahren e) beispielhaft genannt wurden.

Die Formeln (IX) oder (X) geben Definitionen der Verbindungen, die als Ausgangsstoffe bei dem erfindungsgemäßen Verfahren h) benötigt werden.

Die Verbindungen der Formeln (IX) oder (X) sind als Acylierungsmittel bekannt, und als Beispiele hierfür seien

Acetylchlorid, Acetanhydrid und Propionylchlorid genannt.

Die Formel (XI) gibt eine Definition der Verbindungen, die als Ausgangsstoffe bei dem erfindungsgemäßen Verfahren i) benötigt werden.

Die Verbindungen der Formel (XI) sind nahezu bekannte Verbindungen, wobei $R^6$ vorzugsweise $C_1$-$C_4$-Alkylthio, Phenoxy, Phenylthio, $C_1$-$C_4$-Alkylamino oder Di-$C_1$-$C_4$-alkylamino bedeutet.

Als Beispiele seien insbesondere erwähnt:

Methanol, Ethanol, Phenol, Ethanthiol, Benzolthiol, Methylamin, Dimethylamin und Isopropylamin.

Die Verbindungen der Formeln (II), (XII), (XV), (XVII) und (XVIII) sind neu und können durch die folgende Formel (XIX)

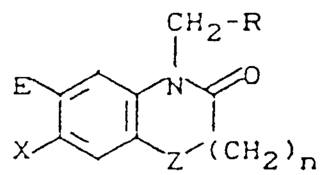

(XIX)

bezeichnet werden, in der

E Wasserstoff, Nitro, Amino, Hydrazino, Cyanato oder Thiocyanato ist,

X Wasserstoff oder Halogen ist,

Z O oder S ist,

R $C_{3-6}$-Cycloalkyl, eine gegebenenfalls substituierte 5-gliedrige heterocyclische Gruppe, die ein bis drei Stickstoff-Atome oder ein bis zwei Stickstoff-Atome zusammen mit einem Sauerstoff- oder einem Schwefel-Atom enthält, oder eine gegebenenfalls substituierte 6-gliedrige heteroaromatische Gruppe, die ein bis drei Stickstoff-Atome enthält, ist, wobei gleiche oder verschiedene Substituenten in jedem Fall Amino, $C_1$-$C_4$-Alkylamino, Di($C_1$-$C_4$-alkyl)amino, Formylamino, $C_1$-$C_4$-Alkylcarbonylamino, Halogen, $C_1$-$C_4$-Alkoxy, $C_1$-$C_4$-Alkylthio, $C_1$-$C_4$-Alkyl, Halogeno-$C_1$-$C_4$-alkyl und/oder $C_1$-$C_4$-Alkoxy-$C_1$-$C_4$-alkyl sind

n 0 oder 1 ist, ausgenommen die Verbindungen, in denen R einen Pyridinrest darstellt.

In der Formel (XIX) entsprechen für R die bevorzugten, besonders bevorzugten und ganz besonders bevorzugten Definitionen denjenigen, die bereits im Vorstehenden bei der Beschreibung der Verbindungen der Formel (I) erwähnt wurden.

Zu geeigneten Verdünnungsmitteln bei dem erfindungsgemäßen Verfahren a) zählen alle inerten Lösungsmitel. Diese umfassen vorzugsweise organische Säuren wie Essigsäure, Propionsäure und dergleichen.

Das Verfahren a) kann nach einer in Agr. Biol. Chem., Band 40, Nr. 4, S. 745, beschriebenen, bereits oben erwähnten Methode durchgeführt werden, und praktisch können die angestrebten Verbindungen dadurch erhalten werden, daß die beiden Ausgangsstoffe der Formeln (II) und (III) in Essigsäure zum Rückfluß erhitzt werden.

Für die Durchführung des Verfahrens a) beträgt das Stoffmengen-Verhältnis der Verbindungen der Formel (II) zu den Verbindungen der Formel (III) 0,8 bis 1,0, vorzugsweise 0,9 bis 0,99.

Die Reaktionstemperatur kann bei der Durchführung des Verfahrens a) innerhalb eines relativ breiten Bereichs variiert werden. Im allgemeinen wird die Reaktion bei Temperaturen zwischen 60 °C und 150 °C, vorzugsweise zwischen 90 °C und 120 °C, durchgeführt.

Bei der Durchführung des Verfahrens b) umfassen geeignete Verdünnungsmittel vorzugsweise Wasser, Nitrile wie Acetonitril, Alkohole wie Ethanol, Säureamide wie N,N-Dimethylformamid, Sulfoxide wie Dimethylsulfoxid, Ketone wie Aceton, Ether wie Tetrahydrofuran, Dioxan und Ethylenglycoldimethylether und dergleichen.

Das Verfahren b) kann auch in Gegenwart einer Base durchgeführt werden, und zu Beispielen für diese Basen zählen vorzugsweise Natriumcarbonat, Kaliumcarbonat, Natriumhydrid, Kaliumhydrid, Kaliumhydroxid, Natriumhydroxid, Natriummethoxid, Natriumethoxid, Kalium-t-butoxid und dergleichen.

Die Reaktionstemperatur des Verfahrens b) kann innerhalb eines Bereichs von beträchtlicher Breite variiert werden. Im allgemeinen wird die Reaktion bei Temperaturen zwischen etwa 20 °C und etwa 150 °C, vorzugsweise zwischen etwa 30 °C und etwa 100 °C, durchgeführt.

Die Reaktion des Verfahrens b) kann unter normalem, erhöhtem oder erniedrigtem Druck durchgeführt werden.

Bei der Durchführung des Verfahrens b) werden auf 1 mol der Verbindungen der Formel (IV) 1 bis etwa 1,2 mol der Verbindungen der Formel (V) in Gegenwart eines inerten Lösungsmittels und einer Base eingesetzt.

Bei der Durchführung der Verfahren c) und d) umfassen geeignete Verdünnungsmittel vorzugsweise Alkohole wie Ethanol, Isopropanol, t-Butanol; Methylcellosolve (Ethylenglycolmonomethylether), Dioxan, N,N-Dimethylformamid, Dimethylsulfoxid, Sulfolan und dergleichen.

Die Verfahren c) und d) können bei Temperaturen zwischen etwa 50 °C und etwa 130 °C, vorzugsweise zwischen etwa 70 °C und etwa 90 °C, durchgeführt werden.

Die Verfahren c) und d) können nach der in Agr. Biol. Chem., Band 40, Nr. 4, S. 745, beschriebenen Arbeitsweise durchgeführt werden. Beispielsweise werden bei der Durchführung des Verfahrens c) auf 1 mol der Verbindungen der Formel (VI) 0,01 bis 0,5 mol Natriumethoxid eingesetzt und bei einer Reaktionstemperatur, wie sie oben angegeben ist, während eines Zeitraums von 0,5 bis 16 h in Gegenwart von oben bereits beschriebenen inerten Lösungsmitteln zur Einwirkung gebracht, und bei der Durchführung des Verfahrens d) werden an Stelle der Verbindungen der Formel (VI) die Verbindungen der Formel (VII) unter den gleichen Reaktionsbedingungen wie beim vorstehenden Verfahren c) eingesetzt.

In der Praxis können die mittels des Verfahrens c) angestrebten Verbindungen der Formel (I) vorzugsweise auch dadurch erhalten werden, daß nacheinander die Verfahren q) und c) durchgeführt werden, ohne die Verbindungen der Formel (VI) zu isolieren.

In der Praxis können die mittels des Verfahrens d) angestrebten Verbindungen der Formel (I) vorzugsweise auch dadurch erhalten werden, daß nacheinander die Verfahren r) und d) durchgeführt werden, ohne die Verbindungen der Formel (VII) zu isolieren.

Bei der Durchführung des Verfahrens e) umfassen geeignete Verdünnungsmittel vorzugsweise Toluol, Xylol, Benzol und dergleichen.

Das Verfahren e) kann bei Temperaturen zwischen etwa 20 °C und etwa 150 °C, vorzugsweise zwischen etwa 50 °C und etwa 130 °C, durchgeführt werden.

Für die Durchführung des Verfahrens e) beträgt das Stoffmengen-Verhältnis des Chlorierungsmittel zu den Verbindungen der Formel (VIII) 1 bis 3, vorzugsweise 1 bis 1,5.

Das Verfahren e) kannen nach einer in der JP-OS 30761/1987 beschriebenen Arbeitsweise durchgeführt werden.

Die mittels des Verfahrens e) angestrebten Verbindungen der Formel (I) können vorzugsweise auch dadurch erhalten werden, daß nacheinander die Verfahren s) und e) durchgeführt werden, ohne die Verbindungen der Formel (VIII) zu isolieren.

Bei der Durchführung des Verfahrens f) umfassen geeignete Verdünnungsmittel vorzugsweise Wasser, Alkohole wie Ethanol und dergleichen, und zu den Reduktionsmitteln zählen vorzugsweise Alkalimetallhydride wie Natriumborhydrid, ALuminiumborhydrid und dergleichen.

Das Verfahren f) kann bei Temperaturen zwischen etwa -10 °C und etwa 80 °C, vorzugsweise zwischen etwa 0 °C und etwa 50 °C, durchgeführt werden.

Für die Durchführung des Verfahrens f) ist die Menge des Reduktionsmittels eine solche, die wenigstens 2 mol, vorzugsweise 2,2 bis 4 mol Wasserstoff, auf 1 mol der Verbindungen der Formel (Ia) äquivalent ist.

Bei der Durchführung des Verfahrens g) umfassen geeignete Verdünnungsmittel vorzugsweise Dichloromethan, Chloroform und dergleichen, und als Chlorierungsmittel sei Thionylchlorid beispielhaft genannt.

Das Verfahren g) kann bei Temperaturen zwischen etwa 30 °C und etwa 70 °C, vorzugsweise zwischen etwa 30 °C und etwa 50 °C, durchgeführt werden.

Das Verfahren h) kann bei Temperaturen zwischen etwa 0 °C und etwa 80 °C durchgeführt werden.

Für die Durchführung des Verfahrens g) beträgt das Stoffmengen-Verhältnis des Chlorierungsmittels zu den Verbindungen der Formel (Ib) 1 bis 2, vorzugsweise 1,05 bis 1,5.

Bei der Durchführung des Verfahrens h) umfassen geeignete Verdünnungsmittel vorzugsweise Benzol, Xylol, Toluol, Tetrahydrofuran und dergleichen.

Das Verfahren h) kann auch in Gegenwart einer Base durchgeführt werden, und Beispiele hierfür umfassen vorzugsweise Pyridin, 4-(Dimethylamino)pyridin, Triethylamin, Dimethylanilin und dergleichen.

Für die Durchführung des Verfahrens h) beträgt das Stoffmengen-Verhältnis der Verbindungen der

Formel (IX) zu den Verbindungen der Formel (Ib) 0,9 bis 1,2, vorzugsweise 1 bis 1,1.

Bei der Durchführung des Verfahrens i) umfassen geeignete Verdünnungsmittel vorzugsweise Alkohole wie Ethanol, Ketone wie Aceton und Methylethylketon, Ether wie Tetrahydrofuran, Nitrile wie Acetonitril, aromatische Kohlenwasserstoffe wie Benzol, Toluol, Xylol und dergleichen.

Das Verfahren i) kann in Gegenwart einer Base durchgeführt werden, und Beispiele hierfür umfassen diejenigen Basen, die für das Verfahren h) beispielhaft angegeben sind.

Das Verfahren i) kann bei Temperaturen zwischen etwa 0 °C und etwa 100 °C durchgeführt werden.

Für die Durchführung des Verfahrens i) beträgt das Stoffmengen-Verhältnis der Verbindungen der Formel (XI) zu den Verbindungen der Formel (Ic) 0,98 bis 1,5, vorzugsweise 1 bis 1,2.

Die erfindungsgemäßen aktiven Verbindungen können als Entlaubungsmittel, Abbrandmittel, Mittel zur Zerstörung breitblättriger Pflanzen und insbesondere als Unkrautvernichtungsmittel eingesetzt werden. Unter "Unkräutern" im weitesten Sinne sind alle Pflanzen zu verstehen, die an Stellen wachsen, wo sie nicht erwünscht sind. Ob die erfindungsgemäßen Substanzen als totale oder selektive Herbizide wirken, hängt im wesentlichen von der verwendeten Menge ab.

Die aktiven Verbindungen gemäß der Erfindung können beispielsweise in Verbindung mit den folgenden Pflanzen verwendet werden:

Dikotyledonen-Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea.

Dikotyledonen-Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita.

Monokotyledonen-Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Monokotyledonen-Kulturen der Gattungen:

Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern erstreckts sich auch auf andere Pflanzen in gleicher Weise.

In Abhängigkeit von den Konzentrationen eignen sich die Verbindungen zur totalen Bekämpfung von Unkräutern, beispielsweise auf Industriegelände und Bahnkörpern sowie auf Wegen und Plätzen mit oder ohne Baumbestand. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe können in übliche Präparate wie Lösungen, Emulsionen, Suspensionen, Pulver, Stäubemittel, Schäume, Pasten, lösliche Pulver, Granulat, Aerosole, mit dem Wirkstoff imprägnierte natürliche und synthetische Materialien, sehr feine Kapseln in polymeren Substanzen, Beschichtungsmittel zum Aufbringen auf das Saatgut und Formulierungen, die in Verbindung mit Verbrennungseinrichtungen wie Räucherpatronen, Räucherdosen und Räucherschlangen eingesetzt werden, sowie ULV-Kaltvernebelungs- und Warmvernebelungspräparate umgewandelt werden.

Diese Präparate können in bekannter Weise hergestellt werden, beispielsweise durch Vermischen der Wirkstoffe mit Streckmitteln, d.h. flüssigen oder verflüssigten gasförmigen oder festen Verdünnungsmitteln oder Trägern, gegebenenfalls unter Verwendung von grenzflächenaktiven Mitteln, d.h. Emulgatoren und/oder Dispergiermitteln und/oder schaumbildenden Mitteln. Bei Verwendung von Wasser als Streckmittel

können beispielsweise auch organische Lösungsmittel als Hilfslösungsmittel verwendet werden.

Als flüssige Verdünnungsmittel oder Träger eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Unter verflüssigten gasförmigen Verdünnungsmitteln oder Trägern sind Flüssigkeiten zu verstehen, die bei normaler Temperatur und unter normalem Druck gasförmig sein würden, beispielsweise Aerosol-Treibmittel wie halogenierte Kohlenwasserstoffe sowie Butan, Propan, Stickstoff und Kohlenstoffdioxid.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate, Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B. Gummi arabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe oder Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,1 bis 95 Gewichts-%, vorzugsweise von 0,5 bis 90 Gewichts-% des Wirkstoffs.

Die Wirkstoffe gemäß der vorliegenden Erfindung können als solche oder in Form ihrer Präparate auch zur Unkrautbekämpfung als Mischungen mit bekannten Herbiziden eingesetzt werden, wobei Fertigpräparate oder Tankmischungen möglich sind.

Mischungen mit anderen aktiven Verbindungen wie Herbiziden, Fungiziden, Insektisiden, Akariziden, Nematiziden, vogelabweisenden Mitteln, Pflanzennährstoffen sowie Mitteln zur Verbesserung der Bodenstruktur sind ebenfalls möglich.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben oder Streuen.

Die erfindungsgemäßen Wirkstoffe können vor oder nach dem Auflaufen der Pflanzen zur Anwendung gelangen.

Sie können auch vor der Einsaat in den Boden eingearbeitet werden. Insbesondere werden sie nach dem Auflaufen der Pflanzen angewandt.

Die eingesetzten Mengen der aktiven Verbindung können innerhalb eines Bereichs von erheblicher Breite variiert werden. Sie hängen wesentlich von der Art der angestrebten Wirkung ab. Im allgemeinen liegen die aufgewandten Mengen des Wirkstoffs zwischen etwa 0,0005 und etwa 3 kg/ha, vorzugsweise zwischen etwa 0,001 und etwa 2 kg/ha.

Die Herstellung und Verwendung der erfindungsgemäßen Verbindungen sind den folgenden Beispielen zu entnehmen.

## Herstellungsbeispiele

### Beispiel 1

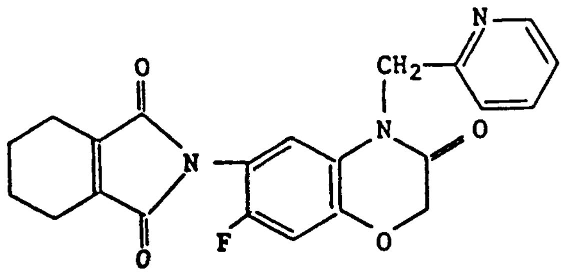

Eine Mischung aus 6-Amino-7-fluoro-4-(pyridin-2-ylmethyl)-2H-1,4-benzoxazin-3(4H)on (2 g), 3,4,5,6-Tetrahydrophthalsäureanhydrid (1,2 g) und Essigsäure (50 ml) wurde 1 h unter Rückfluß erhitzt. Die Essigsäure wurde unter vermindertem Druck abdestilliert, und der Rückstand wurde in Toluol (100 ml) gelöst, mit einer gesättigten wäßrigen Natriumhydrogencarbonat-Lösung und dann mit Wasser gewaschen und über wasserfreiem Natriumsulfat getrocknet. Das Toluol wurde unter vermindertem Druck abdestilliert, und der resultierende Rückstand wurde aus Ethanol umkristallisiert, wodurch das angestrebte Produkt, nämlich 7-Fluoro-4-(pyridin-2-ylmethyl)-6-(4,5,6,7-tetrahydro-2H-isoindol-1,3-dion-2-yl)-2H-1,4-benzoxazin-3-(4H)-on (2,4 g) erhalten wurde; Schmp. 188-190 °C.

In der gleichen Weise, wie sie in Beispiel 1 dargestellt ist, ist es möglich, 6-(3,4-Dimethyl-2,5-dioxo-2,5-dihydropyrrol-1-yl)-7-fluoro-4-(pyridin-2-ylmethyl)-2H-1,4-benzoxazin-3(4H)-onherzustellen.

Beispiel 2

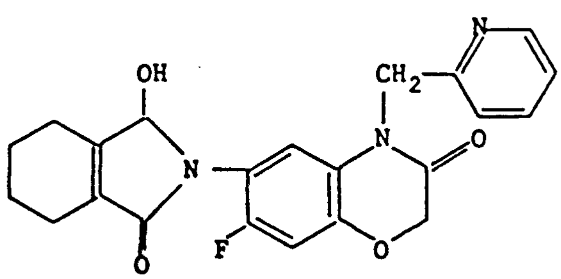

Die in Beispiel 1 erhaltene Verbindung (6,9 g) wurde in Methanol (200 ml) gelöst. Zu dieser Lösung wurde portionsweise Natriumborhydrid (0,7 g) bei einer Temperatur von 20 °C bis 30 °C hinzugefügt. Die Reaktionsmischung wurde 30 min bei Raumtemperatur gerührt, in Eiswasser (600 ml) gegossen und mit Ethylacetat (250 ml) extrahiert. Der Extrakt wurde mit Wasser gewaschen, über wasserfreiem Natriumsulfat getrocknet und zur Entfernung des Lösungsmittels destilliert, so daß das gewünschte Produkt, d.h. 7-Fluoro-4-(pyridin-2-ylmethyl)-6-(3-hydroxy-4,5,6,7-tetrahydroisoindolin-1-on-2-yl)-2H-1,4-benzoxazin-3(4H)on (6,9 g), als amorphes Material erhalten wurde.

Beispiel 3

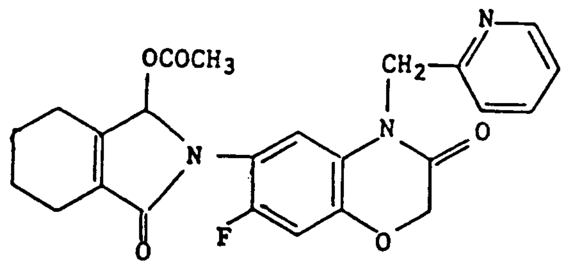

Das in Beispiel 2 erhaltene Produkt (1 g) wurde in Chloroform (20 ml) gelöst. Zu dieser Lösung wurden Triethylamin (0,5 g) und danach Acetanhydrid (0,5 g) bei einer Temperatur von 10 °C bis 20 °C hinzugefügt. Die Reaktionsmischung wurde 30 min bei Raumtemperatur gerührt und mit einer 2-proz. wäßrigen Salzsäure-Lösung (10 ml) versetzt. Die organische Schicht wurde abgetrennt, mit Wasser gewaschen und über wasserfreiem Natriumsulfat getrocknet. Nach dem Abdestillieren des Lösungsmittels wurde das angestrebte Produkt, d.h. 6-(3-Acetoxy-4,5,6,7-tetrahydroisoindolin-1-ony-2-yl)-7-fluoro-4-(pyridin-2-ylmethyl)-2H-1,4-benzoxazin-3(4H)on (1 g) erhalten; $n_D^{20}$ = 1,5725.

Beispiel 4

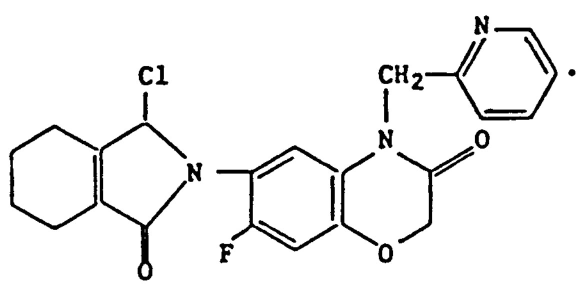

Die in Beispiel 2 erhaltene Verbindung (4,1 g) wurde in Chloroform (40 ml) gelöst. Zu dieser Lösung wurde Thionylchlorid (1,4 g) bei einer Temperatur von 10 °C bis 20 °C hinzugefügt. Die Reaktionsmischung wurde 30 min bei Raumtemperatur und dann weitere 2 h bei 30 °C bis 40 °C gerührt. Danach wurde die Reaktionsmischung auf eine Temperatur von 10 °C bis 15 °C abgekühlt, wodurch ein kristallines Produkt gebildet wurde, das durch Filtration gesammelt, mit Chloroform (10 ml) gewaschen und getrocknet wurde, wonach das angestrebte Produkt, d.h. 6-(3-Chloro-4,5,6,7-tetrahydroisoindolin-1-on-2-yl)-7-fluoro-4-(pyridin-2-ylmethyl)-2H-1,4-benzoxazin-3(4H)on-hydrochlorid (4,1 g) erhalten wurde.

Die in Beispiel 4 erhaltene Verbindung ist sehr reaktionsfähig, so daß es möglich ist, die Verbindung mit verschiedenartigen nucleophilen Agentien in Gegenwart einer Base wie Triethylamin umzusetzen.

Beispielsweise führt die Reaktion der Verbindung aus Beispiel 4 mit Ethanol zur Bildung von 6-(3-Ethoxy-4,5,6,7-tetrahydroisoindolin-1-on-2-yl)-7-fluoro-4-(pyridin-2-ylmethyl)-2H-1,4-benzoxazin-3(4H)on.

Wenn die Verbindung aus Beispiel 4 mit Ethanthiol umgesetzt wird, wird 6-(3-Ethylthio-4,5,6,7-tetrahydroisoindolin-1-on-2-yl)-7-fluoro-4-(pyridin-2-ylmethyl)-2H-1,4-benzoxazin-3(4H)on gebildet.

Wenn die Verbindung aus Beispiel 4 mit Dimethylamin umgesetzt wird, wird 6-(3-N,N-Dimethylamino-4,5,6,7-tetrahydroisoindolin-1-on-2-yl)-7-fluoro-4-(pyridin-2-ylmethyl)-2H-1,4-benzoxazin-3(4H)on gebildet.

Wenn die Verbindung aus Beispiel 4 mit wasserfreiem Kaliumfluorid in Dimethylsulfoxid umgesetzt wird, findet eine Halogen-Austauschreaktion statt, so daß 6-(3-Fluoro-4,5,6,7-tetrahydroisoindolin-1-on-2-yl)-7-fluoro-4-(pyridin-2-ylmethyl)-2H-1,4-benzoxazin-3(4H)on gebildet wird.

Beispiel 5

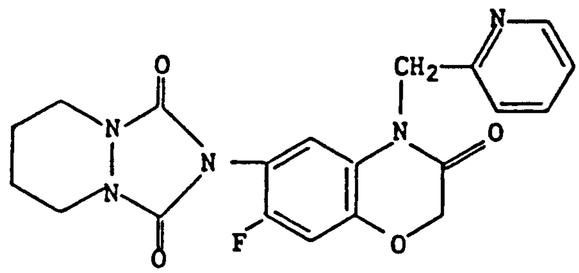

7-Fluoro-6-isocyanato-4-(pyridin-2-ylmethyl)-2H-1,4-benzoxazin-3(4H)on-hydrochlorid (2 g) wurde in Tetrahydrofuran (50 ml) suspendiert. Zu dieser Suspension wurde 1-Ethoxycarbonyl-hexahydropyridazin (1,2 g) hinzugefügt. Die Reaktionsmischung wurde bei einer Temperatur von 10 °C bis 20 °C gerührt und zur Entfernung des Triethylamin-hydrochlorids filtriert. Das Filtrat wurde konzentriert, so daß eine viskose Flüssigkeit (2,3 g) erhalten wurde. Dieses Produkt wurde NMR- und IR-spektroskopisch untersucht. Es handelte sich um eine Harnstoff-Verbindung der Formel

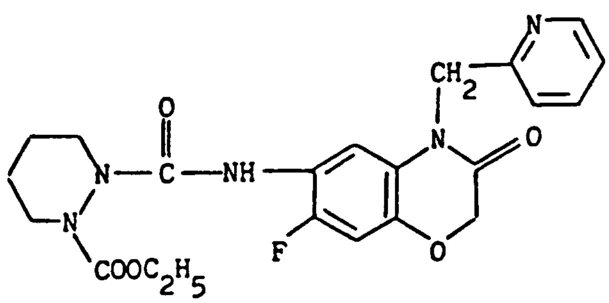

Die Harnstoff-Verbindung (2,2 g) wurde mit Ethanol (10 ml) vermischt, das eine katalytische Menge Natriumethoxid enthielt. Diese Mischung wurde 3 h unter Rückfluß erhitzt und dann auf eine Temperatur von 0 °C bis 10 °C abgekühlt. Dabei bildete sich ein kristallines Produkt, das durch Filtration gesammelt, mit einer kleinen Menge Ethanol gewaschen und getrocknet wurde. Erhalten wurde das gewünschte Produkt, d.h. 7-Fluoro-4-(pyridin-2-ylmethyl)-6-(1,2-tetramethylen-urazol-4-yl)-2H-1,4-benzoxazin-3(4H)-on (1,53 g); Schmp. 200-201 °C.

Beispiel 6

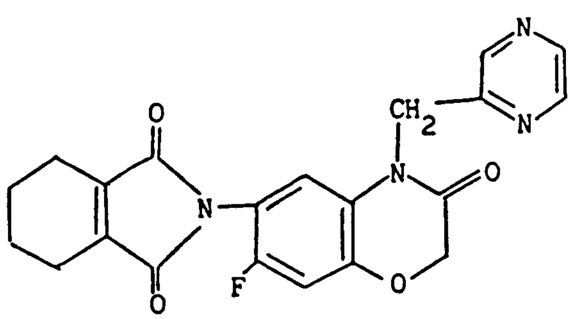

Eine Mischung aus 2-Methylpyrazin (5,64 g), α,α'-Azobis-(isobutyronitril) (0,05 g), N-Chlorosuccinimid (2,7 g) und Kohlenstofftetrachlorid (100 ml) wurde nach einer bekannten, in J. Org. Chem., Band 38, S. 2049, beschriebenen Arbeitsweise 4 h unter Rückfluß und unter Bestrahlung mit Licht einer elektrischen Glühlampe (100 V; 300 W) erhitzt. Die Reaktionsmischung wurde auf eine Temperatur von 10 °C bis 20 °C

gekühlt und filtriert. Das Filtrat wurde konzentriert, wonach eine Mischung (4,4 g) aus 2-(Chloromethyl)-pyrazin und 2-Methylpyrazin erhalten wurde. Das Stoffmengen-Verhältnis der ersteren zu der letzteren Verbindung wurde mittels H-NMR-Analyse bestimmt; das fragliche Stoffmengen-Verhältnis betrug 1 : 2. Die Mischung wurde mit 7-Fluoro-6-(4,5,6,7-tetrahydro-2H-isoindol-1,3-dion-2-yl)-2H-1,4-benzoxazin-3(4H)on (3,9 g) (das nach dem in der EP-OS 170 191 offenbarten Verfahren hergestellt worden war), Kaliumcarbonat (2 g) und Acetonitril (100 ml) versetzt und 4 h unter Rückfluß erhitzt. Die Mischung wurde abkühlen gelassen und dann zur Entfernung der Substanzen mit niedrigerem Siedepunkt aus der Mischung unter vermindertem Druck destilliert.

Der erhaltene Rückstand wurde mit Toluol (150 ml) vermischt, dann mit Wasser (100 ml) versetzt und gerührt. Danach wurde die organische Schicht abgetrennt, mit einer 5-proz. wäßrigen Kaliumhydroxid-Lösung und dann mit Wasser gewaschen und über wasserfreiem Natriumsulfat getrocknet. Das Lösungsmittel wurde unter vermindertem Druck abdestilliert. Der auf diese Weise erhaltene Rückstand wurde aus Ethanol umkristallisiert, so daß die gewünschte Verbindung, d.h. 7-Fluoro-4-(pyrazin-2-ylmethyl)-6-(4,5,6,7-tetrahydro-2H-isoindol-1,3-dion-2-yl)-2H-1,4-benzoxazin-3(4H)on (1,72 g) erhalten wurde; Schmp. 191-194 °C.

Beispiel 7

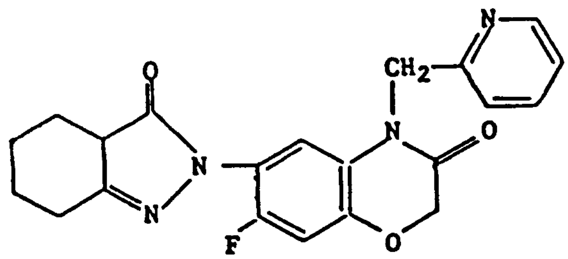

Eine Mischung aus 7-Fluoro-6-hydrazino-4-(pyridin-2-ylmethyl)-2H-1,4-benzoxazin-3(4H)on (1,49 g), 2-Ethoxycarbonyl-cyclohexanon (0,9 g) und Ethanol (20 ml) wurde mit einer katalytischen Menge Natriumethoxid vermischt und 5 h unter Rückfluß erhitzt. Die Reaktionsmischung wurde zur Entfernung des Ethanols destilliert, mit Toluol (30 ml) versetzt, unter Rückfluß 10 min erhitzt, auf eine Temperatur von 0 °C bis 10 °C gekühlt und filtriert. Der auf diese Weise erhaltene feste Anteil wurde mit n-Hexan gewaschen und getrocknet, so daß die gewünschte Verbindung, d.h. 7-Fluoro-6-(3,3a,4,5,6,7-hexahydro-2H-indazol-3-on-2-yl)-4-(pyridin-2-ylmethyl)-2H-1,4-benzoxazin-3(4H)on (1,24 g), erhalten wurde; Schmp. 216-219 °C.

Beipiel 8

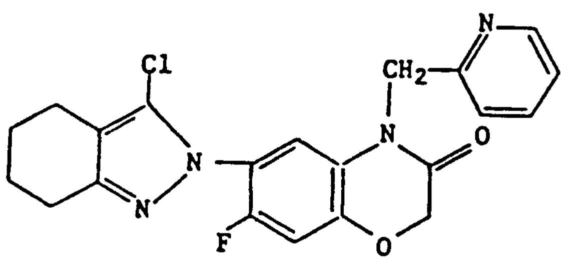

Eine Mischung aus 7-Fluoro-6-(3,3a,4,5,6,7-hexahydro-2H-indazol-3-on-2-yl)-4-(pyridin-2-ylmethyl)-2H-1,4-benzoxazin-3(4H)on (1,24 g), Phosgen (0,62 g) und Toluol (20 ml) wurde mit 1 Tropfen N,N-Dimethylformamid versetzt. Die Reaktionsmischung wurde 5 h unter Rückfluß erhitzt, auf eine niedrigere Temperatur abgekühlt und zur Entfernung des Lösungsmittels destilliert. Der erhaltene Rückstand wurde mit Ethylacetat (100 ml) und mit einer gesättigten wäßrigen Natriumhydrogencarbonat-Lösung (20 ml) vermischt. Die so erhaltene flüssige Mischung wurde gerührt, und die organische Schicht wurde abgetrennt, über wasserfreiem Natriumsulfat getrocknet und zur Entfernung des Ethylacetats destilliert. Der erhaltene Rückstand wurde mittels Silicagel-Säulenchromatographie (Elutionsmittel: Toluol/Tetrahydrofuran = 4/1) aufgearbeitet, so daß die gewünschte Verbindung, d.h. 7-Fluoro-6-(3-chloro-4,5,6,7-tetrahydro-2H-indazol-2-yl)-4-(pyridin-2-ylmethyl)-2H-1,4-benzoxazin-3(4H)on (0,8 g) erhalten wurde; Schmp. 198-201 °C.

Beispiel 9

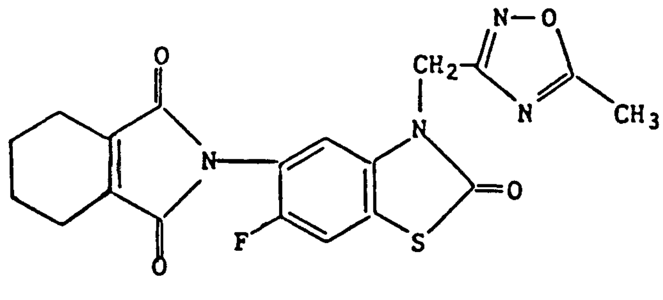

Eine Mischung aus 5-Amino-6-fluoro-3-(5-methyl-1,2,4-oxadiazol-3-ylmethyl)-2(3H)-benzothiazolon (2,8 g) und Essigsäure (20 ml) wurde mit 3,4,5,6-Tetrahydrophthalsäureanhydrid (1,6 g) versetzt, 30 min bei einer Temperatur von 20 °C bis 30 °C gerührt und dann 2 h unter Rückfluß erhitzt. Die Reaktionsmischung wurde zur Entfernung der Essigsäure unter vermindertem Druck destilliert, und der Rückstand wurde chromatographisch unter Verwendung einer Silicagel-Säule und eines Elutionsmittels aus einem Tetrahydrofuran/Toluol-Gemisch (1/3-Gemisch) aufgearbeitet, so daß die gewünschte Verbindung, d.h. 6-Fluoro-3-(5-methyl-1,2,4-oxadiazol-3-ylmethyl)-5-(4,5,6,7-tetrahydro-2H-isoindol-1,3-dion-2-yl)-2(3H)-benzothiazolon (2,9 g) erhalten wurde; Schmp. 198-199 °C.

Beispiel 10

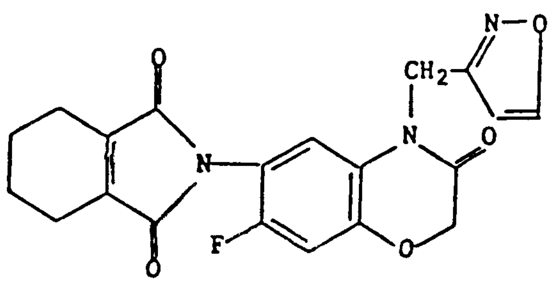

Die Verbindung 3-(Chloromethyl)-isoxazol (1,2 g) wurde tropfenweise bei einer Temperatur von 20 °C bis 30 °C unter Rühren zu einer Mischung aus 7-Fluoro-6-(4,5,6,7-tetrahydro-2H-isoindol-1,3-dion-2-yl)-2H-1,4-benzoxazin-3(4H)-on (3,16 g), Acetonitril (50 ml) und Kaliumcarbonat (1,5 g) hinzugefügt. Die Reaktionsmischung wurde 3 h unter Rückfluß erhitzt, auf Raumtemperatur gekühlt und filtriert. Das Filtrat wurde unter vermindertem Druck zur Trockne konzentriert. Der erhaltene Rückstand wurde mit Toluol (150 ml) vermischt, wodurch eine Suspension gebildet wurde, die dann zur Entfernung der ungelösten Stoffe filtriert wurde. Das Filtrat wurde unter vermindertem Druck konzentriert, wodurch ein viskoses Material erhalten wurde, das in einer minimalen Menge Ethanol gelöst wurde. Die Ethanol-Lösung wurde gekühlt, wonach ein kristallines Produkt ausfiel. Dieses Produkt wurde durch Filtration abgetrennt und getrocknet, wonach die gewünschte Verbindung, d.h. 7-Fluoro-4-(isoxazol-3-ylmethyl)-6-(4,5,6,7-tetrahydro-2H-isoindol-1,3-dion-2-yl)-2H-1,4-benzoxazin-3(4H)-on (3,3 g) erhalten wurde; Schmp. 206-210 °C.

Beispiel 11

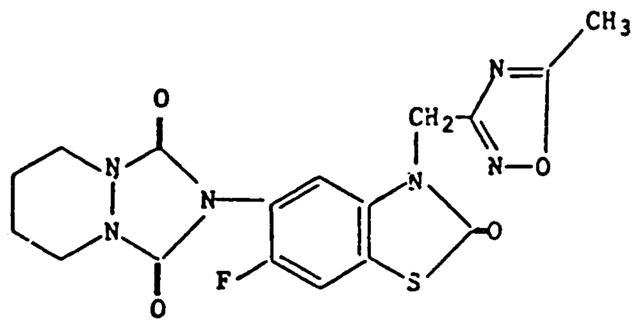

Die Verbindung 5-Isocyanato-6-fluoro-4-(5-methyl-1,2,4-oxadiazol-3-ylmethyl)-2(3H)-benzothiazolon (0,73 g) wurde in Tetrahydrofuran (15 ml) gelöst. Zu der entstandenen Lösung wurde tropfenweise 1-Ethoxycarbonylhexahydropyridazin (0,4 g) bei Raumtemperatur hinzugefügt. Man ließ die Reaktionsmischung über Nacht stehen und destillierte sie dann zur Entfernung des Lösungsmittels, so daß ein poröses Material (1,08 g) erhalten wurde. Dieses Material (1 g) wurde in Ethanol (30 ml) gelöst, und die erhaltene Lösung wurde mit einer katalytischen Menge Natriumethoxid versetzt, 1,5 h unter Rückfluß erhitzt und dann auf eine Temperatur von 0 °C bis 10 °C gekühlt. Das dabei gebildete kristalline Produkt wurde mittels Filtration abgetrennt, mit einer kleinen Menge von kaltem Ethanol gewaschen und getrocknet, so daß die gewünschte Verbindung, d.h. 6-Fluoro-4-(5-methy-1-1,2,4-oxadiazo-3-ylmethyl)-5-(1,2-tetramethylen-1,2,4-triazolidin-3,5-dion-4-yl)-2(3H)-benzothiazolon (0,75 g) erhalten wurde; Schmp. 251-255 °C.

Im Verfahren des Beispiels 11 kann das oben genannte Isocyanat-Derivat durch 5-Isothiocyanato-6-fluoro-4-(5-methyl-1,2,4-oxadiazol-3-ylmethyl)-2(3H)-benzothiazolon ersetzt werden, so daß die gewünschte Verbindung, d.h. 6-Fluoro-5-(1,2-tetramethylen-1,2,4-triazolidin-3-on-5-thion-4-yl)-4-(5-methyl-1,2,4-oxadiazol-3-ylmethyl)-2(3H)-benzothiazolon in hoher Ausbeute erhalten werden kann.

Beispiel 12

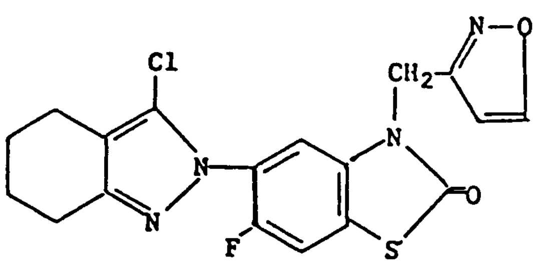

Eine Mischung aus 6-Fluoro-5-hydrazino-3-(isoxazol-3-ylmethyl)-2(3H)-benzothiazolon (2,8 g), 2-Ethoxycarbonyl-cyclohexanon (1,8 g), Natriumethoxid (0,03 g) und Ethanol (40 ml) wurde 5 h unter Rückfluß erhitzt. Das Ethanol wurde abdestilliert, und der Rückstand wurde mit Toluol (50 ml) versetzt. Die so gebildete Lösung wurde 10 min unter Rückfluß erhitzt und dann auf eine Temperatur von 0 °C bis 10 °C abgekühlt, worauf sich ein festes Produkt abschied, das durch Filtration gesammelt und mit n-Hexan gewaschen wurde. Die angestrebte Zwischenverbindung, d.h. 6-Fluoro-5-(3,3a,4,5,6,7-hexahydro-2H-indol-3-on-2-yl)-3-(isoxazol-3-ylmethyl)-2(3H)-benzothiazolon (3,1 g) wurde erhalten.

Die gesamte Menge der Zwischenverbindung wurde mit Toluol (100 ml), Trichloromethyl-chlorameisensäureester (1,5 g) und N.N-Dimethylformamid (0,05 g) versetzt, und die erhaltene Mischung wurde 5 h unter Rückfluß erhitzt. Die Komponenten mit niedrigeren Siedepunkten wurden abdestilliert, und der Rückstand wurde mit Ethylacetat (150 ml) versetzt. Die entstandene Lösung wurde mit einer wäßrigen Natriumhydrogencarbonat-Lösung gewaschen, mit wasserfreiem Natriumsulfat getrocknet und zur Entfernung des Lösungsmittels destilliert.

Der Rückstand wurde mittels Silicagel-Säulenchromatographie (Elutionsmittel: Tetrahydrofuran/Toluol =

1/4) aufgearbeitet, so daß die gewünschte Verbindung, d.h. 6-Fluoro-5-(3-chloro-4,5,6,7-tetrahydro-2H-indol-2-yl)-3-(isoxazol-3-ylmethyl)-2(3H)-benzothiazolon (1,8 g) als poröses Material erhalten wurde.

Nach den gleichen Arbeitsweisen wie in den vorstehenden Beispielen können die Verbindungen der Formel (I) erhalten werden, die, zusammen mit den in den Beispielen 1 bis 12 erhaltenen Verbindungen, in den folgenden Tabellen 1 bis 14 aufgeführt sind.

## Tabelle 1

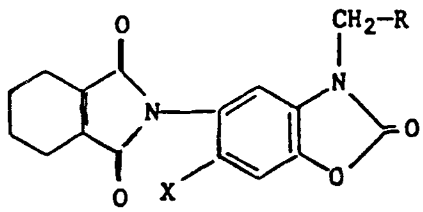

| Verbindung Nr. | X | R | |
|---|---|---|---|
| 1 | F | 5-methoxy-1,2,4-thiadiazol-3-yl | |
| 2 | F | 5-ethoxy-1,2,4-thiadiazol-3-yl | |
| 3 | F | 5-propoxy-1,2,4-thiadiazol-3-yl | |
| 4 | F | 5-isopropoxy-1,2,4-thiadiazol-3-yl | |
| 5 | F | 5-butoxy-1,2,4-thiadiazol-3-yl | |
| 6 | F | 5-dimethylamino-1,2,4-thiadiazol-3-yl | |
| 7 | F | 1,2,5-thiadiazol-3-yl | |
| 8 | F | 3-chloro-1,2,5-thiadiazol-4-yl | |
| 9 | F | 3-methyl-1,2,5-thiadiazol-4-yl | |
| 10 | F | 2-amino-1,3,4-thiadiazol-5-yl | |
| 11 | F | 2-chloro-1,3,4-thiadiazol-5-yl | |
| 12 | F | 2-methoxy-1,3,4-thiadiazol-5-yl | |
| 13 | F | 2-methylthio-1,3,4-thiadiazol-5-yl | |
| 14 | F | 2-methyl-1,3,4-thiadiazol-5-yl | |
| 15 | F | 1,3-thiazol-2-yl | |
| 16 | F | 5-chloro-1,3-thiadiazol-2-yl | |

Tabelle 1 - Fortsetzung

| Verbindung Nr. | X | R | |
|---|---|---|---|
| 17 | F | 4,5-dichloro-1,3-thiadiazol-2-yl | |
| 18 | F | 3-methyl-1,3-thiazol-2-yl | |
| 19 | F | 5-chloro-4-methyl-1,3-thiazol-2-yl | |
| 20 | F | 4,5-dimethyl-1,3-thiazol-2-yl | |
| 21 | F | 1,3-thiazol-4-yl | |
| 22 | F | 2-amino-1,3-thiazol-4-yl | |
| 23 | F | 2-acetamido-1,3-thiazol-4-yl | |
| 24 | F | 2-chloro-1,3-thiazol-4-yl | |
| 25 | F | 2-methyl-1,3-thiazol-4-yl | |
| 26 | F | 2-trifluoromethyl-1,3-thiazol-4-yl | |
| 27 | F | 2-ethyl-1,3-thiazol-4-yl | |
| 28 | F | 1,2,3-thiadiazol-4-yl | |
| 29 | F | 5-chloro-1,2,3-thiadiazol-4-yl | |
| 30 | F | 5-methyl-1,2,3-thiadiazol-4-yl | |
| 31 | F | 5-methyl-1,2,4-oxadiazol-3-yl | Schmp. 188-193°C |
| 32 | F | 5-methoxymethyl-1,2,4-oxadiazol-3-yl | |
| 33 | F | 5-ethyl-1,2,4-oxadiazol-3-yl | |
| 34 | F | 3-methyl-1,2,4-oxadiazol-5-yl | |
| 35 | F | 3-trifluoromethyl-1,2,4-oxadiazol-5-yl | |
| 36 | F | 3-ethyl-1,2,4-oxadiazol-5-yl | |
| 37 | F | 3-isopropyl-1,2,4-oxadiazol-5-yl | |
| 38 | F | 3-methyl-1,2,5-oxadiazol-4-yl | |
| 39 | F | 1,3,4-oxadiazol-2-yl | |

Tabelle 1 - Fortsetzung

| Verbindung Nr. | X | R | |
|---|---|---|---|
| 40 | F | 2-methyl-1,3,4-oxadiazol-5-yl | |
| 41 | F | 2-ethyl-1,3,4-oxadiazol-5-yl | |
| 42 | F | 1,2-thiazol-3-yl | |
| 43 | F | 4-chloro-1,2-thiazol-3-yl | |
| 44 | F | 4-bromo-1,2-thiazol-3-yl | |
| 45 | F | 2-furyl | |
| 46 | F | 3-furyl | |
| 47 | F | 2-thienyl | |
| 48 | F | 3-thienyl | |
| 49 | F | 1-methylpyrazol-3-yl | |
| 50 | F | 4,5-dichloro-1-methylpyrazol-3-yl | |
| 51 | F | 1-methylimidazol-2-yl | |
| 52 | F | 1-methylimidazol-4-yl | |
| 53 | F | 1-methylimidazol-5-yl | |
| 54 | F | 1-methyl-1,2,4-triazol-3-yl | |
| 55 | F | 1-methyl-1,3,4-triazol-2-yl | |
| 56 | F | 1-methyl-1,2,3,4-tetrazol-5-yl | |
| 57 | F | 1,2-oxazol-3-yl | Schmp. 189-191.5°C |
| 58 | F | 5-methyl-1,2-oxazol-3-yl | |
| 59 | F | 3-methyl-1,2-oxazol-5-yl | |
| 60 | H | 1-methyl-1,2,4-triazol-3-yl | Schmp. 134-139°C |
| 61 | H | 1,2-oxazol-3-yl | |
| 62 | H | 5-methyl-1,2-oxazol-3-yl | |

Tabelle 1 - Fortsetzung

| Verbindung Nr. | X | R | |
|---|---|---|---|
| 63 | H | 5-methyl-1,2,4-oxadiazol-3-yl | |
| 64 | H | 3-methyl-1,2,4-oxadiazol-5-yl | |
| 65 | H | 3-methyl-1,2,5-oxadiazol-4-yl | |
| 66 | H | 1,3,4-oxadiazol-2-yl | |
| 67 | H | 2-methyl-1,3,4-oxadiazol-5-yl | |
| 68 | H | 1,3-thiazol-4-yl | |
| 69 | H | 2-methyl-1,3-thiazol-4-yl | |
| 70 | H | 5-methoxy-1,2,4-thiadiazol-3-yl | |
| 71 | H | 1,2,5-thiadiazol-3-yl | |
| 72 | H | 3-methyl-1,2,5-thiadiazol-4-yl | |
| 73 | H | 2-methyl-1,3,4-thiadiazol-5-yl | |

Tabelle 2

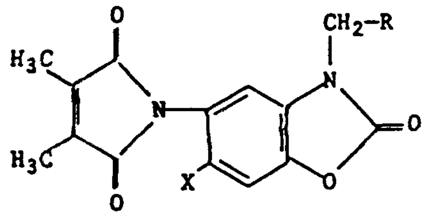

| Verbindung Nr. | X | R | |
|---|---|---|---|
| 74 | F | 1-methyl-1,2,4-triazol-3-yl | |
| 75 | F | 1,2-oxazol-3-yl | Schmp. 154-155°C |
| 76 | F | 5-methyl-1,2-oxazol-3-yl | |
| 77 | F | 5-methyl-1,2,4-oxazol-3-yl | Schmp. 180-182°C |
| 78 | F | 3-methyl-1,2,4-oxazol-5-yl | |
| 79 | F | 1,3-thiazol-4-yl | |
| 80 | F | 2-methyl-1,3-thiazol-4-yl | |
| 81 | F | 2-methyl-1,3,4-thiadiazol-5-yl | |
| 82 | H | 1-methyl-1,2,4-triazol-3-yl | |
| 83 | H | 1,2-oxazol-3-yl | Schmp. 190-191°C |
| 84 | H | 5-methyl-1,2-oxazol-3-yl | |
| 85 | H | 5-methyl-1,2,4-oxazol-3-yl | |
| 86 | H | 3-methyl-1,2,4-oxazol-5-yl | |
| 87 | H | 1,3-thiazol-4-yl | |
| 88 | H | 2-methyl-1,3-thiazol-4-yl | |
| 89 | H | 2-methyl-1,3,4-thiadiazol-5-yl | |

## Tabelle 3

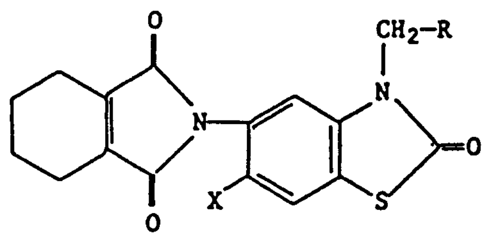

| Verbindung Nr. | X | R | |
|---|---|---|---|
| 90 | F | 5-methoxy-1,2,4-thiadiazol-3-yl | amorph |
| 91 | F | 5-ethoxy-1,2,4-thiadiazol-3-yl | Schmp. 83-89°C |
| 92 | F | 5-propoxy-1,2,4-thiadiazol-3-yl | |
| 93 | F | 5-isopropoxy-1,2,4-thiadiazol-3-yl | |
| 94 | F | 5-butoxy-1,2,4-thiadiazol-3-yl | |
| 95 | F | 5-dimethylamino-1,2,4-thiadiazol-3-yl | |
| 96 | F | 1,2,5-thiadiazol-3-yl | |
| 97 | F | 3-chloro-1,2,5-thiadiazol-4-yl | |
| 98 | F | 3-methyl-1,2,5-thiadiazol-4-yl | |
| 99 | F | 2-amino-1,3,4-thiadiazol-5-yl | |
| 100 | F | 2-chloro-1,3,4-thiadiazol-5-yl | |
| 101 | F | 2-methoxy-1,3,4-thiadiazol-5-yl | |
| 102 | F | 2-methylthio-1,3,4-thiadiazol-5-yl | |
| 103 | F | 2-methyl-1,3,4-thiadiazol-5-yl | |
| 104 | F | 1,3-thiazol-2-yl | |
| 105 | F | 5-chloro-1,3-thiadiazol-2-yl | |

Tabelle 3 - Fortsetzung

| Verbindung Nr. | X | R | |
|---|---|---|---|
| 106 | F | 4,5-dichloro-1,3-thiadiazol-2-yl | |
| 107 | F | 3-methyl-1,3-thiazol-2-yl | |
| 108 | F | 5-chloro-4-methyl-1,3-thiazol-2-yl | |
| 109 | F | 4,5-dimethyl-1,3-thiazol-2-yl | |
| 110 | F | 1,3-thiazol-4-yl | Schmp. 168-170°C |
| 111 | F | 2-amino-1,3-thiazol-4-yl | |
| 112 | F | 2-acetamido-1,3-thiazol-4-yl | |
| 113 | F | 2-chloro-1,3-thiazol-4-yl | |
| 114 | F | 2-methyl-1,3-thiazol-4-yl | |
| 115 | F | 2-trifluoromethyl-1,3-thiazol-4-yl | |
| 116 | F | 2-ethyl-1,3-thiazol-4-yl | |
| 117 | F | 1,2,3-thiadiazol-4-yl | |
| 118 | F | 5-chloro-1,2,3-thiadiazol-4-yl | |
| 119 | F | 5-methyl-1,2,3-thiadiazol-4-yl | |
| 120 | F | 5-methyl-1,2,4-oxadiazol-3-yl | Schmp. 198-199°C |
| 121 | F | 5-methoxymethyl-1,2,4-oxadiazol-3-yl | |
| 122 | F | 5-ethyl-1,2,4-oxadiazol-3-yl | Schmp. 83-89°C |
| 123 | F | 3-methyl-1,2,4-oxadiazol-5-yl | Schmp. 191-193°C |
| 124 | F | 3-ethyl-1,2,4-oxadiazol-5-yl | |
| 125 | F | 3-trifluoromethyl-1,2,4-oxadiazol-5-yl | |
| 126 | F | 3-methoxymethyl-1,2,4-oxadiazol-5-yl | |

Tabelle 3 - Fortsetzung

| Verbindung Nr. | X | R | |
|---|---|---|---|
| 127 | F | 3-methyl-1,2,5-oxadiazol-4-yl | Schmp. 166-170°C |
| 128 | F | 1,3,4-oxadiazol-2-yl | |
| 129 | F | 2-methyl-1,3,4-oxadiazol-5-yl | |
| 130 | F | 2-ethyl-1,3,4-oxadiazol-5-yl | |
| 131 | F | 1,2-thiadiazol-3-yl | |
| 132 | F | 4-chloro-1,2-thiazol-3-yl | |
| 133 | F | 4-bromo-1,2-thiazol-3-yl | |
| 134 | F | 2-furyl | |
| 135 | F | 3-furyl | |
| 136 | F | 2-thienyl | |
| 137 | F | 3-thienyl | |
| 138 | F | 1-methylpyrazol-3-yl | |
| 139 | F | 4,5-dichloro-1-methylpyrazol-3-yl | |
| 140 | F | 1-methyl-imidazol-2-yl | |
| 141 | F | 1-methyl-imidazol-4-yl | |
| 142 | F | 1-methyl-imidazol-5-yl | |
| 143 | F | 1-methyl-1,2,4-triazol-3-yl | |
| 144 | F | 1-methyl-1,3,4-triazol-2-yl | |
| 145 | F | 1-methyl-1,2,3,4-tetrazol-5-yl | |
| 146 | F | 1,2-oxazol-3-yl | Schmp. 196-200°C |
| 147 | F | 5-methyl-1,2-oxazol-3-yl | |
| 148 | F | 3-methyl-1,2-oxazol-5-yl | amorph |

Tabelle 3 - Fortsetzung

| Verbindung Nr. | X | R | |
|---|---|---|---|
| 149 | H | 1-methyl-1,2,4-triazol-3-yl | |
| 150 | H | 1,2-oxazol-3-yl | |
| 151 | H | 5-methyl-1,2-oxazol-3-yl | |
| 152 | H | 5-methyl-1,2,4-oxadiazol-3-yl | |
| 153 | H | 3-methyl-1,2,4-oxadiazol-5-yl | |
| 154 | H | 1,3-thiazol-4-yl | |
| 155 | H | 2-methyl-1,3-thiazol-4-yl | |
| 156 | H | 2-methyl-1,3,4-thiadiazol-5-yl | |
| 157 | F | 3-isopropyl-1,2,4-oxadiazol-5-yl | |
| 158 | F | 2-tetrahydrofuryl | $n_D^{20}$ 1.5675 |
| 159 | F | pyridin-2-yl | Schmp. 176-180°C |
| 160 | F | pyridazin-3-yl | |
| 161 | F | pyrimidin-4-yl | |
| 162 | F | pyrazin-2-yl | Schmp. 100-102°C |
| 163 | F | pyrimidin-2-yl | |
| 164 | F | 1,3,5-triazin-2-yl | |
| 165 | F | cyclopropyl | Schmp. 110-115°C |
| 166 | F | 1,2,4-triazol-1-yl | Schmp. 188-192°C |

## Tabelle 4

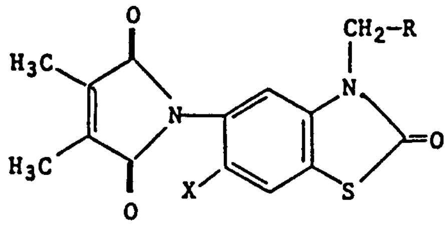

| Verbindung Nr. | X | R | |
|---|---|---|---|
| 167 | F | 1-methyl-1,2,4-triazol-3-yl | |
| 168 | F | 1,2-oxazol-3-yl | |
| 169 | F | 1,2-oxazol-5-yl | Schmp. 219-220°C |
| 170 | F | 5-methyl-1,2,4-oxazol-3-yl | |
| 171 | F | 3-methyl-1,2,4-oxazol-5-yl | |
| 172 | F | 1,3-thiazol-4-yl | |
| 173 | F | 2-methyl-1,3-thiazol-4-yl | |
| 174 | F | 2-methyl-1,3,4-thiadiazol-5-yl | |
| 175 | F | cyclopropyl | Schmp. 171-173°C |
| 176 | F | pyridin-2-yl | Schmp. 180-188°C |

Tabelle 5

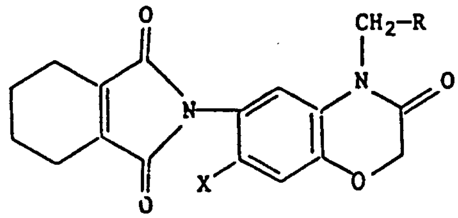

| Verbindung Nr. | X | R | |
|---|---|---|---|
| 177 | F | 2-tetrahydrofuryl | |
| 178 | F | 5-methoxy-1,2,4-thiadiazol-3-yl | amorph |
| 179 | F | 5-ethoxy-1,2,4-thiadiazol-3-yl | Schmp. 121-124°C |
| 180 | F | 5-propoxy-1,2,4-thiadiazol-3-yl | |
| 181 | F | 5-isopropoxy-1,2,4-thiadiazol-3-yl | |
| 182 | F | 5-dimethylamino-1,2,4-thiadiazol-3-yl | |
| 183 | F | 1,2,5-thiadiazol-3-yl | Schmp. 215-220°C |
| 184 | F | 3-chloro-1,2,5-thiadiazol-4-yl | |
| 185 | F | 3-methyl-1,2,5-thiadiazol-4-yl | |
| 186 | F | 2-amino-1,3,4-thiadiazol-5-yl | |
| 187 | F | 2-chloro-1,3,4-thiadiazol-5-yl | |
| 188 | F | 2-methoxy-1,3,4-thiadiazol-5-yl | |
| 189 | F | 2-methylthio-1,3,4-thiadiazol-5-yl | |
| 190 | F | 2-methyl-1,3,4-thiadiazol-5-yl | |
| 191 | F | 1,3-thiazol-2-yl | |
| 192 | F | 5-chloro-1,3-thiadiazol-2-yl | |

Tabelle 5 - Fortsetzung

| Verbindung Nr. | X | R | |
|---|---|---|---|
| 193 | F | 4,5-dichloro-1,3-thiadiazol-2-yl | |
| 194 | F | 3-methyl-1,3-thiazol-2-yl | |
| 195 | F | 5-chloro-4-methyl-1,3-thiazol-2-yl | |
| 196 | F | 4,5-dimethyl-1,3-thiazol-2-yl | |
| 197 | F | 1,3-thiazol-4-yl | Schmp. 200-202°C |
| 198 | F | 2-amino-1,3-thiazol-4-yl | |
| 199 | F | 2-acetamido-1,3-thiazol-4-yl | |
| 200 | F | 2-chloro-1,3-thiazol-4-yl | |
| 201 | F | 2-methyl-1,3-thiazol-4-yl | |
| 202 | F | 2-trifluoromethyl-1,3-thiazol-4-yl | |
| 203 | F | 2-ethyl-1,3-thiazol-4-yl | |
| 204 | F | 1,2,3-thiadiazol-4-yl | |
| 205 | F | 5-chloro-1,2,3-thiadiazol-4-yl | |
| 206 | F | 5-methyl-1,2,3-thiadiazol-4-yl | |
| 207 | F | 5-methyl-1,2,4-oxadiazol-3-yl | Schmp. 200-205°C |
| 208 | F | 5-methoxymethyl-1,2,4-oxadiazol-3-yl | |
| 209 | F | 5-ethyl-1,2,4-oxadiazol-3-yl | Schmp. 74-79°C |
| 210 | F | 3-propyl-1,2,4-oxadiazol-5-yl | |
| 211 | F | 3-methyl-1,2,4-oxadiazol-5-yl | Schmp. 196-201°C |
| 212 | F | 3-trifluoromethyl-1,2,4-oxadiazol-5-yl | |
| 213 | F | 3-ethyl-1,2,4-oxadiazol-5-yl | Schmp. 74-79°C |

Tabelle 5 - Fortsetzung

| Verbindung Nr. | X | R | |
|---|---|---|---|
| 193 | F | 4,5-dichloro-1,3-thiadiazol-2-yl | |
| 194 | F | 3-methyl-1,3-thiazol-2-yl | |
| 195 | F | 5-chloro-4-methyl-1,3-thiazol-2-yl | |
| 196 | F | 4,5-dimethyl-1,3-thiazol-2-yl | |
| 197 | F | 1,3-thiazol-4-yl | Schmp. 200-202°C |
| 198 | F | 2-amino-1,3-thiazol-4-yl | |
| 199 | F | 2-acetamido-1,3-thiazol-4-yl | |
| 200 | F | 2-chloro-1,3-thiazol-4-yl | |
| 201 | F | 2-methyl-1,3-thiazol-4-yl | |
| 202 | F | 2-trifluoromethyl-1,3-thiazol-4-yl | |
| 203 | F | 2-ethyl-1,3-thiazol-4-yl | |
| 204 | F | 1,2,3-thiadiazol-4-yl | |
| 205 | F | 5-chloro-1,2,3-thiadiazol-4-yl | |
| 206 | F | 5-methyl-1,2,3-thiadiazol-4-yl | |
| 207 | F | 5-methyl-1,2,4-oxadiazol-3-yl | Schmp. 200-205°C |
| 208 | F | 5-methoxymethyl-1,2,4-oxadiazol-3-yl | |
| 209 | F | 5-ethyl-1,2,4-oxadiazol-3-yl | Schmp. 74-79°C |
| 210 | F | 3-propyl-1,2,4-oxadiazol-5-yl | |
| 211 | F | 3-methyl-1,2,4-oxadiazol-5-yl | Schmp. 196-201°C |
| 212 | F | 3-trifluoromethyl-1,2,4-oxadiazol-5-yl | |
| 213 | F | 3-ethyl-1,2,4-oxadiazol-5-yl | Schmp. 74-79°C |

Tabelle 5 - Fortsetzung

| Verbindung Nr. | X | R | |
|---|---|---|---|
| 235 | F | 3-methyl-1,2-oxazol-5-yl | Schmp. 194-195°C |
| 236 | H | 1-methyl-1,2,4-triazol-3-yl | Schmp. 219-225°C |
| 237 | H | 1,2-oxazol-3-yl | Schmp. 208-212°C |
| 238 | H | 5-methyl-1,2-oxazol-3-yl | Schmp. 189-195°C |
| 239 | H | 5-methyl-1,2,4-oxadiazol-3-yl | Schmp. 160-167°C |
| 240 | H | 3-methyl-1,2,4-oxadiazol-5-yl | Schmp. 195-199°C |
| 241 | H | 1,3-thiazol-4-yl | Schmp. 140-143°C |
| 242 | H | 2-methyl-1,3-thiazol-4-yl | |
| 243 | H | 2-methyl-1,3,4-thiadiazol-5-yl | |
| 244 | F | 3-isopropyl-1,2,4-oxadiazol-5-yl | amorph |
| 245 | H | 5-ethoxy-1,2,4-thiadiazol-3-yl | Schmp. 112-125°C |
| 246 | H | 4-methyl-1,2,5-oxadiazol-3-yl | Schmp. 194-197°C |
| 247 | H | pyridin-2-yl | Schmp. 185-188°C |
| 248 | F | pyridin-2-yl | Schmp. 188-190°C |
| 249 | F | 6-chloropyridin-2-yl | Schmp. 97-110°C |
| 250 | F | 4-chloropyridin-2-yl | |
| 251 | F | 4-fluoropyridin-2-yl | |
| 252 | F | 5-chloropyridin-2-yl | |
| 253 | F | 4-methylpyridin-2-yl | |
| 254 | F | 5-methylpyridin-2-yl | |
| 255 | F | pyridazin-3-yl | |
| 256 | F | pyrimidin-4-yl | |

Tabelle 5 - Fortsetzung

| Verbindung Nr. | X | R | |
|---|---|---|---|
| 257 | F | pyrazin-2-yl | Schmp. 191-194°C |
| 258 | F | pyrimidin-2-yl | |
| 259 | F | 4-chloro-6-methylpyrimidin-2-yl | |
| 260 | F | 4,6-dimethylpyrimidin-2-yl | |
| 261 | F | 1,3,5-triazin-2-yl | |

<u>Tabelle 6</u>

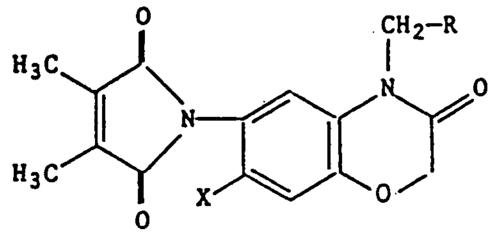

| Verbindung Nr. | X | R | |
|---|---|---|---|
| 262 | F | 1-methyl-1,2,4-triazol-3-yl | |
| 263 | F | 1,2-oxazol-3-yl | Schmp. 256-258° C |
| 264 | F | 5-methyl-1,2-oxazol-3-yl | |
| 265 | F | 5-methyl-1,2,4-oxadiazol-3-yl | Schmp. 184-190° C |
| 266 | F | 5-ethyl-1,2,4-oxadiazol-3-yl | Schmp. 105-115°C |
| 267 | F | 3-methyl-1,2,5-oxadiazol-4-yl | |
| 268 | F | 1,3,4-oxadiazol-2-yl | |
| 269 | F | 2-methyl-1,3,4-oxadiazol-5-yl | |
| 270 | F | 1,3-thiazol-4-yl | Schmp. 172-174°C |
| 271 | F | 2-methyl-1,3-thiazol-4-yl | Schmp. 137-141°C |
| 272 | F | 5-methoxy-1,2,4-thiadiazol-3-yl | Schmp. 145-150°C |
| 273 | F | 1,2,5-thiadiazol-3-yl | |
| 274 | F | 3-methyl-1,2,5-thiadiazol-4-yl | |
| 275 | F | 2-methyl-1,3,4-thiadiazol-5-yl | |
| 276 | H | 1-methyl-1,2,4-triazol-3-yl | |
| 277 | H | 1,2-oxazol-3-yl | |

Tabelle 6 - Fortsetzung

| Verbindung Nr. | X | R | |
|---|---|---|---|
| 278 | H | 5-methyl-1,2-oxazol-3-yl | |
| 279 | H | 5-methyl-1,2,4-oxazol-3-yl | |
| 280 | H | 3-methyl-1,2,4-oxazol-5-yl | Schmp. 209-215°C |
| 281 | H | 1,3-thiazol-4-yl | |
| 282 | H | 2-methyl-1,3-thiazol-4-yl | |
| 283 | H | 2-methyl-1,3,4-thiadiazol-5-yl | |
| 284 | F | pyridin-2-yl | Schmp. 140-141°C |

Tabelle 7

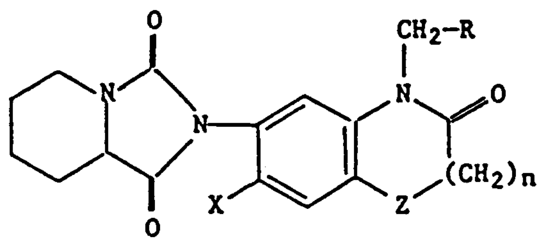

| Verbindung Nr. | X | Z | n | R | |
|---|---|---|---|---|---|
| 285 | H | O | 0 | 1,2-oxazol-5-yl | |
| 286 | F | O | 0 | 1,2-oxazol-5-yl | |
| 287 | H | O | 1 | 1,2-oxazol-5-yl | |
| 288 | H | O | 1 | 5-methyl-1,2,4-oxadiazol-3-yl | |
| 289 | F | O | 1 | 1,2-oxazol-3-yl | Schmp. 155-162°C |
| 290 | F | O | 1 | 5-methyl-1,2,4-oxadiazol-3-yl | amorph |
| 291 | F | S | 0 | 1,2-oxazol-3-yl | Schmp. 187-188°C |
| 292 | F | S | 0 | 5-methyl-1,2,4-oxadiazol-3-yl | Schmp. 120-125°C |
| 293 | H | O | 1 | pyridin-2-yl | |
| 294 | F | O | 1 | pyridin-2-yl | Schmp. 127-128°C (Zers.) |

# Tabelle 8

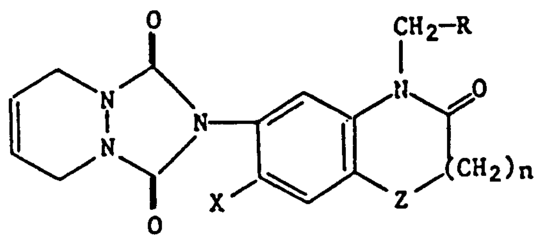

| Verbindung Nr. | X | Z | n | R | |
|---|---|---|---|---|---|
| 295 | H | O | O | 1,2-oxazol-3-yl | Schmp. 214-215°C |
| 296 | H | O | 1 | 1,2-oxazol-3-yl | Schmp. 248-250°C |
| 297 | H | O | 1 | 5-methyl-1,2,4-oxadiazol-3-yl | |
| 298 | F | O | 1 | 1,2-oxazol-3-yl | Schmp. 248-251°C |
| 299 | F | O | 1 | 5-methyl-1,2,4-oxadiazol-3-yl | Schmp. 180-181°C |
| 300 | F | S | 0 | 1,2-oxazol-3-yl | Schmp. 140-143°C |
| 301 | F | S | O | 5-methyl-1,2,4-oxadiazol-3-yl | Schmp. 225-227°C |
| 302 | H | O | 1 | pyridin-2-yl | |
| 303 | F | O | 1 | pyridin-2-yl | Schmp. 167-169°C |

## Tabelle 9

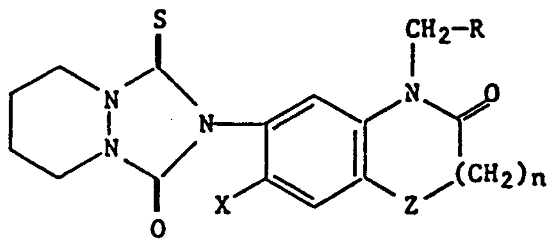

| Verbindung Nr. | X | Z | n | R | |
|---|---|---|---|---|---|
| 304 | F | O | 1 | 1,2-oxazol-3-yl | Schmp. 225-227°C |
| 305 | F | O | 1 | 5-methyl-1,2,4-oxadiazol-3-yl | Schmp. 266-272°C |
| 306 | F | S | 0 | 1,2-oxazol-3-yl | Schmp. 254-256°C |
| 307 | F | S | 0 | 5-methyl-1,2,4-oxadiazol-3-yl | Schmp. 202-206°C |
| 308 | H | O | 1 | pyridin-2-yl | |
| 309 | F | O | 1 | pyridin-2-yl | Schmp. 163-168°C |

Tabelle 10

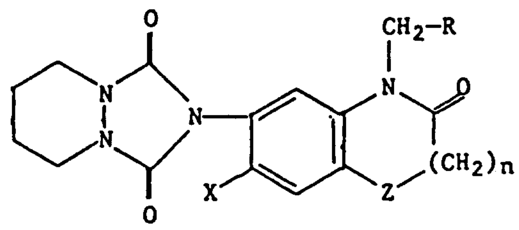

| Verbindung Nr. | X | Z | n | R | |
|---|---|---|---|---|---|
| 310 | H | O | 0 | 1,2-oxazol-3-yl | Schmp. 178-179°C |
| 311 | H | O | 1 | 1,2-oxazol-3-yl | Schmp. 239-243°C |
| 312 | H | O | 1 | 5-methyl-1,2,4-oxadiazol-3-yl | Schmp. 221-222°C |
| 313 | F | O | 1 | 1,2-oxazol-3-yl | Schmp. 233-234°C |
| 314 | F | O | 1 | 5-methyl-1,2,4-oxadiazol-3-yl | Schmp. 227-228°C |
| 315 | F | S | 0 | 1,2-oxazol-3-yl | Schmp. 237-238°C |
| 316 | F | S | 0 | 5-methyl-1,2,4-oxadiazol-3-yl | Schmp. 251-255°C |
| 317 | H | O | 1 | pyridin-2-yl | |
| 318 | F | O | 1 | pyridin-2-yl | Schmp. 200-210°C |

Tabelle 11

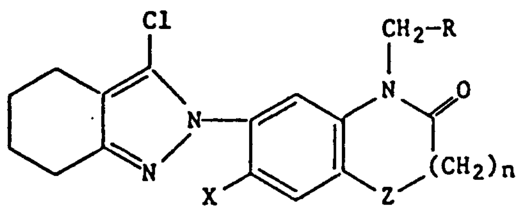

| Verbindung Nr. | X | Z | n | R | |
|---|---|---|---|---|---|
| 319 | H | O | 1 | 1,2-oxazol-3-yl | |
| 320 | H | O | 1 | 5-methyl-1,2,4-oxadiazol-3-yl | |
| 321 | F | O | 1 | 1,2-oxazol-3-yl | |
| 322 | F | O | 1 | 5-methyl-1,2,4-oxadiazol-3-yl | |
| 323 | F | S | 0 | 1,2-oxazol-3-yl | |
| 324 | F | S | 0 | 5-methyl-1,2,4-oxadiazol-3-yl | amorph |
| 325 | H | O | 1 | pyridin-2-yl | amorph |
| 326 | F | O | 1 | pyridin-2-yl | Schmp. 198-201°C |

Tabelle 12

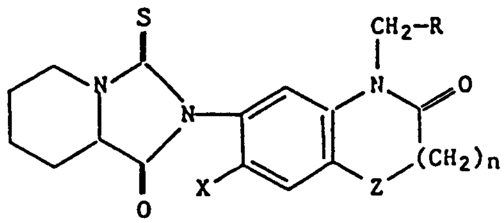

| Verbindung Nr. | X | Z | n | R | |
|---|---|---|---|---|---|
| 327 | H | O | 1 | pyridin-2-yl | Schmp. 218-222°C |
| 328 | F | O | 1 | pyridin-2-yl | amorph |
| 329 | F | S | 0 | 1,2-oxazol-3-yl | amorph |
| 330 | F | O | 1 | 1,2-oxazol-3-yl | Schmp. 186-192°C |

Tabelle 13

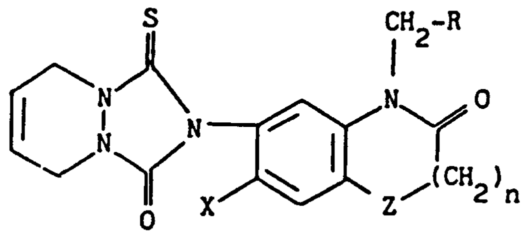

| Verbindung Nr. | X | Z | n | R | |
|---|---|---|---|---|---|
| 331 | H | O | 1 | pyridin-2-yl | Schmp. 222-224°C |
| 332 | F | O | 1 | pydirin-2-yl | |
| 333 | F | S | 0 | 1,2-oxazol-3-yl | Schmp. 246-247°C |
| 334 | F | S | O | 5-methyl-1,2,4-oxadiazol-3-yl | Schmp. 225-227°C |
| 335 | F | O | 1 | 1,2-oxadiazol-3-yl | Schmp. 252-253°C |

## Tabelle 14

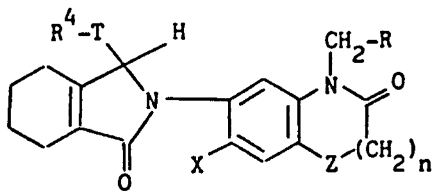

| Verbindung Nr. | $R^4$-T | X | Z | n | R | |
|---|---|---|---|---|---|---|
| 336 | -OH | F | O | 1 | pyridin-2-yl | amorph |
| 337 | Cl | F | O | 1 | pyridin-2-yl | (Hydrochlorid) ) amorph |
| 338 | $-O\overset{\overset{O}{\|}}{C}CH_3$ | F | O | 1 | pyridin-2-yl | $n_D^{20}$ 1.5725 |
| 339 | $-O\overset{\overset{O}{\|}}{C}C_2H_5$ | F | O | 1 | pyridin-2-yl | |
| 340 | $-OCH_3$ | F | O | 1 | pyridin-2-yl | |
| 341 | $-OC_2H_5$ | F | O | 1 | pyridin-2-yl | amorph |
| 342 | $-SCH_3$ | F | O | 1 | pyridin-2-yl | |
| 343 | $-SC_2H_5$ | F | O | 1 | pyridin-2-yl | |
| 344 | $-N(CH_3)_2$ | F | O | 1 | pyridin-2-yl | |

Beispiel 13

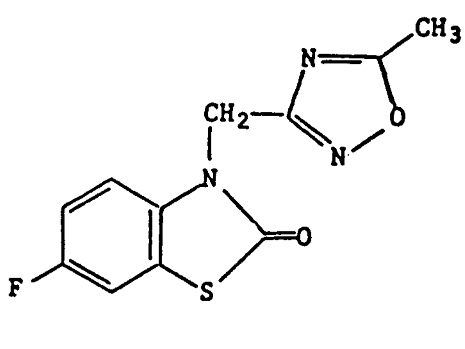

Eine Mischung aus 6-Fluoro-2(3H)-benzothiazolon (8,45 g), Acetonitril (100 ml) und Kaliumcarbonat (7,6 g) wurde hergestellt. Zu dieser Mischung wurde tropfenweise 3-Chloromethyl-5-methyl-1,2,4-oxadiazol (7,3 g) bei einer Temperatur von 40 °C bis 50 °C unter Rühren hinzugefügt. Die Reaktionsmischung wurde 5 h

unter Rückfluß erhitzt. Die Reaktionsmischung wurde dann abgekühlt und filtriert. Das Filtrat wurde unter vermindertem Druck destilliert, und der Rückstand wurde mit Ethylacetat (200 ml) und Wasser (100 ml) versetzt. Die resultierende organische Schicht wird abgetrennt und mit einer 5-proz. wäßrigen Kaliumhydroxid-Lösung, mit Wasser und dann mit einer gesättigten wäßrigen Natriumchlorid-Lösung gewaschen und über wasserfreiem Natriumsulfat getrocknet. Das Lösungsmittel wurde abdestilliert, und der Rückstand wurde in einer minimalen Menge Ethanol unter Erhitzen gelöst. Die so gebildete Lösung wurde auf eine Temperatur von 0 °C bis 5 °C gekühlt, wodurch ein kristallines Produkt erhalten wurde. Dieses wurde dann durch Filtration abgetrennt, wodurch die gewünschte Verbindung, d.h. 6-Fluoro-3-(5-methyl-1,2,4-oxadiazol-3-ylmethyl)-2(3H)-benzothiazolon, (12,1 g) erhalten wurde; Schmp. 142-144 °C.

Beispiel 14

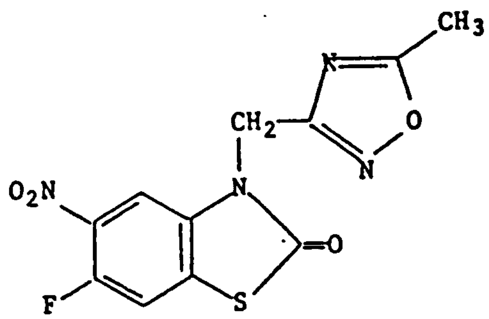

Die Verbindung 6-Fluoro-3-(5-methyl-1,2,4-oxadiazol-3-ylmethyl)-2(3H)-benzothiazolon (11,5 g) wurde portionsweise zu konz. Schwefelsäure (200 g) hinzugefügt, die auf eine Temperatur von 5 °C bis 10 °C gekühlt worden war. Die Reaktionsmischung wurde 10 min bei einer Temperatur von 0 °C bis 5 °C gerührt. Zu der Reaktionsmischung wurde tropfenweise 98-proz. Salpetersäure (3 g) hinzugefügt. Die Reaktionsmischung wurde 2 h bei einer Temperatur von 5 °C bis 10 °C gerührt und dann auf Eis gegossen. Ethylacetat (1 Liter) wurde zu der Mischung gegeben, und das Ganze wurde gerührt. Die Ethylacetat-Schicht wurde abgetrennt, mit Wasser, mit einer gesättigten wäßrigen Natriumhydrogencarbonat-Lösung und dann mit einer gesättigten wäßrigen Natriumchlorid-Lösung gewaschen und über wasserfreiem Natriumsulfat getrocknet. Das Lösungsmittel wurde abdestilliert, und der Rückstand wurde aus Ethanol umkristallisiert, so daß das gewünschte Produkt, d.h. 6-Fluoro-3-(5-methyl-1,2,4-oxadiazol-3-ylmethyl)-5-nitro-2(3H)-benzothiazolon, (13,0 g) erhalten wurde; Schmp. 192-193 °C.

Beispiel 15

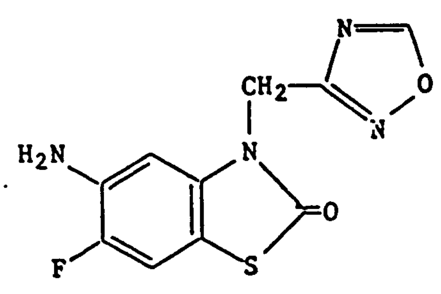

Eine Mischung aus 6-Fluoro-3-(5-methyl-1,2,4-oxadiazol-3-ylmethyl)-5-nitro-2(3H)-benzothiazolon (3,1 g) und Ethanol (40 ml) wurde hergestellt. Zu dieser Mischung wurde tropfenweise eine Lösung von Zinn(II)-chlorid (3,3 Stoffmengen-Äquivalente pro Stoffmengen-Äquivalent der Nitro-Verbindung) in konz. Salzsäure (40 ml) bei einer Temperatur von 10 °C bis 15 °C hinzugefügt. Die Reaktionsmischung wurde 1,5 h bei einer Temperatur von 60 °C bis 70 °C gerührt und unter vermindertem Druck zur Entfernung des Ethanols und der Salzsäure destilliert. Der resultierende Rückstand wurde mit Eis (20 g) versetzt. Zu der dadurch gebildeten Lösung wurde tropfenweise eine 25-proz. wäßrige Kaliumhydroxid-Lösung bei einer Temperatur

von 10 °C bis 15 °C hinzugefügt, um die Mischung alkalisch zu machen. Die Mischung wurde mit Dichloromethan (2 x 100 ml) extrahiert. Der Dichloromethan-Extrakt wurde mit Wasser gewaschen, über wasserfreiem Natriumsulfat getrocknet und unter vermindertem Druck eingedampft, wonach die gewünschte Verbindung, d.h. 5-Amino-6-fluoro-3-(5-methyl-1,2,4-oxadiazol-3-ylmethyl)-2(3H)-benzothiazolon, (2,2 g) erhalten wurde; Schmp. 158-161 °C.

Es ist ebenfalls möglich, die oben bezeichnete Verbindung mittels eines anderen Herstellungsverfahrens herzustellen, wie nachstehend gezeigt wird.

6-Fluoro-2(3H)-benzothiazolon (82,9 g) wurde in konz. Schwefelsäure (800 g) mit 98-proz. Salpetersäure (5 % Überschuß über die theoretische Menge) 1,5 h bei einer Temperatur von 0 °C bis 10 °C unter Rühren nitriert. Die Reaktionsmischung wurde in Eiswasser gegossen und filtriert. Der durch die Filtration abgetrennte feste Stoff wurde getrocknet und aus Aceton umkristallisiert, so daß 6-Fluoro-5-nitro-2(3H)-benzothiazolon (67,7 g) erhalten wurde. Die Reinheit dieser Verbindung betrug 92 % (die Reinheit wurde mit einer HPLC-Methode bestimmt). Die Gesamtmenge dieser Verbindung wurde mit Eisen-Pulver reduziert, wodurch 5-Amino-6-fluoro-2(3H)-benzothiazolon (52,4 g) erhalten wurde. 4 g der Amino-Verbindung wurden einer Alkylierungs-Operation mit Hilfe von 3-Chloromethyl-5-methyl-1,2,4-oxadiazol (2,9 g) in Acetonitril (100 ml) in Gegenwart von Kaliumcarbonat (3 g) als Dehydrochlorierungsmittel untervorfen. Diese Operation wurde 2 h unter Rückfluß durchgeführt, so daß die gewünschte Verbindung, d.h. 5-Amino-6-fluoro-3-(5-methyl-1,2,4-oxadiazol-3-ylmethyl)-2(3H)-benzothiazolon, (5,3 g) erhalten wurde; Schmp. 156-157 °C.

Beispiel 16

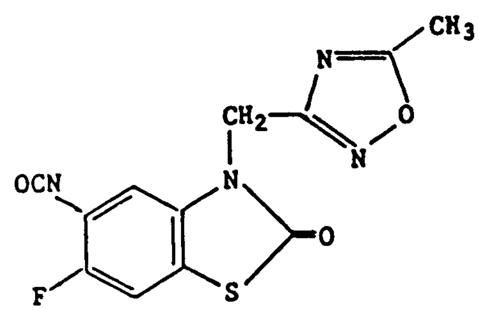

Eine Mischung aus 5-Amino-6-fluoro-3-(5-methy1-1,2,4-oxadiazol-3-ylmethyl)-2(3H)-benzothiazolon (1,33 g) und Dioxan (23 g) wurde hergestellt. Zu dieser Mischung wurde tropfenweise Trichloromethyl-chlorameisensäureester (1 g) hinzugefügt. Die Reaktionsmischung wurde 3 h unter Rückfluß erhitzt. Die Komponenten mit niedrigeren Siedepunkten wurden abdestilliert, und der Rückstand wurde in Toluol (50 ml) gelöst. Die resultierende Lösung wurde unter vermindertem Druck zur Trockne konzentriert, so daß die gewünschte Verbindung, d.h. 6-Fluoro-5-isocyanato-3-(5-methyl-1,2,4-oxadiazol-3-ylmethyl)-2(3H)-benzothiazolon, (1,46 g) erhalten wurde; Schmp. 125-127 °C.

Beispiel 17

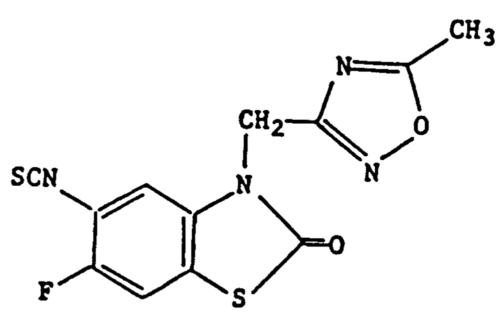

Eine Mischung aus 5-Amino-6-fluoro-3-(5-methyl-1,2,4-oxadiazol-3-ylmethyl)-2(3H)-benzothiazolon (1,33 g), Dichloromethan (10 ml) und Eis (10 g) wurde hergestellt. Zu dieser Mischung wurden Thiophosgen (0,7 g) und dann portionsweise Natriumhydrogencarbonat bei einer Temperatur von 5 °C bis 10 °C hinzugegeben, um die Mischung leicht alkalisch zu machen. Die Mischung wurde 20 min gerührt, und dann wurde geprüft, ob die Mischung alkalisch war (falls die Mischung sauer war, wurde eine weitere Menge Natriumhydrogencarbonat zugegeben). Die Dichloromethan-Schicht wurde abgetrennt, und die wäßrige Schicht wurde mit frischem Dichloromethan (2 x 10 ml) extrahiert. Die DichloromethanSchichten wurden vereinigt, mit Wasser gewaschen und zur Entfernung des Dichloromethan-Lösungsmittels destilliert. Der verbleibende feste Rückstand wurde aus einem Toluol-Hexan-Lösungsmittelgemisch umkristallisiert, so daß die gewünschte Verbindung, d.h. 6-Fluoro-5-isothiocyanato-3-(5-methyl-1,2,4-oxadiazol-3-ylmethyl)-2(3H)-benzothiazolon, (1,49 g) erhalten wurde; Schmp. 168-170 °C.

Beispiel 18

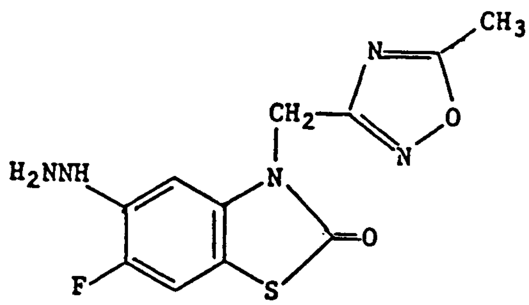

5-Amino-6-fluoro-3-(5-methyl-1,2,4-oxadiazol-3-ylmethyl)-2(3H)-benzothiazolon (1,6 g) wurde zu einer Mischung von konz. Salzsäure (5 ml) und Wasser (5 ml) hinzugefügt. Zu der entstandenen Lösung wurde tropfenweise eine wäßrige Lösung von salpetriger Säure (0,45 g) bei einer Temperatur von 0 °C bis 5 °C hinzugefügt. Die Reaktionsmischung wurde zur Vervollständigung der Diazotierungs-Reaktion 30 min bei 0 °C bis 5 °C gerührt.

Die Reaktionsmischung, die das diazotierte Produkt enthielt, wurde tropfenweise bei einer Temperatur von 0 °C bis 5 °C Zu einer Mischung von Zinn(II)-chloriddihydrat (4,23 g) und konz. Salzsäure (6 ml) hinzugefügt. Danach wurde die Reaktionsmischung 30 min bei einer Temperatur von 0 °C bis 5 °C gerührt. Zu der Reaktionsmischung wurde tropfenweise eine 25-proz. wäßrige Kaliumhydroxid-Lösung gegeben, um die Mischung alkalisch zu machen, und die Mischung wurde mit Dichloromethan (2 x 20 ml) extrahiert. Die abgetrennte Dichloromethan-Schicht wurde mit Wasser gewaschen und über wasserfreiem Natriumsulfat getrocknet. Das Lösungsmittel wurde abdestilliert, so daß das gewünschte Produkt, d.h. 6-Fluoro-5-hydrazino-3-(5-methyl-1,2,4-oxadiazol-3-ylmethyl)-2(3H)-benzothiazolon, (1,4 g) erhalten wurde; Schmp. 190-192 °C (Zers.).

Beispiel 19

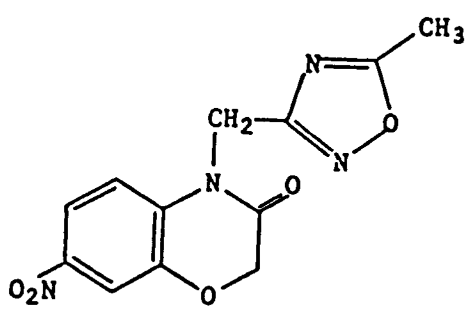

Eine Mischung aus 7-Nitro-2H-1,4-benzoxazin-3(4H)-on (3,88 g) (diese Verbindung kann mittels eines in J. Chem. Soc. 1928, S. 3046, oder in Synthesis 1982, S. 986, aufgezeigten Verfahrens hergestellt werden),

Kaliumcarbonat (3,1 g) und Acetonitril (80 ml) wurde hergestellt. Zu dieser Mischung wurde tropfenweise 3-Chloromethyl-5-methyl-1,2,4-oxadiazol (2,92 g) bei einer Temperatur von 40 °C bis 50 °C hinzugefügt. Die Reaktionsmischung wurde kontinuierlich 4 h unter Rückfluß gerührt und dann gekühlt. Danach wurde das Lösungsmittel unter vermindertem Druck abdestilliert, und der Rückstand wurde mit Ethylacetat (150 ml) und Wasser (50 ml) vermischt. Die organische Schicht wurde abgetrennt, mit einer 5-proz. wäßrigen Kaliumhydroxid-Lösung, mit Wasser und dann mit einer gesättigten wäßrigen Natriumchlorid-Lösung gewaschen und über wasserfreiem Natriumsulfat getrocknet. Das Lösungsmittel wurde unter vermindertem Druck abdestilliert, und der so erhaltene Rückstand wurde aus Toluol umkristallisiert, so daß die gewünschte Verbindung, d.h. 4-(5-Methyl-1,2,4-oxadiazol-3-ylmethyl)-7-nitro-2H-1,4-benzoxazin-3(4H)-on, (3,22 g) erhalten wurde. Schmp. 182-183 °C.

Beispiel 20

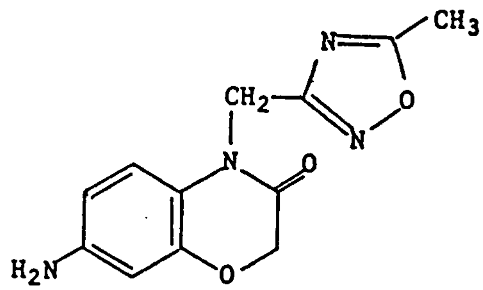

Eine Mischung aus 4-(5-Methyl-1,2,4-oxadiazol-3-ylmethyl)-7-nitro-2H-1,4-benzoxazin-3(4H)-on (3,2 g) und Ethanol (40 ml) wurde hergestellt. Zu dieser Mischung wurde eine Lösung von Zinn(II)-chlorid-dihydrat (11,33 g) in konz. Salzsäure (44 ml) bei einer Temperatur von 10 bis 20 °C hinzugefügt. Die Reaktionsmischung wurde bei einer Temperatur von 60 °C bis 70 °C gerührt und unter vermindertem Druck zur Trockne destilliert. Der verbleibende Rückstand wird mit Eis (150 g) vermischt. Zu der auf diese Weise erhaltenen Mischung wurde tropfenweise eine 25-proz. wäßrige Kaliumhydroxid-Lösung bei einer Temperatur von 0 °C bis 10 °C hinzugefügt, um die Mischung alkalisch zu machen.

Die resultierende alkalische Lösung wurde mit Dichloromethan (2 x 80 ml) extrahiert. Die Dichloromethan-Schicht wurde mit Wasser gewaschen und über wasserfreiem Natriumsulfat getrocknet. Das Dichloromethan-Lösungsmittel wurde unter vermindertem Druck abdestilliert, und der verbleibende feste Rückstand (1,3 g) wurde aus Toluol umkristallisiert, so daß die gewünschte Verbindung, d.h. 7-Amino-4-(5-methyl-1,2,4-oxadiazol-3-ylmethyl)-2H-1,4-benzoxazin-3(4H)-on, (1,1 g) erhalten wurde; Schmp. 146-147,5 °C.

Beispiel 21

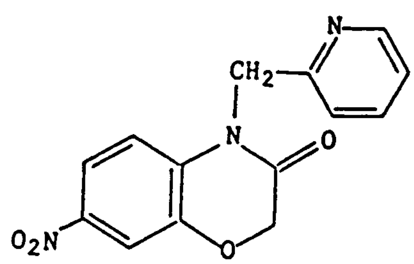

Eine Mischung aus 7-Nitro-2H-1,4-benzoxazin-3(4H)-on (1,94 g), Kaliumcarbonat (15,18 g) und Acetonitril (200 ml) wurde 30 min bei 60 °C gerührt, auf eine Temperatur von 20 °C bis 30 °C gekühlt und tropfenweise mit 2-(Chloromethyl)pyridin (14 g) versetzt. Die Reaktionsmischung wurde 30 min auf eine Temperatur von 60 °C bis 65 °C und danach weitere 3 h zum Rückfluß erhitzt, auf eine Temperatur von 20

° C bis 30 ° C abgekühlt und filtriert.

Das resultierende Filtrat wurde unter vermindertem Druck destilliert, um die niedriger siedenden Substanzen zu entfernen. Der verbleibende Rückstand wurde in Dichloromethan (200 ml) gelöst und mit einer 5-proz. wäßrigen Kaliumhydroxid-Lösung und dann mit Wasser gewaschen und über wasserfreiem Natriumsulfat getrocknet. Das Dichloromethan wurde abdestilliert. Der dabei erhaltene Rückstand wurde aus Ethanol umkristallisiert, so daß die gewünschte Verbindung, d.h. 7-Nitro-4-(pyridin-2-ylmethyl)-2H-1,4-benzoxazin-3(4H)-on, (23,1 g) erhalten wurde; Schmp. 175-176 ° C.

Beispiel 22

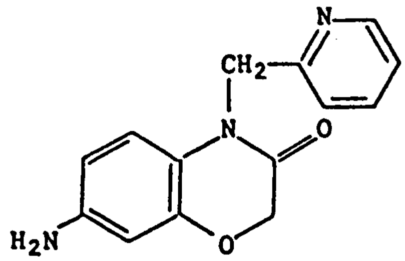

Eine Mischung aus Eisen-Pulver (19 g), Wasser (100 ml), Essigsäure (5,7 g) und Ethanol (50 ml) wurde bei einer Temperatur von 70 ° C bis 80 ° C gerührt. Zu dieser Mischung wurde portionsweise die Verbindung aus Beispiel 21 (16 g) hinzugegeben. Die Reaktionsmischung wurde weiterhin mit Ethanol (50 ml) versetzt und 1 h zum Rückfluß erhitzt. Eine beträchtliche Menge Ethanol wurde unter Atmosphärendruck abdestilliert. Dann wurde die Reaktionsmischung mit Dichloromethan (200 ml) versetzt und genügend gerührt und dann auf eine Temperatur von 10 ° C bis 20 ° C abgekühlt. Danach wurde die Mischung mit Hilfe von Celite filtriert, und der Filterkuchen wurde mit Dichloromethan (2 x 50 ml) gewaschen. Die organische Schicht wurde aus dem Filtrat und der Waschflüssigkeit abgetrennt, mit einer gesättigten wäßrigen Natriumhydrogencarbonat-Lösung gewaschen und über wasserfreiem Natriumsulfat getrocknet. Das Lösungsmittel wurde abdestilliert, und der Rückstand wurde aus einem Toluol-Hexan(3:1)-Lösungsmittelgemisch umkristallisiert, so daß die gewünschte Verbindung, d.h. 7-Amino-4-(pyridin-2-ylmethyl)-2H-1,4-benzoxazin-3(4H)-on,(12 g) erhalten wurde; Schmp. 140-141 ° C.

Beispiel 23

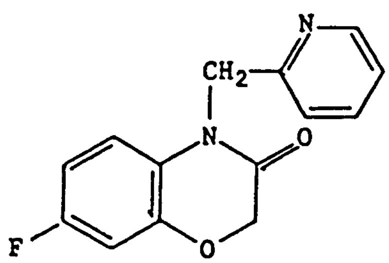

Die Verbindung (4,13 g) aus Beispiel 22 wurde in einer Mischung aus konzentrierter Salzsäure (5 g) und Wasser (1,2 g) gelöst. Zu der entstandenen Lösung wurde bei einer Temperatur von 0 ° C bis 5 ° C portionsweise Natriumnitrit (1,2 g) hinzugefügt. Die Reaktionsmischung wurde 30 min bei 0 ° C bis 5 ° C gerührt und tropfenweise mit 42-proz. Borfluorvasserstoffsäure (7 g) versetzt, 30 min bei 0 ° C bis 5 ° C und weiter 2 h bei 20 ° C bis 30 ° C gerührt und wiederum auf 0 ° C bis 5 ° C gekühlt. Das dabei gebildete kristalline Produkt wurde durch Filtration abgetrennt, mit Eiswasser (20 ml) gewaschen und an der Luft getrocknet.

Das so erhaltene getrocknete feste Produkt (5,5 g) wurde in Dichlorobenzol (50 ml) suspendiert, und diese Suspension wurde 30 min unter Rückfluß erhitzt. Das Dichlorobenzol und die Substanzen mit

niedrigeren Siedepunkten wurden unter vermindertem Druck abdestilliert, und der erhaltene Rückstand wurde mit einer 5-proz. wäßrigen Kaliumhydroxid-Lösung (200 ml) versetzt. Die so gebildete Mischung wurde 1 h bei einer Temperatur von 20 °C bis 30 °C gerührt und dann mit Ethylacetat (200 ml) versetzt, um eine Extraktionsoperation vorzunehmen. Der Ethylacetat-Extrakt wurde über wasserfreiem Natriumsulfat getrocknet. Das Lösungsmittel wurde abdestilliert, und der Rückstand wurde mit heißem Hexan extrahiert. Der Hexan-Extrakt wurde auf eine Temperatur von 5 °C bis 10 °C gekühlt, und das resultierende kristalline Produkt wurde durch Filtration abgetrennt, so daß die gewünschte Verbindung, d.h. 7-Fluoro-4-(pyridin-2-ylmethyl)-2H-1,4-benzoxazin-3(4H)-on, (1,9 g) erhalten wurde; Schmp. 82-84 °C.

Beispiel 24

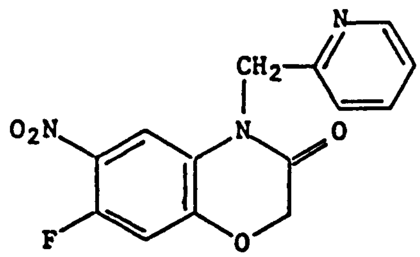

Die Verbindung (8,96 g) aus Beispiel 23 wurde portionsweise zu konzentrierter Schwefelsäure (100 g) hinzugegeben, die auf eine Temperatur von 0 °C bis 5 °C gekühlt worden war. Die resultierende Lösung wurde 10 min gerührt und dann tropfenweise mit 98-proz. Salpetersäure (2,34 g) bei einer Temperatur von 0 °C bis 5 °C versetzt. Die Reaktionsmischung wurde 1 h bei einer Temperatur von 5 °C bis 10 °C gerührt und auf Eis (300 g) gegossen. Dann wurde die Reaktionsmischung mit Natriumhydrogencarbonat neutralisiert und mit Ethylacetat (250 ml) extrahiert. Die organische Schicht wurde abgetrennt, mit Wasser gewaschen und über wasserfreiem Natriumsulfat getrocknet. Das Lösungsmittel wurde abdestilliert, und der Rückstand wurde aus Ethanol umkristallisiert, so daß die gewünschte Verbindung, d.h. 7-Fluoro-6-nitro-4-(pyridin-2-ylmethyl)-2H-1,4-benzoxazin-3(4H)-on, (5,8 g) erhalten wurde; Schmp. 129-130 °C.

Beispiel 25

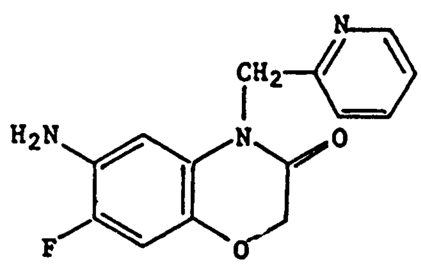

Die Verbindung (12,56 g) aus Beispiel 24 wurde nach einer Arbeitsweise, die der in Beispiel 22 gezeigten ähnelt, mit Hilfe von Eisen-Pulver (13,3 g), Wasser (80 ml), Essigsäure (4 g) und Ethanol (100 ml) reduziert. Die gewünschte Verbindung, d.h. 6-Amino-7-fluoro-4-(pyridin-2-ylmethyl)-2H-1,4-benzoxazin-3-(4H)-on, (10,4 g) wurde erhalten; Schmp. 149-152 °C.

Beispiel 26

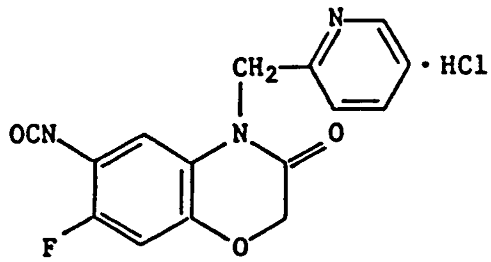

Die Verbindung (5 g) aus Beispiel 25 wurde in Dioxan (60 ml) gelöst. Zu dieser Lösung wurde tropfenweise Trichloromethyl-chlorameisensäureester (4 g) bei einer Temperatur von 10 °C bis 20 °C hinzugefügt. Die Reaktionsmischung wurde 5 h unter Rühren zum Rückfluß erhitzt und dann auf eine Temperatur von 20 °C bis 25 °C gekühlt. Der feste Anteil wurde durch Filtration abgetrennt und mit Toluol (2 x 10 ml) gewaschen, so daß die gewünschte Verbindung, d.h. 7-Fluoro-6-isocyanato-4-(pyridin-2-ylmethyl)-2H-1,4-benzoxazin-3(4H)-on-hydrochlorid, (6,1 g) erhalten wurde; Schmp. 300-305 °C.

Beispiel 27

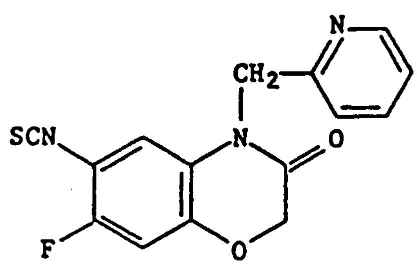

Eine Mischung aus der Verbindung (2,73 g) aus Beispiel 25, Dichloromethan (30 ml) und Wasser (25 ml) wurde auf eine Temperatur von 5 °C bis 15 °C gekühlt. Zu dieser Mischung wurde tropfenweise Thiophosgen (1,3 g) hinzugegeben. Danach wurde Natriumhydrogencarbonat portionsweise zu der Reaktionsmischung hinzugegeben, bis die Reaktionsmischung schwache Alkalität zeigte. Die Reaktionsmischung wurde 30 min bei einer Temperatur von 10 °C bis 20 °C gerührt. Die organische Schicht wurde abgetrennt und über wasserfreiem Natriumsulfat getrocknet. Das Lösungsmittel wurde abdestilliert, und der Rückstand wurde aus einem Toluol/n-Hexan-Gemisch umkristallisiert, so daß die gewünschte Verbindung, d.h. 7-Fluoro-6-isothiocyanato-4-(pyridin-2-ylmethyl)-2H-1,4-benzoxazin-3(4H)-on, (2,3 g) erhalten wurde; Schmp. 106-109 °C.

Beispiel 28

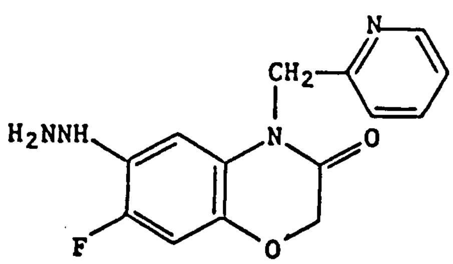

Eine Mischung aus der Verbindung (5,46 g) aus Beispiel 25, konzentrierter Salzsäure (10 ml) und

Wasser (10 ml) wurde hergestellt. Zu dieser Mischung wurde bei einer Temperatur von 0 °C bis 5 °C portionsweise Natriumnitrit (1,4 g) hinzugefügt. Die Mischung wurde 30 min bei einer Temperatur von 0 °C bis 5 °C gerührt.

Die resultierende diazotierte Flüssigkeit wurde tropfenweise zu einer Lösung aus Zinn(II)-chloriddihydrat (14 g) und konzentrierter Salzsäure (14 ml) hinzugefügt.

Die Reaktionsmischung wurde 30 min bei einer Temperatur von 0 °C bis 5 °C gerührt und bei einer Temperatur unterhalb von 10 °C tropfenweise mit einer 40-proz. wäßrigen Kaliumhydroxid-Lösung versetzt, so daß die Reaktionsmischung alkalisch wurde. Dann wurde die Reaktionsmischung mit Dichloromethan (200 ml) versetzt, mehrere Minuten gerührt und filtriert. Die abgetrennte Dichloromethan-Schicht wurde mit Wasser gewaschen und über wasserfreiem Natriumsulfat getrocknet. Das Lösungsmittel wurde abdestilliert, und der Rückstand wurde aus Ethanol umkristallisiert, so daß die gewünschte Verbindung, d.h. 7-Fluoro-6-hydrazino-4-(pyridin-2-ylmethyl)-2H-1,4-benzoxazin-3(4H)-on, (3,5 g) erhalten wurde; Schmp. 150-154 °C.

Nach den gleichen Arbeitsweisen wie in den vorstehenden Beispielen 13 bis 28 können die Zwischenprodukte für die Verbindungen der Formel (I) erhalten werden, die, zusammen mit den in den Beispielen 13 bis 28 erhaltenen Zwischenprodukten, in der folgenden Tabelle 15 aufgeführt sind.

Tabelle 15

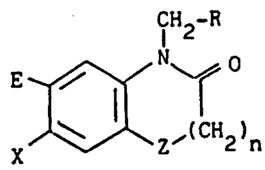

| Zwischenprodukt | E | X | Z | n | R | Schmp. °C |
|---|---|---|---|---|---|---|
| 1 | $-NH_2$ | H | O | 0 | cyclopropyl | |
| 2 | $-NH_2$ | F | O | 0 | cyclopropyl | |
| 3 | $-NH_2$ | F | S | 0 | cyclopropyl | |
| 4 | -NCO | F | S | 0 | cyclopropyl | |
| 5 | -NCS | F | S | 0 | cyclopropyl | |
| 6 | $-NH_2$ | H | O | 1 | cyclopropyl | |
| 7 | H | $H_2N$ | O | 1 | cyclopropyl | 127-129 |
| 8 | H | F | O | 1 | cyclopropyl | 55- 56 |
| 9 | $-NO_2$ | F | O | 1 | cyclopropyl | |
| 10 | $-NH_2$ | F | O | 1 | cyclopropyl | |
| 11 | -NCO | F | O | 1 | cyclopropyl | |
| 12 | -NCS | F | O | 1 | cyclopropyl | |
| 13 | $-NH_2$ | H | O | 0 | isoxazol-3-yl | 127-130 |
| 14 | -NCO | H | O | 0 | isoxazol-3-yl | 96-101 |
| 15 | -NCS | H | O | 0 | isoxazol-3-yl | |
| 16 | $-NH_2$ | F | O | 0 | isoxazol-3-yl | 166-171 |
| 17 | -NCO | F | O | 0 | isoxazol-3-yl | |
| 18 | -NCS | F | O | 0 | isoxazol-3-yl | |
| 19 | H | F | S | 0 | isoxazol-3-yl | 94- 97 |

Tabelle 15 - Fortsetzung

| Zwischenprodukt | E | X | Z | n | R | Schmp. °C |
|---|---|---|---|---|---|---|
| 20 | $-NO_2$ | F | S | 0 | isoxazol-3-yl | 166-168 |
| 21 | $-NH_2$ | F | S | 0 | isoxazol-3-yl | 164-166 |
| 22 | -NCO | F | S | 0 | isoxazol-3-yl | 188-189 |
| 23 | -NCS | F | S | 0 | isoxazol-3-yl | 157-162 |
| 24 | $-NH_2$ | H | O | 1 | isoxazol-3-yl | 130-132 |
| 25 | -NCO | H | O | 1 | isoxazol-3-yl | Öl |
| 26 | -NCS | H | O | 1 | isoxazol-3-yl | |
| 27 | H | F | O | 1 | isoxazol-3-yl | 109.5-110.5 |
| 28 | $-NO_2$ | F | O | 1 | isoxazol-3-yl | 153-155 |
| 29 | $-NH_2$ | F | O | 1 | isoxazol-3-yl | 187-188 |
| 30 | -NCO | F | O | 1 | isoxazol-3-yl | 111-113 |
| 31 | -NCS | F | O | 1 | isoxazol-3-yl | 125-126 |
| 32 | $-NH_2$ | H | O | 0 | 5-methyl-1,2,4-oxadiazol-3-yl | |
| 33 | $-NH_2$ | F | O | 0 | 5-methyl-1,2,4-oxadiazol-3-yl | |
| 34 | H | F | S | 0 | 5-methyl-1,2,4-oxadiazol-3-yl | 142-144 |
| 35 | $-NO_2$ | F | S | 0 | 5-methyl-1,2,4-oxadiazol-3-yl | 192-193 |
| 36 | $-NH_2$ | F | S | 0 | 5-methyl-1,2,4-oxadiazol-3-yl | 156-157 |
| 37 | -NCO | F | S | 0 | 5-methyl-1,2,4-oxadiazol-3-yl | 125-127 |

Tabelle 15 - Fortsetzung

| Zwischenprodukt | E | X | Z | n | R | Schmp. °C |
|---|---|---|---|---|---|---|
| 38 | -NCS | F | S | 0 | 5-methyl-1,2,4-oxadiazol-3-yl | 168-170 |
| 39 | $-NHNH_2$ | F | S | 0 | 5-methyl-1,2,4-oxadiazol-3-yl | 190-192 (Zers.) |
| 40 | $-NH_2$ | H | O | 1 | 5-methyl-1,2,4-oxadiazol-3-yl | 178-181 |
| 41 | -NCO | H | O | 1 | 5-methyl-1,2,4-oxadiazol-3-yl | |
| 42 | -NCS | H | O | 1 | 5-methyl-1,2,4-oxadiazol-3-yl | |
| 43 | $-NH_2$ | F | O | 1 | 5-methyl-1,2,4-oxadiazol-3-yl | 154-157 |
| 44 | -NCO | F | O | 1 | 5-methyl-1,2,4-oxadiazol-3-yl | Öl |
| 45 | -NCS | F | O | 1 | 5-methyl-1,2,4-oxadiazol-3-yl | 110-110 |
| 46 | $-NH_2$ | H | O | 0 | 3-methyl-1,2,4-oxadiazol-5-yl | |
| 47 | $-NH_2$ | F | O | 0 | 3-methyl-1,2,4-oxadiazol-5-yl | |
| 48 | H | F | S | 0 | 3-methyl-1,2,4-oxadiazol-5-yl | 103-105 |
| 49 | $-NO_2$ | F | S | 0 | 3-methyl-1,2,4-oxadiazol-5-yl | 145-146 |
| 50 | $-NH_2$ | F | S | 0 | 3-methyl-1,2,4-oxadiazol-5-yl | |

Tabelle 15 - Fortsetzung

| Zwischenprodukt | E | X | Z | n | R | Schmp. °C |
|---|---|---|---|---|---|---|
| 51 | $-NH_2$ | H | O | 1 | 3-methyl-1,2,4-oxadiazol-5-yl | |
| 52 | $-NH_2$ | F | O | 1 | 3-methyl-1,2,4-oxadiazol-5-yl | |
| 53 | $-NH_2$ | H | O | 0 | 3-methyl-1,2,5-oxadiazol-5-yl | |
| 54 | $-NH_2$ | F | O | 0 | 3-methyl-1,2,5-oxadiazol-5-yl | |
| 55 | H | F | S | 0 | 3-methyl-1,2,5-oxadiazol-5-yl | |
| 56 | $-NO_2$ | F | S | 0 | 3-methyl-1,2,5-oxadiazol-5-yl | |
| 57 | $-NH_2$ | F | S | 0 | 3-methyl-1,2,5-oxadiazol-5-yl | |
| 58 | -NCO | F | S | 0 | 3-methyl-1,2,5-oxadiazol-5-yl | |
| 59 | -NCS | F | S | 0 | 3-methyl-1,2,5-oxadiazol-5-yl | |
| 60 | $-NH_2$ | H | O | 1 | 3-methyl-1,2,5-oxadiazol-5-yl | |
| 61 | $-NH_2$ | F | O | 1 | 3-methyl-1,2,5-oxadiazol-5-yl | |
| 62 | $-NH_2$ | F | O | 0 | thiazol-4-yl | |
| 63 | $-NH_2$ | F | S | 0 | thiazol-4-yl | 147-149 |
| 64 | $-NH_2$ | H | O | 1 | thiazol-4-yl | |
| 65 | $-NH_2$ | F | O | 1 | thiazol-4-yl | |
| 66 | $-NH_2$ | F | O | 0 | 1,2,5-thiadiazol-3-yl | |
| 67 | $-NH_2$ | F | S | 0 | 1,2,5-thiadiazol-3-yl | |
| 68 | $-NH_2$ | H | O | 1 | 1,2,5-thiadiazol-3-yl | |

Tabelle 15 - Fortsetzung

| Zwischenprodukt | E | X | Z | n | R | Schmp. °C |
|---|---|---|---|---|---|---|
| 69 | $-NH_2$ | F | O | 1 | 1,2,5-thiadiazol-3-yl | |
| 70 | $-NH_2$ | F | O | 0 | 5-methoxy-1,2,4-thiadiazol-3-yl | |
| 71 | $-NH_2$ | F | S | 0 | 5-methoxy-1,2,4-thiadiazol-3-yl | |
| 72 | $-NH_2$ | H | O | 1 | 5-methoxy-1,2,4-thiadiazol-3-yl | |
| 73 | $-NH_2$ | F | O | 1 | 5-methoxy-1,2,4-thiadiazol-3-yl | |
| 74 | $-NH_2$ | H | O | 0 | pyridin-2-yl | |
| 75 | $-NH_2$ | F | O | 0 | pyridin-2-yl | |
| 76 | H | F | S | 0 | pyridin-2-yl | 121-122.5 |
| 77 | $-NO_2$ | F | S | 0 | pyridin-2-yl | 166-171 |
| 78 | $-NH_2$ | F | S | 0 | pyridin-2-yl | 129-132 |
| 79 | -NCO | F | S | 0 | pyridin-2-yl | |
| 80 | -NCS | F | S | 0 | pyridin-2-yl | |
| 81 | $-NO_2$ | H | O | 1 | pyridin-2-yl | 141-143 |
| 82 | $-NH_2$ | H | O | 1 | pyridin-2-yl | 146-148 |
| 83 | -NCO | H | O | 1 | pyridin-2-yl | (Hydrochlorid) |
| 84 | -NCS | H | O | 1 | pyridin-2-yl | 118-120 |
| 85 | $-NHNH_2$ | H | O | 1 | pyridin-2-yl | 100-108 |
| 86 | H | F | O | 1 | pyridin-2-yl | 77- 78 |
| 87 | $-NO_2$ | F | O | 1 | pyridin-2-yl | 129-130 |

Tabelle 15 - Fortsetzung

| Zwischenprodukt | E | X | Z | n | R | Schmp. °C |
|---|---|---|---|---|---|---|
| 88 | $-NH_2$ | F | O | 1 | pyridin-2-yl | 150-151 |
| 89 | -NCO | F | O | 1 | pyridin-2-yl | 300-305 (Hydrochlorid) |
| 90 | -NCS | F | O | 1 | pyridin-2-yl | 106-109 |
| 91 | $-NHNH_2$ | F | O | 1 | pyridin-2-yl | 150-154 |
| 92 | $-NH_2$ | H | O | 0 | pyrazin-2-yl | |
| 93 | $-NH_2$ | F | O | 0 | pyrazin-2-yl | |
| 94 | $-NH_2$ | F | S | 0 | pyrazin-2-yl | |
| 95 | -NCO | F | S | 0 | pyrazin-2-yl | |
| 96 | -NCS | F | S | 0 | pyrazin-2-yl | |
| 97 | $-NH_2$ | H | O | 1 | pyrazin-2-yl | |
| 98 | $-NH_2$ | F | O | 1 | pyrazin-2-yl | |

Verwendungsbeispiele

**Vergleichs-Substanzen:**

**E-1:**

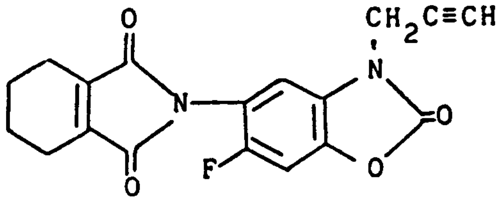

**(offenbart in der EP-OS 170 191, Verbindung Nr. 2)**

**E-2:**

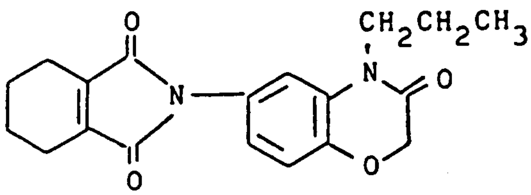

**(offenbart in der EP-OS 170 191, Verbindung Nr. 10)**

Beispiel 29

Test gegen Unkräuter in einem überfluteten Reisfeld durch Wasseroberflächen-Anwendung

Herstellung einer Wirkstoff-Formulierung

Träger: 5 Gewichtsteile Aceton;
Emulgator: 1 Gewichtsteil Benzyloxypolyglycolether.

Eine Wirkstoff-Formulierung in Form eines emulgierbaren Konzentrats wurde erhalten durch Vermischen von 1 Gewichtsteil der aktiven Verbindung mit den oben angegebenen Mengen des Trägers und des emulgierenden Mittels. Eine vorher festgelegte Menge des Präparats wurde mit Wasser verdünnt.

Test-Verfahren

Reis-Kulturboden wurde in Töpfe (5 $dm^2$; 25 cm x 20 cm x 9 cm) gefüllt, und Reis-Setzlinge (Varietät: "Nihonbare") im 2,5-Blattstadium (15 cm hoch) wurden jeweils an zwei Stellen pro Topf als Stock von drei Setzlingen umgepflanzt. Samen von Hühnerhirse (Echinochloa oryzicola Vasing.), Schirmkraut (Cyperus difformis L.), Monochoria (Monochoria vaginalis) sowie einjährigen breitblättrigen Unkräutern Falsche Pimpernelle (Lindernia pyxidaria L.), Rotala indica, Amerikanischer Wasserwurz (Elatine triandra), Rotstengel (Ammannia multiflora Roxburgh) und Dopatrium junceum Hamilton wurden eingesät, und die Töpfe wurden feucht gehalten. Zwei Tage später wurden die Töpfe bis zu einer Tiefe von etwa 2 bis 3 cm überflutet. Fünf Tage nach dem Umpflanzen der Setzlinge wurde die Verbindung der vorliegenden Erfindung, in Form eines wie im Vorstehenden hergestellten emulgierbaren Konzentrats, mittels einer Pipette in einer vorher festgelegten Menge auf die Oberfläche des Wassers aufgebracht. Danach wurde der Zustand der etwa 3 cm tiefen Überflutung aufrechterhalten, und vier Wochen nach der chemischen Behandlung wurden die herbizide Wirkung und die Phytotoxizität gegenüber Reis untersucht und mit Hilfe der folgenden Skala von 0 bis 5 bewertet:

| Bewertung | Herbizide Wirkung (bewertet als Unkrautvernichtungs-Verhältnis, bezogen auf eine unbehandelte Fläche) |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |

| Bewertung | Phytotoxizität gegenüber Reis (bewertet unter Bezug auf die unbehandelte Fläche) |
|---|---|
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Test-Ergebnisse sind in Tabelle 16 aufgeführt.

Tabelle 16

| Akt. Verbindung Nr. | Wirkstoff-Menge kg/ha | Herbizide Wirkung gegen Unkräuter Hühnerhirse | Schirmkraut | Monochoria | Breitblättrige Unkräuter | Phytotoxizität gegen Reis |
|---|---|---|---|---|---|---|
| 146 | 0,25 | 4 | 5 | 5 | 5 | 0 |
| | 0,125 | 3 | 5 | 4 | 5 | 0 |
| 159 | 0,25 | 5 | 5 | 5 | 5 | 2 |
| | 0,125 | 5 | 5 | 5 | 5 | 1 |
| | 0,06 | 5 | 5 | 5 | 5 | 0 |
| 183 | 0,25 | 5 | 5 | 5 | 5 | 1 |
| | 0,125 | 4 | 5 | 5 | 5 | 0 |
| | 0,06 | 4 | 5 | 4 | 5 | 0 |
| 197 | 0,25 | 5 | 5 | 5 | 5 | 1 |
| | 0,125 | 4 | 5 | 5 | 5 | 0 |
| 249 | 0,25 | 4 | 5 | 5 | 5 | 0 |
| | 0,125 | 3 | 4 | 4 | 5 | 0 |
| 257 | 0,25 | 4 | 5 | 5 | 5 | 0 |
| | 0,125 | 4 | 5 | 4 | 5 | 0 |
| 284 | 0,25 | 5 | 5 | 5 | 5 | 0 |
| | 0,125 | 4 | 5 | 5 | 5 | 0 |

Tabelle 16 - Fortsetzung

| Akt. Verbindung Nr. | Wirkstoff-Menge kg/ha | Herbizide Wirkung gegen Unkräuter Hühnerhirse | Schirmkraut | Monochoria | Breitblättrige Unkräuter | Phytotoxizität gegen Reis |
|---|---|---|---|---|---|---|
| Vergleichs-Verbindung | | | | | | |
| E-1 | 0,5 | 5 | 5 | 5 | 5 | 4 |
| | 0,25 | 4 | 5 | 3 | 4 | 3 |
| | 0,125 | 2 | 5 | 3 | 4 | 2 |
| E-2 | 0,25 | 3 | 5 | 4 | 4 | 3 |
| | 0,125 | 1 | 3 | 2 | 2 | 2 |

Beispiel 30

Test gegen Unkräuter auf höher gelegenem Ackerboden durch Bodenbehandlung vor dem Auflaufen:

In einem Gewächshaus wurden Sojabohnen-Samen in Töpfe von 500 $cm^2$ Fläche, die mit Ackererde gefüllt waren, eingesät, und Erde, die Samen von Hühnerhirse (Echinocloa crus-galli), grauem Amaranth (Amaranthus lividus L.) und Gänsefuß (Ghenopodium album L.) enthielt, wurde über die Erde in den Töpfen in einer Tiefe von 1 cm gestreut.

Einen Tag nach der Einsaat wurde eine Test-Chemikalie in einer vorher festgelegten Konzentration, hergestellt wie in Beispiel 29, gleichmäßig über die Oberflächenschicht des Bodens in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 29 geprüft. Die Ergebnisse sind in Tabelle 17 aufgeführt.

Tabelle 17

| Akt. Verbindung Nr. | Wirkstoff-Menge kg/ha | Herbizide Wirkung gegen Unkräuter: Hühnerhirse | Grauer Amaranth | Gänsefuß | Phytotoxizität gegen Sojabohnen |
|---|---|---|---|---|---|
| 146 | 0,25 | 4 | 5 | 5 | 0 |
| | 0,125 | 3 | 5 | 5 | 0 |
| 159 | 0,25 | 5 | 5 | 5 | 1 |
| | 0,125 | 5 | 5 | 5 | 0 |
| | 0,06 | 4 | 5 | 5 | 0 |
| 183 | 0,25 | 4 | 5 | 5 | 0 |
| | 0,125 | 3 | 5 | 5 | 0 |
| 248 | 0,25 | 4 | 5 | 5 | 1 |
| | 0,125 | 3 | 5 | 5 | 0 |
| 257 | 0,25 | 5 | 5 | 5 | 0 |
| | 0,125 | 3 | 5 | 5 | 0 |
| Vergleichs-Verbindung | | | | | |
| E-1 | 0,5 | 4 | 5 | 5 | 4 |
| | 0,25 | 3 | 5 | 5 | 3 |
| | 0,125 | 3 | 5 | 3 | 2 |
| E-2 | 0,5 | 4 | 5 | 5 | 4 |
| | 0,25 | 3 | 5 | 5 | 3 |

Beispiel 31

Test gegen Unkräuter auf höher gelegenem Ackerboden durch Laub-Behandlung:

In einem Gewächshaus wurden Weizen-Samen und Mais-Samen in Töpfe von 500 $cm^2$ Fläche, die mit Ackererde gefüllt waren, eingesät, und Erde, die Samen von Digitaria (Digitaria sanguinalis), grauem Amaranth (Amaranthus lividus L.) und Gänsefuß (Chenopodium album L.) enthielt, wurde über die Erde in den Töpfen in einer Tiefe von 1 cm gestreut.

Nach der Einsaat wurden die Pflanzen 14 Tage angezogen, und eine Test-Chemikalie in einer vorher festgelegten Konzentration, hergestellt wie in Beispiel 29, wurde gleichmäßig über Test-Pflanzen in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 29 geprüft. Die Ergebnisse sind in Tabelle 18 aufgeführt.

Tabelle 18

| Akt. Verbindung Nr. | Wirkstoff-Menge kg/ha | Herbizide Wirkung gegen Unkräuter Digitaria | Grauer Amaranth | Gänsefuß | Phytotoxizität gegen Weizen | Mais |
|---|---|---|---|---|---|---|
| 146 | 0,25 | 4 | 5 | 5 | 0 | 0 |
| | 0,125 | 3 | 5 | 5 | 0 | 0 |
| 159 | 0,25 | 5 | 5 | 5 | 1 | 1 |
| | 0,125 | 5 | 5 | 5 | 0 | 0 |
| | 0,06 | 4 | 5 | 5 | 0 | 0 |
| 197 | 0,25 | 4 | 5 | 5 | 1 | 1 |
| | 0,125 | 3 | 5 | 5 | 0 | 0 |
| 257 | 0,25 | 4 | 5 | 5 | 1 | 1 |
| | 0,125 | 4 | 5 | 5 | 0 | 0 |
| 284 | 0,25 | 4 | 5 | 5 | 0 | 0 |
| | 0,125 | 3 | 5 | 5 | 0 | 0 |
| Vergleichs-Verbindung | | | | | | |
| E-1 | 0,5 | 5 | 5 | 5 | 4 | 4 |
| | 0,25 | 5 | 5 | 5 | 2 | 3 |
| | 0,125 | 4 | 5 | 5 | 1 | 1 |
| E-2 | 0,25 | 4 | 5 | 5 | 3 | 4 |
| | 0,125 | 3 | 5 | 5 | 2 | 3 |

## Patentansprüche

1. Benzo-anellierte cyclische Verbindungen der Formel (I)

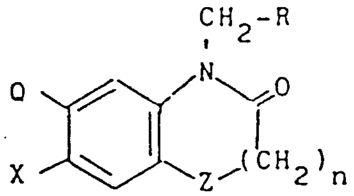

(I)

,

in der

Q

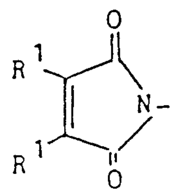 , 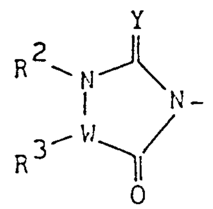 ,

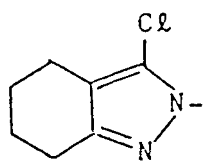 oder 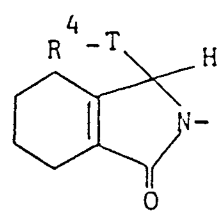

ist,

$R^1$ Methyl ist oder ein $R^1$ zusammen mit einem anderen $R^1$ Tetramethylen bilden kann,

$R^2$ zusammen mit $R^3$ Tetramethylen oder $-CH_2CH=CHCH_2-$ bezeichnet,

$R^4$ Wasserstoff, $C_1$-$C_4$-Alkyl, $C_1$-$C_4$-Alkylcarbonyl oder Phenyl ist,

Y O oder S ist,

W - H- oder - - ist,

T O, S, -NH- oder - $-C_1$-$C_4$-alkyl ist und

$R^4$ zusammen mit T Chlor bezeichnen kann,

Z O oder S ist,

X Wasserstoff oder Halogen ist,

n 0 oder 1 ist und

R $C_{3-6}$-Cycloalkyl, eine gegebenenfalls substituierte 5-gliedrige heterocyclische Gruppe, die ein bis drei Stickstoff-Atome oder ein bis zwei Stickstoff-Atome zusammen mit einem Sauerstoff- oder einem Schwefel-Atom enthält, oder eine gegebenenfalls substituierte 6-gliedrige heteroaromatische Gruppe, die ein bis drei Stickstoff-Atome enthält, ist, wobei gleiche oder verschiedene Substituenten in jedem Fall Amino, $C_1$-$C_4$-Alkylamino, Di($C_1$-$C_4$-alkyl)amino, Formylamino, $C_1$-$C_4$-Alkylcarbonylamino, Halogen, $C_1$-$C_4$-Alkoxy, $C_1$-$C_4$-Alkylthio, $C_1$-$C_4$-Alkyl, Halogeno-$C_1$-$C_4$-alkyl und/oder $C_1$-$C_4$-Alkoxy-$C_1$-$C_4$-alkyl sind, sowie in dem Fall, in dem R eine 6-gliedrige heteroaromatische Gruppe ist, auch deren Hydrochlorid-Salze.

**2.** Verbindungen der Formel (I) nach Anspruch 1, dadurch gekennzeichnet, daß

Q

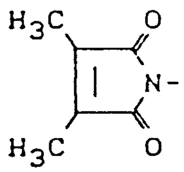 , 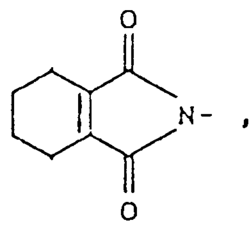 , 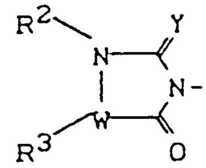 oder

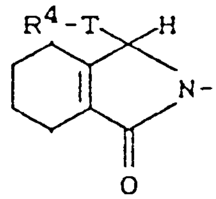

ist, worin

$R^2$ zusammen mit $R^3$ Tetramethylen oder $-CH_2CH=CHCH_2-$ bezeichnet,

$R^4$ Wasserstoff, Methyl, Ethyl, Methylcarbonyl oder Phenyl ist,

T O oder S ist oder $R^4$ zusammen mit T Chlor bezeichnen kann,

Y O oder S ist und

W - H- oder - - ist,

und

Z O oder S ist,

X Wasserstoff oder Fluor ist,

n 0 oder 1 ist und

R Cyclopropyl, eine gegebenenfalls substituierte 5-gliedrige heterocyclische Gruppe, die wenigstens ein Stickstoff-Atom und entweder ein Sauerstoff-Atom oder ein Schwefel-Atom enthält, oder eine 6-gliedrige heteroaromatische Gruppe, die ein oder zwei Stickstoff-Atome enthält, ist, wobei gleiche oder verschiedene Substituenten in jedem Fall Amino, Methylamino, Ethylamino, Dimethylamino, Formylamino, Acetamido, Fluoro, Chloro, Bromo, Methoxy, Ethoxy, Methylthio, Ethylthio, Methyl, Ethyl, Isopropyl, Trichloromethyl, Trifluoromethyl und/oder Methoxymethyl sind.

**3.** Verbindungen der Formel (I) nach Anspruch 1, dadurch gekennzeichnet, daß

Q

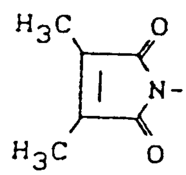 , 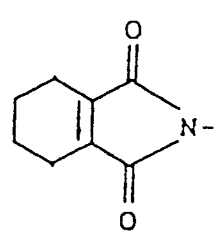 oder 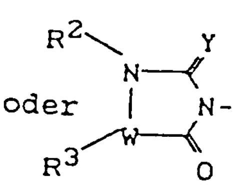

ist, worin

$R^2$ zusammen mit $R^3$ Tetramethylen oder $-CH_2CH=CHCH_2-$ bezeichnet,

Y O oder S ist und

W - H- oder - - ist,

Z O oder S ist,

X Fluor ist,

n 0 oder 1 ist und

R eine gegebenenfalls substituierte heterocyclische Gruppe ist, die die folgenden Systeme Tetrahydrofuran, Furan, Thiophen, Pyrazol, Imidazol, 1,2,4-Triazol, Tetrazol, Isoxazol, Oxazol, 1,2,4-Oxadiazol, 1,2,5-Oxadiazol, 1,3,4-Oxadiazol, Isothiazol, Thiazol, 1,2,3-Thiadiazol, 1,2,4-Thiadiazol, 1,2,5-Thiadiazol, 1,3,4-Thiadiazol, Pyridin, Pyrimidin und Pyrazin umfaßt,

wobei gleiche oder verschiedene Substituenten in jedem Fall Amino, Methylamino, Ethylamino, Dimethylamino, Formylamino, Acetamido, Fluoro, Chloro, Bromo, Methoxy, Ethoxy, Methylthio, Ethylthio, Methyl, Ethyl, Isopropyl, Trichloromethyl, Trifluoromethyl und/oder Methoxymethyl sind.

**4.** Verfahren zur Herstellung der benzo-anellierten cyclischen Verbindungen der Formel (I)

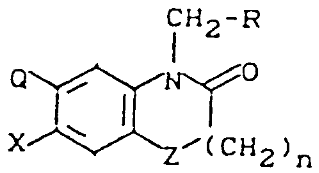

(I)

,

in der Q, X, Z, R und n die in Anspruch 1 angegebenen Bedeutungen haben,
und der Salze derselben,
dadurch gekennzeichnet, daß

a) in dem Fall, in dem Q

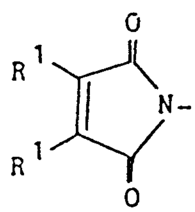

ist,
Verbindungen der Formel (II)

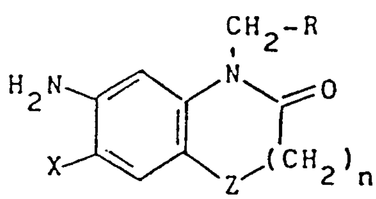

(II)

,

in der Z, X, R und n die im Vorstehenden angegebenen Bedeutungen haben, mit Verbindungen der Formel (III)

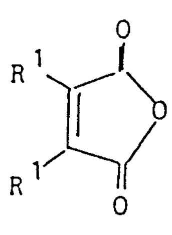

(III)

,

in der $R^1$ die im Vorstehenden angegebenen Bedeutungen hat, in Gegenwart inerter Lösungsmittel umgesetzt werden,
oder

b) in dem Fall, in dem Q

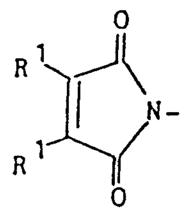

ist,
Verbindungen der Formel (IV)

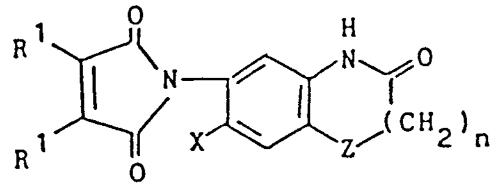

(IV),

in der $R^1$, Z, X und n die im Vorstehenden angegebenen Bedeutungen haben, mit Verbindungen der Formel (V)

$M\text{-}CH_2\text{-}R$ (V),

in der R die im Vorstehenden angegebenen Bedeutungen hat und M Halogen, Methansulfonyloxy, Benzolsulfonylory oder p-Toluolsulfonyloxy ist, in Gegenwart inerter Lösungsmittel und gegebenenfalls in Gegenwart einer Base umgesetzt werden,
oder
c) in dem Fall, in dem Q

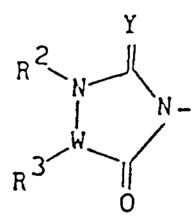

ist, worin
$R^2$ zusammen mit $R^3$ Tetramethylen bezeichnet und
W - H- oder - - ist,
Verbindungen der Formel (VI)

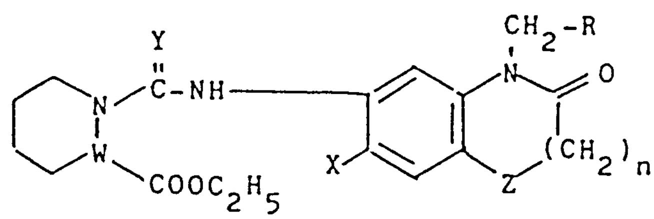

(VI) ,

in der Y, W, Z, X, R und n die im Vorstehenden angegebenen Bedeutungen haben, mit geeigneten Basen in Gegenwart inerter Lösungsmittel umgesetzt werden,

oder

d) in dem Fall, in dem Q

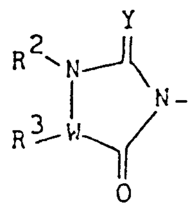

ist, worin

$R^2$ zusammen mit $R^3$ $-CH_2CH=CHCH_2-$ bezeichnet und

W - - ist,

Verbindungen der Formel (VII)

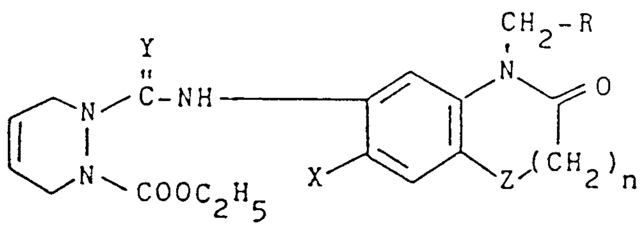

(VII)

,

in der Y, W, Z, X, R und n die im Vorstehenden angegebenen Bedeutungen haben, mit geeigneten Basen in Gegenwart inerter Lösungsmittel umgesetzt werden,

oder

e) in dem Fall, in dem Q

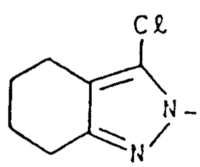

ist,

Verbindungen der Formel (VIII)

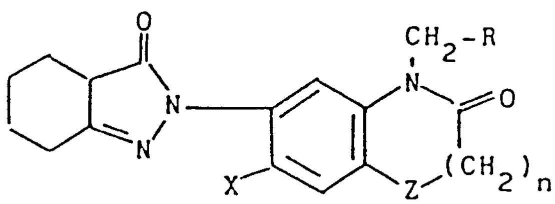

(VIII)

,

in der Z, X, R und n die im Vorstehenden angegebenen Bedeutungen haben, mit einem Chlorierungsmittel in Gegenwart inerter Lösungsmittel umgesetzt werden,

oder

f) in dem Fall, in dem Q

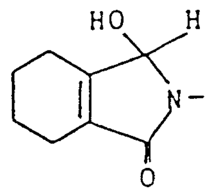

ist,
Verbindungen der Formel (Ia)

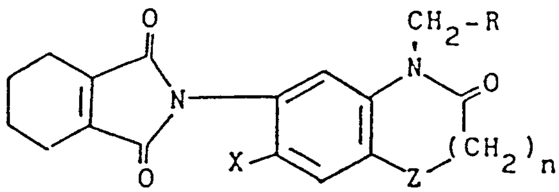

(Ia)

,

in der Z, X, R und n die im Vorstehenden angegebenen Bedeutungen haben, in Gegenwart inerter Lösungsmittel reduziert werden,
oder
g) in dem Fall, in dem Q

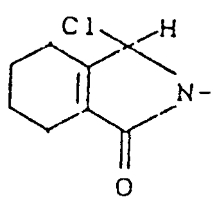

ist,
Verbindungen der Formel (Ib)

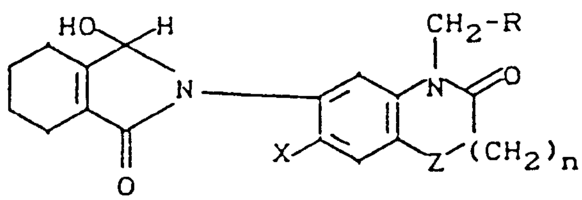

(Ib)

,

in der Z, X, R und n die im Vorstehenden angegebenen Bedeutungen haben, mit einem Chlorierungsmittel in Gegenwart inerter Lösungsmittel umgesetzt werden,
oder
h) in dem Fall, in dem Q

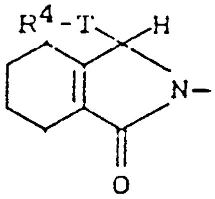

ist, worin $R^4$ Acyl ist und T O ist, $R^4$ durch $R^5$ ersetzt wird und die oben bezeichneten Verbindungen der Formel (Ib)

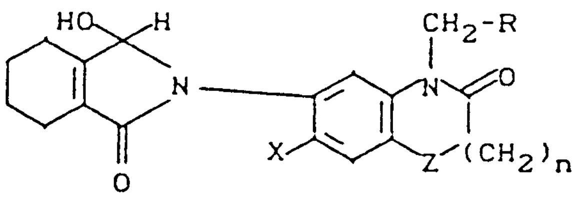 (Ib) ,

in der Z, X, R und n die im Vorstehenden angegebenen Bedeutungen haben, mit Verbindungen der Formel (IX)

$R^5$-Cl (IX) ,

worin $R^5$ Acyl ist,
oder mit Verbindungen der Formel (X)

$(R^5)_2O$ (X) ,

worin $R^5$ die im Vorstehenden angegebenen Bedeutungen hat, in Gegenwart inerter Lösungsmittel und gegebenenfalls in Gegenwart von Basen umgesetzt werden,
oder
i) in dem Fall, in dem Q

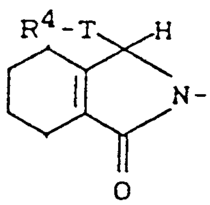

ist, mit der Maßgabe, daß $R^4$-T- nicht -OH, -Cl oder Acyloxy bezeichnet, $R^4$-T- durch $R^6$ ersetzt wird und die oben bezeichneten
Verbindungen der Formel (Ic)

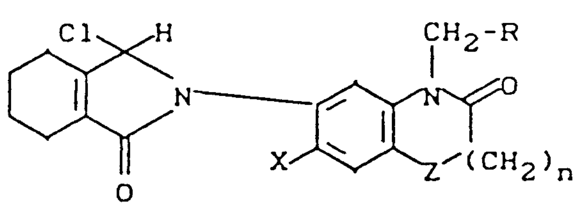 (Ic) ,

in der Z, X, R und n die im Vorstehenden angegebenen Bedeutungen haben, mit Verbindungen der Formel (XI)

$R^6$-H (XI) ,

worin $R^6$ Alkoxy, Alkylthio, Phenoxy, Phenylthio, Alkylamino oder Dialkylamino ist, in Gegenwart inerter Lösungsmittel und gegebenenfalls in Gegenwart einer Base umgesetzt werden.

**5.** Herbizide Zusammensetzungen, dadurch gekennzeichnet, daß sie wenigstens ein Benzoxazinon der Formel (I) nach den Ansprüchen 1 bis 4 enthalten.

**6.** Verfahren zur Bekämpfung von Unkräutern, dadurch gekennzeichnet, daß Benzoxazinone der Formel (I) nach den Ansprüchen 1 bis 4 auf Unkräuter und/oder deren Lebensraum zur Einwirkung gebracht werden.

**7.** Verwendung von Benzoxazinonen der Formel (I) nach den Ansprüchen 1 bis 4 zur Bekämpfung von Unkräutern.

**8.** Verfahren zur Herstellung herbizider Zusammensetzungen, dadurch gekennzeichnet, daß Benzoxazinone der Formel (I) nach den Ansprüchen 1 bis 4 mit Streckmitteln und/oder grenzflächenaktiven Mitteln vermischt werden.

**9.** Verbindungen der Formel (XIX)

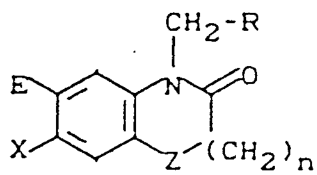

(XIX)

,

in der

E Wasserstoff, Nitro, Amino, Hydrazino, Cyanato oder Thiocyanato ist,

X Wasserstoff oder Halogen ist,

Z O oder S ist,

R $C_{3-6}$-Cycloalkyl, eine gegebenenfalls substituierte 5-gliedrige heterocyclische Gruppe, die ein bis drei Stickstoff-Atome oder ein bis zwei Stickstoff-Atome zusammen mit einem Sauerstoff- oder einem Schwefel-Atom enthält, oder eine gegebenenfalls substituierte 6-gliedrige heteroaromatische Gruppe, die ein bis drei Stickstoff-Atome enthält, ist, wobei gleiche oder verschiedene Substituenten in jedem Fall Amino, $C_1$-$C_4$-Alkylamino, Di($C_1$-$C_4$-alkyl)amino, Formylamino, $C_1$-$C_4$-Alkylcarbonylamino, Halogen, $C_1$-$C_4$-Alkoxy, $C_1$-$C_4$-Alkylthio, $C_1$-$C_4$-Alkyl, Halogeno-$C_1$-$C_4$-alkyl und/oder $C_1$-$C_4$-Alkoxy-$C_1$-$C_4$-alkyl sind

n 0 oder 1 ist.

mit Aushahme der Derivate in denen R einen Pyridinrest darstellt.

## Claims

**1.** Benzo-fused cyclic compounds of the formula (I)

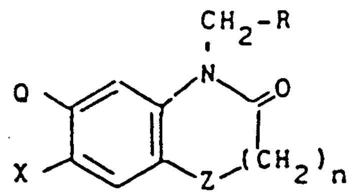

(I)

in which

Q is

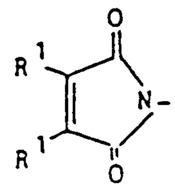 , 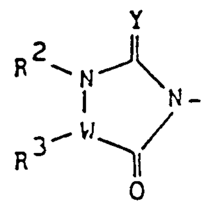 ,

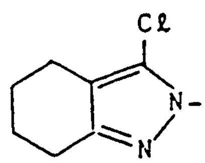 or 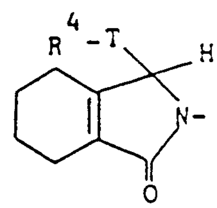

$R^1$ is methyl, or an $R^1$ together with another $R^1$ can form tetramethylene,
$R^2$ together with $R^3$ denotes tetramethylene or $-CH_2CH=CHCH_2-$,
$R^4$ is hydrogen, $C_1$-$C_4$-alkyl, $C_1$-$C_4$-alkylcarbonyl or phenyl,
Y is O or S,
W is -  H- or -  -,
T is O, S, -NH- or -  -$C_1$-$C_4$-alkyl and $R^4$ together with T can denote chlorine,

Z is O or S,

X is hydrogen or halogen,

n is 0 or 1 and

R is $C_{3-6}$-cycloalkyl, an optionally substituted 5-membered heterocyclic group which contains one to three nitrogen atoms or one to two nitrogen atoms together with an oxygen or a sulphur atom, or an optionally substituted 6-membered heteroaromatic group which contains one to three nitrogen atoms, where identical or different substituents in each case are amino, $C_1$-$C_4$-alkylamino, di($C_1$-$C_4$-alkyl)amino, formylamino, $C_1$-$C_4$-alkylcarbonylamino, halogen, $C_1$-$C_4$-alkoxy, $C_1$-$C_4$-alkylthio, $C_1$-$C_4$-alkyl, halogeno-$C_1$-$C_4$-alkyl and/or $C_1$-$C_4$-alkoxy-$C_1$-$C_4$-alkyl, and, in the event that R is a 6-membered heteroaromatic group, also their hydrochloride salts.

**2.** Compounds of the formula (I) according to Claim 1, characterised in that

Q is

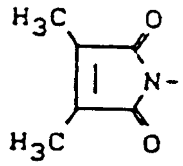 , 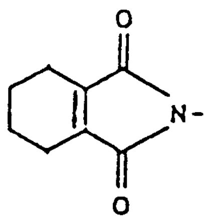 , 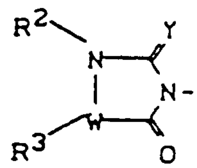 or

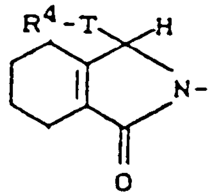

in which
$R^2$ together with $R^3$ denotes tetramethylene or $-CH_2CH=CHCH_2-$,
$R^4$ is hydrogen, methyl, ethyl, methylcarbonyl or phenyl,
T is O or S, or $R^4$ together with T can denote chlorine,
Y is O or S and
W is - - or - -,
and

Z is O or S,
X is hydrogen or fluorine,
n is 0 or 1 and
R is cyclopropyl, an optionally substituted 5-membered heterocyclic group which contains at least one nitrogen atom and either an oxygen atom or a sulphur atom, or a 6-membered heteroaromatic group which contains one or two nitrogen atoms, where identical or different substituents are in each case amino, methylamino, ethylamino, dimethylamino, formylamino, acetamido, fluoro, chloro, bromo, methoxy, ethoxy, methylthio, ethylthio, methyl, ethyl, isopropyl, trichloromethyl, trifluoromethyl and/or methoxymethyl.

**3.** Compounds of the formula (I) according to Claim 1, characterised in that

Q is

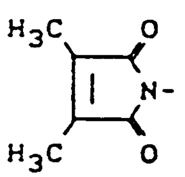 , 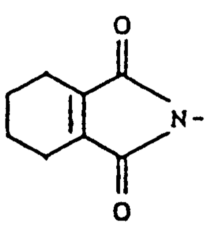 or 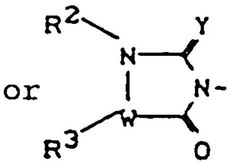

in which
$R^2$ together with $R^3$ denotes tetramethylene or $-CH_2CH=CHCH_2-$,
Y is O or S and
W is - H- or - -,

Z is O or S,
X is fluorine,
n is 0 or 1 and
R is an optionally substituted heterocyclic group which comprises the following systems tetrahydrofuran, furan, thiophene, pyrazole, imidazole, 1,2,4-triazole, tetrazole, isoxazole, oxazole, 1,2,4-oxadiazole, 1,2,5-oxadiazole, 1,3,4-oxadiazole, isothiazole, thiazole, 1,2,3-thiadiazole, 1,2,4-thiadiazole, 1,2,5-thiadiazole, 1,3,4-thiadiazole, pyridine, pyrimidine and pyrazine,

where identical or different substituents in each case are amino, methylamino, ethylamino, dimethylamino, formylamino, acetamido, fluoro, chloro, bromo, methoxy, ethoxy, methylthio, ethylthio, methyl, ethyl, isopropyl, trichloromethyl, trifluoromethyl and/or methoxymethyl.

**4.** Process for the preparation of the benzo-fused cyclic compounds of the formula (I)

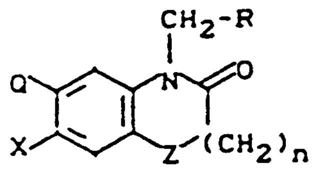

(I)

in which Q, X, Z, R and n have the meanings given in Claim 1,
and of the salts of these compounds,
characterised in that

a) in the event that Q is

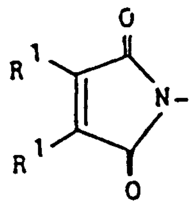

compounds of the formula (II)

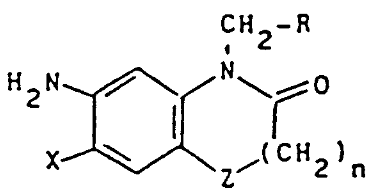

(II)

in which Z, X, R and n have the meanings given above, are reacted with compounds of the formula (III)

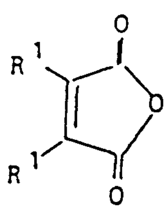

(III)

in which $R^1$ has the meanings given above, in the presence of inert solvents,
or

b) in the event that Q is

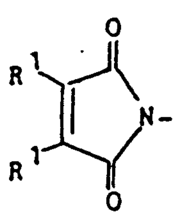

compounds of the formula (IV)

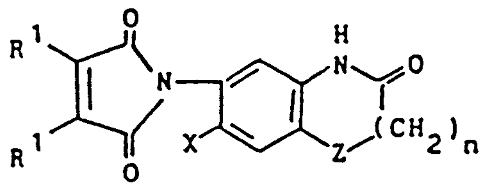

(IV)

in which $R^1$, Z, X and n have the meanings given above, are reacted with compounds of the formula (V)

$M-CH_2-R$ (V)

in which R has the meanings given above and M is halogen, methanesulphonyloxy, benzenesulphonyloxy or p-toluenesulphonyloxy, in the presence of inert solvents and, if appropriate, in the presence of a base,

or

c) in the event that Q is

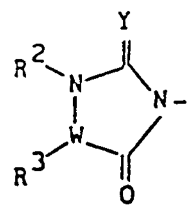

in which

$R^2$ together with $R^3$ denotes tetramethylene and

W is - H- or - -,

compounds of the formula (VI)

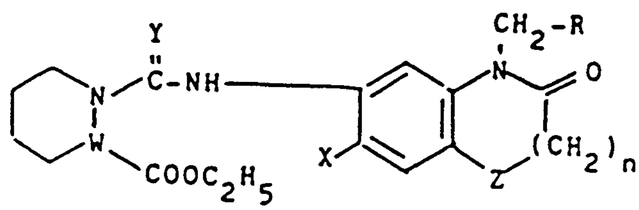

(VI)

in which Y, W, Z, X, R and n have the meanings given above, are reacted with suitable bases in the presence of inert solvents,

or

d) in the event that Q is

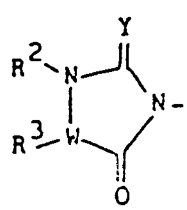

in which

$R^2$ together with $R^3$ denotes $-CH_2CH=CHCH_2-$ and
W is - -,

compounds of the formula (VII)

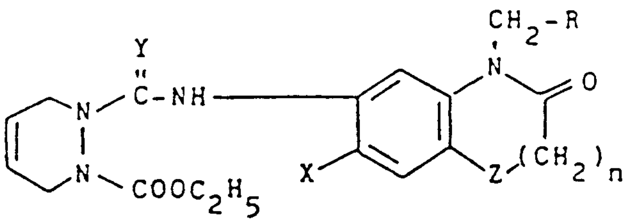

(VII)

in which Y, W, Z, X, R and n have the meanings given above, are reacted with suitable bases in the presence of inert solvents,

or

e) in the event that Q is

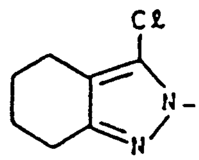

compounds of the formula (VIII)

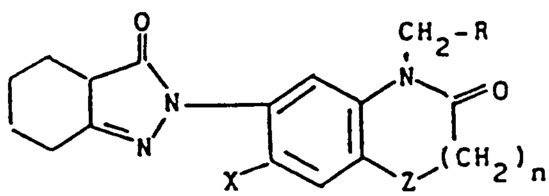

(VIII)

in which Z, X, R and n have the meanings given above, are reacted with a chlorinating agent in the presence of inert solvents,

or

f) in the event that Q is

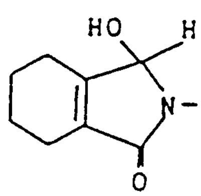

compounds of the formula (Ia)

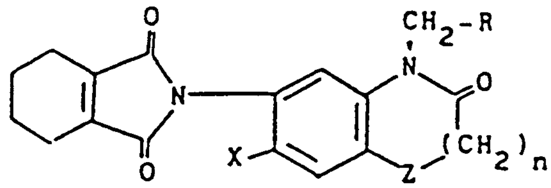

(Ia)

in which Z, X, R and n have the meanings given above, are reduced in the presence of inert solvents,
or
g) in the event that Q is

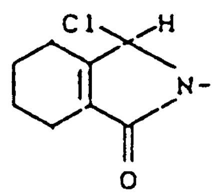

compounds of the formula (Ib)

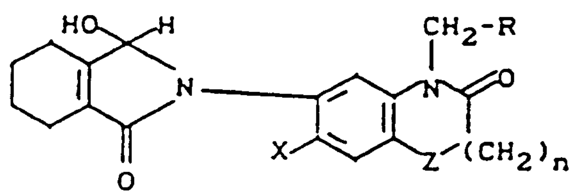

(Ib)

in which Z, X, R and n have the meanings given above, are reacted with a chlorinating agent in the presence of inert solvents,
or
h) in the event that Q is

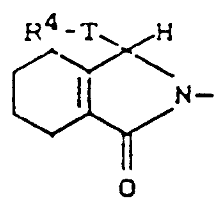

in which $R^4$ is acyl and T is O, $R^4$ is replaced by $R^5$ and the above-designated compounds of the formula (Ib)

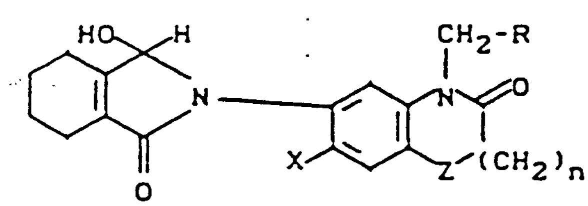

(Ib)

in which Z, X, R and n have the meanings given above, are reacted with compounds of the formula (IX)

$R^5$-Cl (IX),

in which $R^5$ is acyl,
or with compounds of the formula (X)

$(R^5)_2O$ (X),

in which $R^5$ has the meanings given above, in the presence of inert solvents and, if appropriate, in the presence of bases,
or
i) in the event that Q is

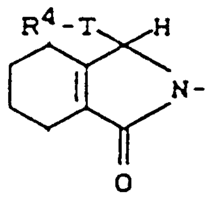

with the proviso that $R^4$-T- does not denote -OH, -Cl or acyloxy, $R^4$-T- is replaced by $R^6$ and the above-designated compounds of the formula (Ic)

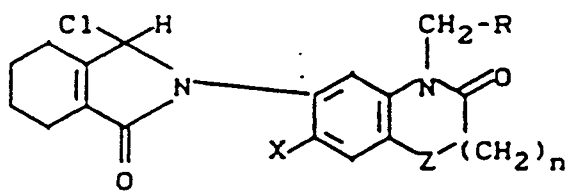

(Ic)

in which Z, X, R and n have the meanings given above, are reacted with compounds of the formula (XI)

$R^6$-H (XI)

in which $R^6$ is alkoxy, alkylthio, phenoxy, phenylthio, alkylamino or dialkylamino, in the presence of inert solvents and, if appropriate, in the presence of a base.

5. Herbicidal compositions, characterised in that they contain at least one benzoxazinone of the formula (I) according to Claims 1 to 4.

6. Process for combating weeds, characterised in that benzoxazinones of the formula (I) according to Claims 1 to 4 are allowed to act on weeds and/or their environment.

7. Use of benzoxazinones of the formula (I) according to Claims 1 to 4 for combating weeds.

8. Process for the preparation of herbicidal compositions, characterised in that benzoxazinones of the formula (I) according to Claims 1 to 4 are mixed with extenders and/or surface-active agents.

9. Compounds of the formula (XIX)

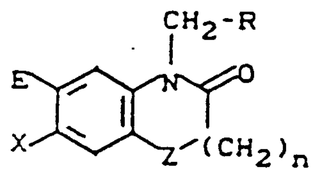

(XIX)

in which

E is hydrogen, nitro, amino, hydrazino, cyanato or thiocyanato,

X is hydrogen or halogen,

Z is O or S,

R is $C_{3-6}$-cycloalkyl, an optionally substituted 5-membered heterocyclic group which contains one to three nitrogen atoms or one to two nitrogen atoms together with an oxygen or a sulphur atom, or an optionally substituted 6-membered heteroaromatic group which contains one to three nitrogen atoms, where identical or different substituents in each case are amino, $C_1$-$C_4$-alkylamino, di($C_1$-$C_4$-alkyl)amino, formylamino, $C_1$-$C_4$-alkylcarbonylamino, halogen, $C_1$-$C_4$-alkoxy, $C_1$-$C_4$-alkylthio, $C_1$-$C_4$-alkyl, halogeno-$C_1$-$C_4$-alkyl and/or $C_1$-$C_4$-alkoxy-$C_1$-$C_4$-alkyl, and

n is 0 or 1,

with the exception of the derivatives in which R represents a pyridine radical.

## Revendications

**1.** Composés cycliques à noyau benzénique condensé de formule I

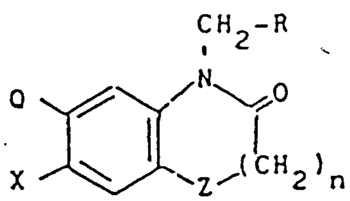

(I)

dans laquelle

Q représente

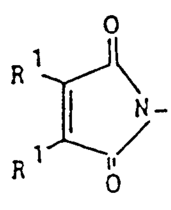

,

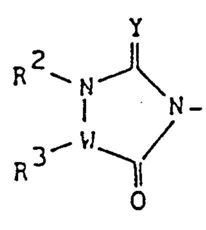

,

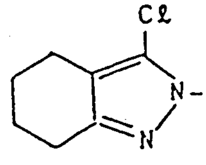

ou

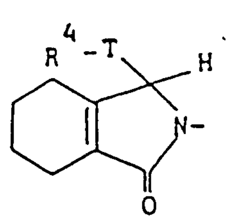

chacun des symboles $R^1$ représente un groupe méthyle ou bien deux groupes $R^1$ forment ensemble un groupe tétraméthylène,

$R^2$ forme avec $R^3$ un groupe tétraméthylène ou $-CH_2CH=CHCH_2-$,

$R^4$ représente l'hydrogène, un groupe alkyle en $C_1$-$C_4$, (alkyle en $C_1$-$C_4$)-carbonyle ou phényle,

Y représente O ou S,
W représente - H- ou - -,
T représente O, S, -NH- ou -N-alkyle en $C_1$-$C_4$, et
$R^4$ peut également représenter avec T, le chlore,
Z représente O ou S,
X représente l'hydrogène ou un halogène,
n est égal à 0 ou 1 et
R représente un groupe cycloalkyle en $C_3$-$C_6$, un groupe hétérocyclique à 5 chaînons éventuellement substitué contenant 1 à 3 atomes d'azote ou 1 ou 2 atomes d'azote et un atome d'oxygène ou de soufre, ou un groupe hétéroaromatique à 6 chaînons éventuellement substitué contenant 1 à 3 atomes d'azote, les substituants, identiques ou différents, étant dans chaque cas des groupes amino, alkylamino en $C_1$-$C_4$, di-(alkyle en $C_1$-$C_4$)-amino, formylamino, (alkyle en $C_1$-$C_4$)-carbonylamino, des halogènes, des groupes alcoxy en $C_1$-$C_4$, alkylthio en $C_1$-$C_4$, alkyle en $C_1$-$C_4$, halogénoalkyle en $C_1$-$C_4$ et/ou (alcoxy en $C_1$-$C_4$)-alkyle en $C_1$-$C_4$, et, dans le cas où R représente un groupe hétéroaromatique à 6 chaînons, leurs chlorhydrates.

**2.** Composés de formule I de la revendication 1, caractérisés en ce que
Q représente

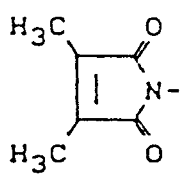 , 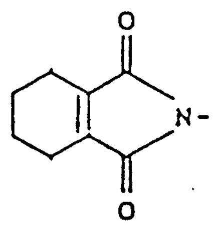 , 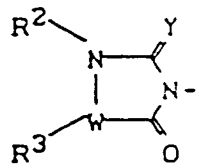 ou

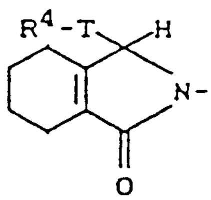

dans lesquels
$R^2$ forme avec $R^3$ un groupe tétraméthylène ou $-CH_2CH=CHCH_2-$,
$R^4$ représente l'hydrogène, un groupe méthyle, éthyle, méthylcarbonyle ou phényle,
T représente O ou S, ou bien $R^4$ et T représentent ensemble le chlore,
Y représente O ou S et
W représente - H- ou - -, et
Z représente O ou S,
X représente l'hydrogène ou le fluor,
n est égal à 0 ou 1, et
R représente un groupe cyclopropyle, un groupe hétérocyclique à 5 chaînons éventuellement substitué contenant au moins 1 atome d'azote et 1 atome d'oxygène ou 1 atome de soufre, ou bien un groupe hétéroaromatique à 6 chaînons contenant 1 ou 2 atomes d'azote, les substituants, identiques ou différents, étant dans chaque cas des groupes amino, méthylamino, éthylamino, diméthylamino, formylamino, acétamido, le fluor, le chlore, le brome, des groupes méthoxy, éthoxy, méthylthio, éthylthio, méthyle, éthyle, isopropyle, trichlorométhyle, trifluorométhyle et/ou méthoxyméthyle.

**3.** Composés de formule I de la revendication 1, caractérisés en ce que
Q représente

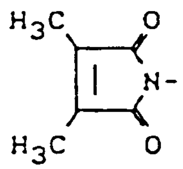 , 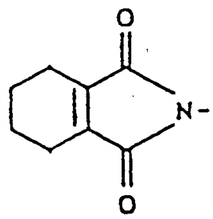 ou 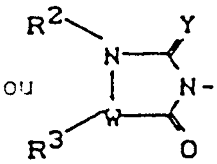

dans lesquels
$R^2$ forme avec $R^3$ un groupe tétraméthylène ou $-CH_2CH=CHCH_2-$,
Y représente O ou S et
W représente - H- ou - -,
Z représente O ou S,
X représente le fluor,
n est égal à 0 ou 1, et
R représente un groupe hétérocyclique éventuellement substitué pris parmi les systèmes suivants : tétrahydrofuranne, furanne, thiophène, pyrazole, imidazole, 1,2,4-triazole, tétrazole, isoxazole, oxazole, 1,2,4-oxadiazole, 1,2,5-oxadiazole, 1,3,4-oxadiazole, isothiazole, thiazole, 1,2,3-thiadiazole, 1,2,4-thiadiazole, 1,2,5-thiadiazole, 1,3,4-thiadiazole, pyridine, pyrimidine et pyrazine, les substituants, identiques ou différents, étant dans chaque cas des groupes amino, méthylamino, éthylamino, diméthylamino, formylamino, acétamido, le fluor, le chlore, le brome, des groupes méthoxy, éthoxy, méthylthio, éthylthio, méthyle, éthyle, isopropyle, trichlorométhyle, trifluorométhyle et/ou méthoxyméthyle.

**4.** Procédé de préparation des composés cycliques à noyau benzénique condensé de formule I

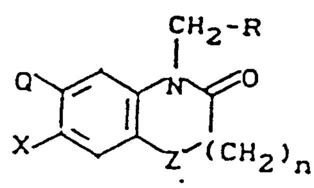

(I)

dans laquelle Q, X, Z, R et n ont les significations indiquées dans la revendication 1,
et de leurs sels,
caractérisé en ce que :
a) dans le cas où Q représente

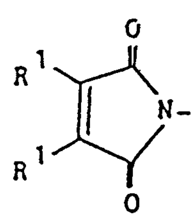

on fait réagir des composés de formule II

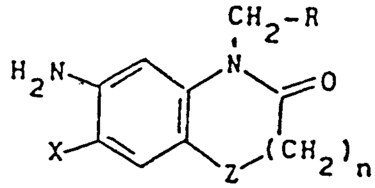

(II)

Z, X, R et n ayant les significations indiquées ci-dessus, avec des composés de formule III

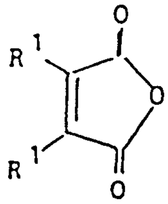

(III)

dans laquelle $R^1$ a les significations indiquées ci-dessus, en présence de solvants inertes, ou bien

b) dans le cas où Q représente

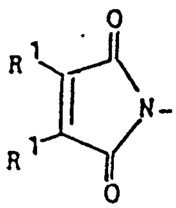

on fait réagir des composés de formule IV

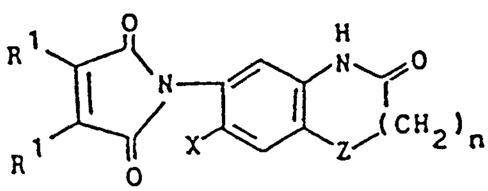

(IV)

dans laquelle $R^1$, Z, X et n ont les significations indiquées ci-dessus, avec des composés de formule V

$M-CH_2-R$ (V)

dans laquelle R a les significations indiquées ci-dessus et M représente un halogène, un groupe méthane-sulfonyloxy, benzène-sulfonyloxy ou p-toluène-sulfonyloxy, en présence de solvants inertes et éventuellement en présence d'une base, ou bien

c) dans le cas où Q représente

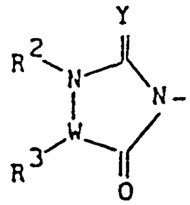

dans lequel
$R^2$ forme avec $R^3$ un groupe tétraméthylène, et
W représente - H- ou - -,
on fait réagir des composés de formule VI

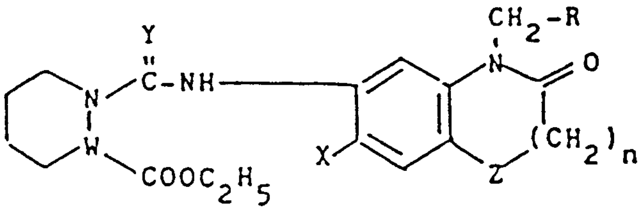

(VI)

dans laquelle Y, W, Z, X, R et n ont les significations indiquées ci-dessus, avec des bases appropriées en présence de solvants inertes, ou bien

d) dans le cas où Q représente

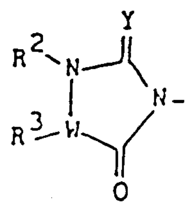

dans lequel
$R^2$ forme avec $R^3$ un groupe $-CH_2CH=CHCH_2-$ et
W représente - -,
on fait réagir des composés de formule VII

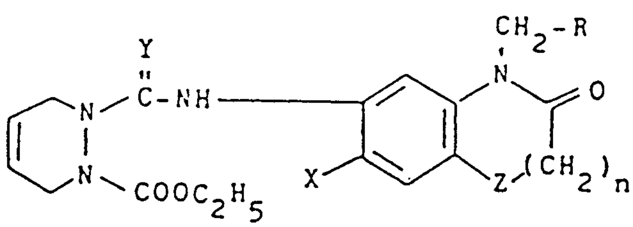

(VII)

dans laquelle Y, W, Z, X, R et n ont les significations indiquées ci-dessus, avec des bases appropriées en présence de solvants inertes, ou bien

e) dans le cas où Q représente

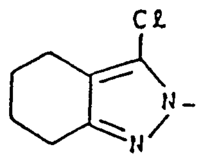

on fait réagir des composés de formule VIII

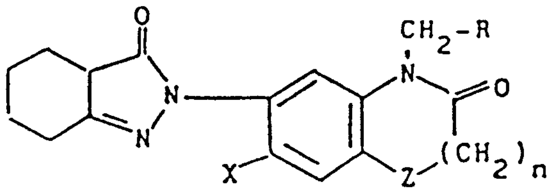

(VIII)

dans laquelle Z, X, R et n ont les significations indiquées ci-dessus, avec un agent chlorant en présence de solvants inertes, ou bien
f) dans le cas où Q représente

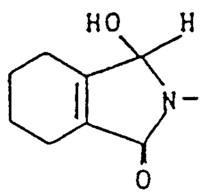

on réduit des composés de formule Ia

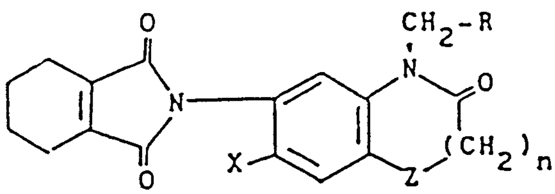

(Ia)

dans laquelle Z, X, R et n ont les significations indiquées ci-dessus, en présence de solvants inertes, ou bien
g) dans le cas où Q représente

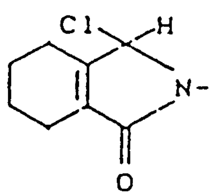

on fait réagir des composés de formule Ib

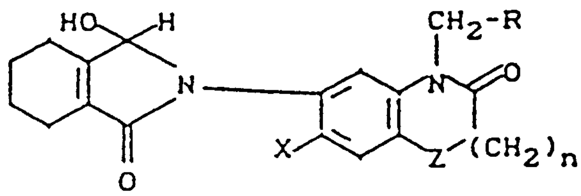

(Ib)

dans laquelle Z, X, R et n ont les significations indiquées ci-dessus, avec un agent chlorant en présence de solvants inertes, ou bien

h) dans le cas où Q représente

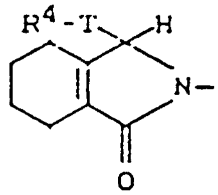

dans lequel $R^4$ représente un groupe acyle et T représente O, on remplace $R^4$ par $R^5$ et on fait réagir les composés, définis ci-dessus, de formule Ib

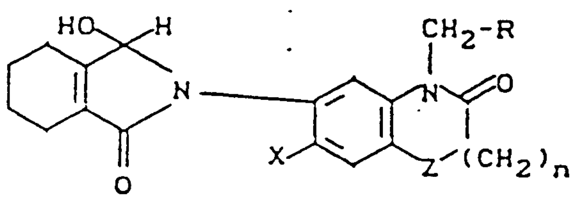

(Ib)

dans laquelle Z, X, R et n ont les significations indiquées ci-dessus, avec des composés de formule IX

$R^5$-Cl (IX)

dans laquelle $R^5$ représente un groupe acyle,

ou avec des composés de formule X

$(R^5)_2O$ (X)

dans laquelle $R^5$ a les significations indiquées ci-dessus, en présence de solvants inertes et éventuellement en présence de bases, ou bien

i) dans le cas où Q représente

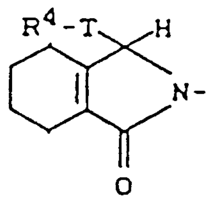

sous réserve que $R^4$-T- ne peut représenter -OH, -Cl ou un groupe acyloxy, on remplace $R^4$-T- par

$R^6$, et on fait réagir les composés, définis ci-dessus, de formule Ic

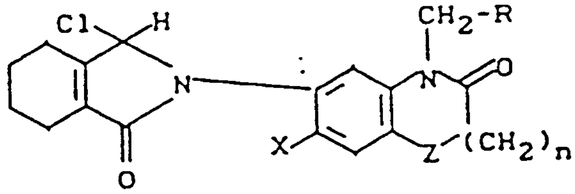

(Ic)

dans laquelle Z, X, R et n ont les significations indiquées ci-dessus, avec des composés de formule XI

$R^6$-H (XI)

dans laquelle $R^6$ représente un groupe alcoxy, alkylthio, phénoxy, phénylthio, alkylamino ou dialkylamino, en présence de solvants inertes et éventuellement en présence d'une base.

**5.** Compositions herbicides, caractérisées en ce qu'elles contiennent au moins une benzoxazinone de formule I selon revendications 1 à 4.

**6.** Procédé pour combattre les végétaux adventices, caractérisé en ce que l'on fait agir sur les végétaux et/ou leur habitat des benzoxazinones de formule I selon revendications 1 à 4.

**7.** Utilisation des benzoxazinones de formule I selon revendications 1 à 4 pour la lutte contre les végétaux adventices.

**8.** Procédé de préparation de compositions herbicides, caractérisé en ce que l'on mélange des benzoxazinones de formule I selon revendications 1 à 4 avec des diluants et/ou des agents tensioactifs.

**9.** Composés de formule XIX

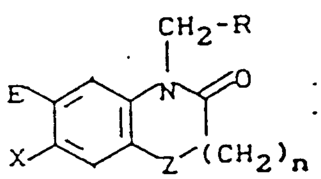

(XIX)

dans laquelle
E représente l'hydrogène, un groupe nitro, amino, hydrazino, cyanato ou thiocyanato,
X représente l'hydrogène ou un halogène,
Z représente O ou S,
R représente un groupe cycloalkyle en $C_3$-$C_6$, un groupe hétérocyclique à 5 chaînons éventuellement substitué qui contient 1 à 3 atomes d'azote ou 1 ou 2 atomes d'azote et 1 atome d'oxygène ou de soufre, ou bien un groupe hétéroaromatique à 6 chaînons éventuellement substitué contenant 1 à 3 atomes d'azote, les substituants, identiques ou différents, étant dans chaque cas des groupes amino, alkylamino en $C_1$-$C_4$, di-(alkyle en $C_1$-$C_4$)-amino, formylamino, (alkyle en $C_1$-$C_4$)-carbonylamino, des halogènes, des groupes alcoxy en $C_1$-$C_4$, alkylthio en $C_1$-$C_4$, alkyle en $C_1$-$C_4$, halogéno-alkyle en $C_1$-$C_4$ et/ou (alcoxy en $C_1$-$C_4$)-alkyle en $C_1$-$C_4$, et
n est égal à 0 ou 1,
à l'exception des composés pour lesquels R représente un radical de pyridine.